# EUROPEAN PATENT APPLICATION

(11) **EP 4 711 359 A2**
(43) Date of publication of application: **18.03.2026**
(21) Application number: 26154817.6
(22) Date of filing: 12.05.2022
(51) Int. Cl.: C07D 277/02

(54) **RESCORCINOLS, METHODS FOR THEIR MANUFACTURE, AND USES THEREOF**

(30) Priority: 12.05.2021 GB 202106787
(62) Divisional of application: 22724861.4
(71) Applicant: Jazz Pharmaceuticals Research UK Limited, Sittingbourne Kent ME9 8AG (GB)
(72) Inventor: SILCOCK, Alan James, Sittingbourne, Kent, ME9 8AG (GB); TSE, Karen Ka-Yen, Sittingbourne, Kent, ME9 8AG (GB); HINCHLIFFE, Paul Stuart, Saffron Walden, CB10 1XL (GB); SHARPE, Andrew, Saffron Walden, CB10 1XL (GB); SIMPSON, Iain David, Saffron Walden, CB10 1XL (GB); PEACH, Joanne, Saffron Walden, CB10 1XL (GB); LEVANTO, Stefano, Saffron Walden, CB10 1XL (GB); MILLET, Antoine, Sittingbourne, Kent, ME9 8AG (GB); MANN, Inderjit Singh, Sittingbourne, Kent, ME9 8AG (GB)
(74) Representative: HGF

(57) **Abstract**

The present invention relates to a group of resorcinol derivatives as a pharmaceutically active compounds and methods of preparation thereof. Resorcinol derivatives have been used to treat various diseases and disorders. While such treatments hold promise, there remains a need in the art for more effective treatments and this has been brought about by way of the resorcinol derivative of the invention.

## Description

### RELATED APPLICATIONS

The present application is related to, and claims the benefit of, GB 2106787.1 filed on 12 May 2021 (12.05.2021), the contents of which are hereby incorporated by reference in their entirety.

### FIELD OF THE INVENTION

The present invention relates to a group of resorcinol derivatives, methods for their manufacture and the use of these compounds as research tools and as pharmaceuticals.

### BACKGROUND TO THE INVENTION

Resorcinol has been widely known as a versatile chemical compound used extensively in the development of advanced chemistries and technologies (Durairaj, 2005). It has been employed in a wide range of applications from medical to chemical, including in the manufacture of resins, plastics, dyes, medicine, and numerous other organic chemical compounds.

Resorcinol appears as white crystals or powder and has a faint, characteristic aromatic odour, with a sweetish bitter taste, and is readily soluble in water and alcohol. Other names for the compound include resorcin, meta-dihydroxybenzene, 1, 3-dihdroxybenzene, 1,3-benzenediol and 3-hydroxyphenol, and its empirical formula is C₆H₆O₂. Structurally, the compound has two hydroxyl groups in the aromatic ring structure, which are located at their meta-positions with respect to each hydroxyl group. The high reactivity of resorcinol is primarily associated with the location of these two hydroxyl groups in the benzene ring, with the hydrogen atoms adjacent to the hydroxyl groups being particularly reactive.

Production of resorcinol can be via several routes, either by using a natural resin such as a distillate of brazilwood, melting any of a large number of resins (such as galbanum and asafoetida) and combining it with potassium hydroxide, or by several synthetic methods. One synthetic manufacturing method is sulfonation of benzene with sulfuric acid and fusing the resulting benzenedisulfonic acid with sodium hydroxide, and subsequently extracting the resorcinol.

One of the uses of resorcinol is its role as a chemical intermediate for the synthesis of pharmaceuticals and other organic compounds. For example, it is used in the production of diazo dyes and plasticizers and as a UV absorber in resins. As a medicine, resorcinol is used as an antiseptic and disinfectant in topical pharmaceutical products in the treatment of skin disorders and infections such as acne, seborrheic dermatitis, eczema, psoriasis, corns, calluses, and warts. Resorcinol works by helping to remove hard, scaly, or roughened skin as it exerts a keratolytic activity. However, the main use of resorcinol is in the production of resins. Reaction with formaldehyde produces resins used to make rayon and nylon amenable to impregnation with rubber, and as adhesives.

The present invention has been devised in light of these considerations.

### BRIEF SUMMARY OF THE INVENTION

At its most general, the present invention relates to invention relates to a group of resorcinol compounds which are biologically active and hence useful in the treatment of diseases. Such compounds may be administered by a wide variety of routes including but not limited to oral, transdermal, buccal, nasal, pulmonary, rectal or ocular. Such compounds may be used for the treatment or prevention of a medical condition including but not limited to epilepsy.

In a first aspect of the invention there is provided a compound of formula (I) or a salt thereof: 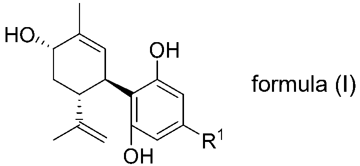 where:
R¹ is CH₃, CH₂CH₃, Cl,

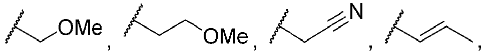

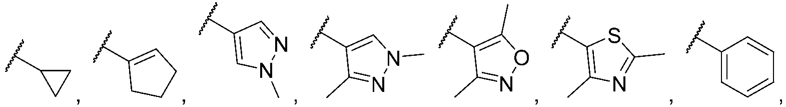

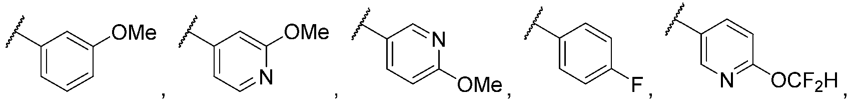

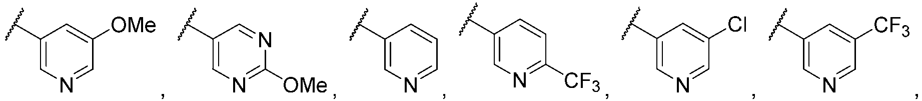

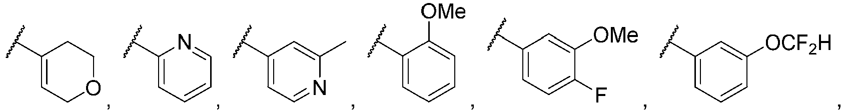

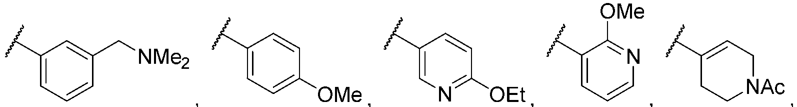

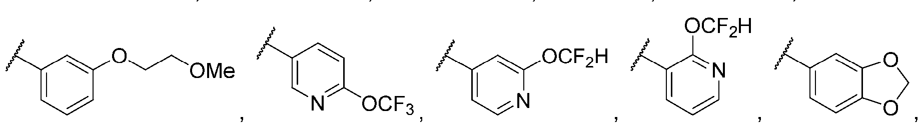

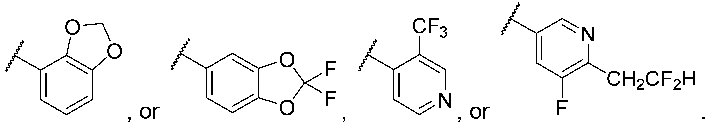

In a second aspect of the invention there is provided a compound of formula (II), or a salt thereof: 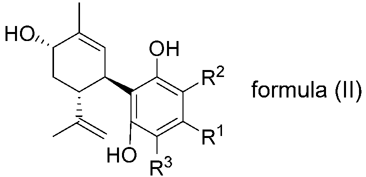 where:
R¹ is CH₂CH₂CH₃, Br, 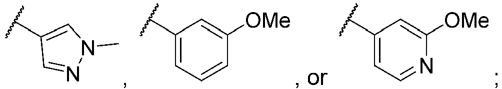
R² is CH₃, F or Cl; and
R³ is H or CH₃.

In a third aspect of the invention there is provided a compound of formula (III), or a salt thereof: 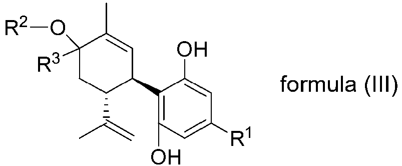 where:
R¹ is CH₂CH₂CH₃ or (CH₂)₄CH₃;
R² is H or CH₃; and
R³ is H or CH₃.

In a fourth aspect of the invention there is provided a compound of formula (IV), or a salt thereof: 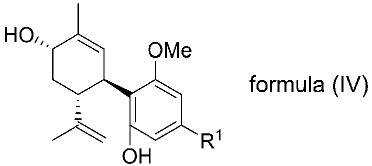 where:
R¹ is CH₂CH₂CH₃ or

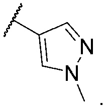

In a sixth aspect of the invention there is provided a pharmaceutical composition comprising a compound of the first, second, third, fourth or fifth aspects, or a salt thereof, together with one or more ingredients selected from carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

In a preferred embodiment, the pharmaceutical composition of the sixth aspect is in a form selected from a liquid, a solution, a suspension, an emulsion, a syrup, an electuary, a mouthwash, a drop, a tablet, a granule, a powder, a lozenge, a pastille, a capsule, a cachet, a pill, an ampoule, a bolus, a suppository, a pessary, a tincture, a gel, a paste, an ointment, a cream, a lotion, an oil, a foam, a spray, and an aerosol.

In a seventh aspect of the invention there is provided a compound of the first, second, third, fourth or fifth aspects, or a salt thereof, or a pharmaceutical composition of the sixth aspect, for use in a method of treatment.

Preferably, the method of treatment of the seventh aspect is a method of treatment of epilepsy, generalised seizure, focal onset seizure or tonic-colic seizure.

In an eighth aspect of the invention there is provided a compound of the first, second, third, fourth or fifth aspects, or a salt thereof, or a pharmaceutical composition of the sixth aspect, for use as a medicament.

Preferably, the medicament of the eighth aspect is a medicament for treating epilepsy, generalised seizure, focal onset seizure or tonic-colic seizure.

In a ninth aspect of the invention there is provided a method of treatment comprising administering to a subject in need of treatment a therapeutically effective amount of the compound of the first, second, third, fourth or fifth aspects, or a salt thereof, or the pharmaceutical composition of the sixth aspect.

In a tenth aspect of the invention there is provided a method of preparing a compound of formula (I), the method comprising:
i) reacting a compound of formula (V) with a compound of formula (VI): 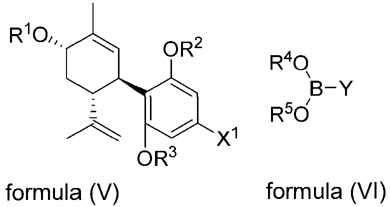 where:
   R¹ is H, Ac, Piv, Bz or PMB;
   R² and R³ are independently H, Ac or Piv;
   R⁴ and R⁵ are OH, or R⁴ and R⁵ together form -C(Me)₂C(Me)₂C-;
   X¹ is Br I or OTf; and
   Y is selected from:

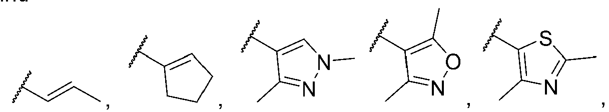

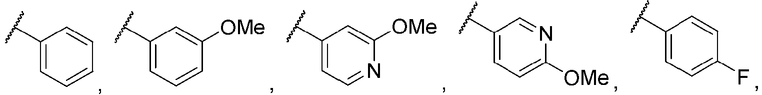

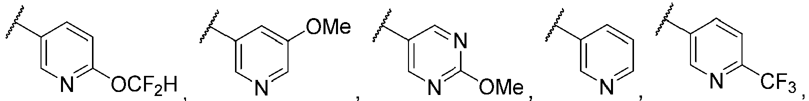

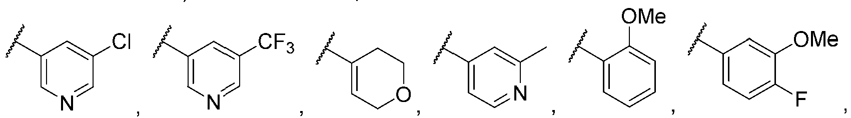

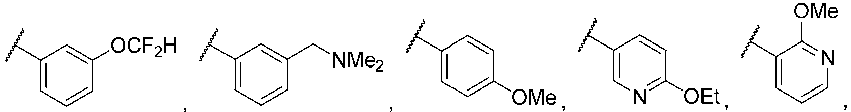

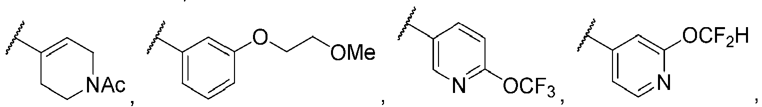

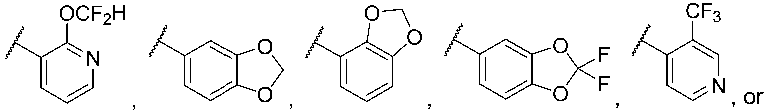

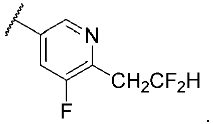

In an eleventh aspect of the invention there is provided an intermediate selected from compounds of formula (V) and (IX): 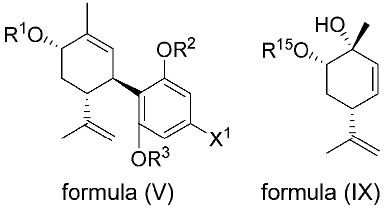 where:
R¹ is H, Ac, Piv, Bz or PMB;
R² and R³ are independently H, Ac or Piv;
X¹ is Br, I or OTf; and
R¹⁵ is H, Bz or PMB.

These and other aspects and embodiments of the invention are described in further detail below.

### BRIEF SUMMARY OF THE DRAWINGS

There present invention is described with reference to the figures listed below:
**Figure 1** shows the effect of compounds **2, 46** and **56** in the mini-MEST test in the mouse.
**Figure 2** shows the effect of compound **3** in the mini-MEST test in the mouse.
**Figure 3** shows the effect of compounds **5** and **10** in the mini-MEST test in the mouse.
**Figure 4** shows the effect of compounds **8** and **18** in the mini-MEST test in the mouse.
**Figure 5** shows the effect of compounds **15, 16, 45** and **53** in the mini-MEST test in the mouse.
**Figure 6** shows the effect of compound **17** in the mini-MEST test in the mouse.
**Figure 7** shows the effect of compound **19, 41** and **58** in the mini-MEST in the mouse.

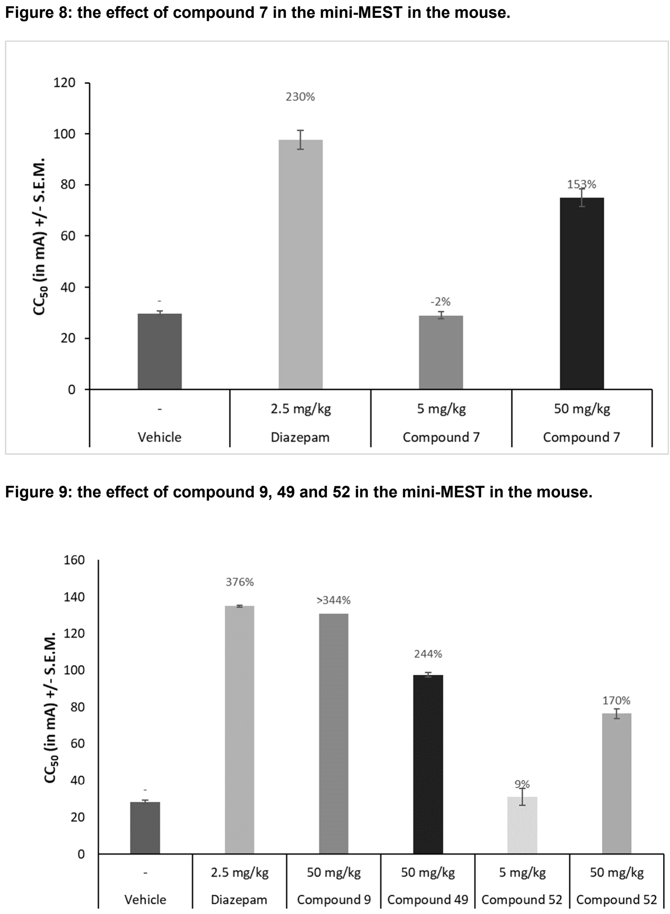

**Figure 8** shows the effect of compound 7 in the mini-MEST in the mouse.
**Figure 9** shows the effect of compound **9, 49** and **52** in the mini-MEST in the mouse.

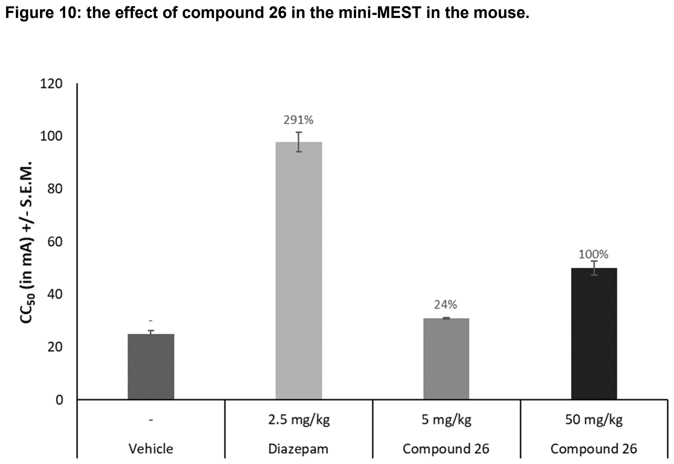

**Figure 10** shows the effect of compound **26** in the mini-MEST in the mouse.

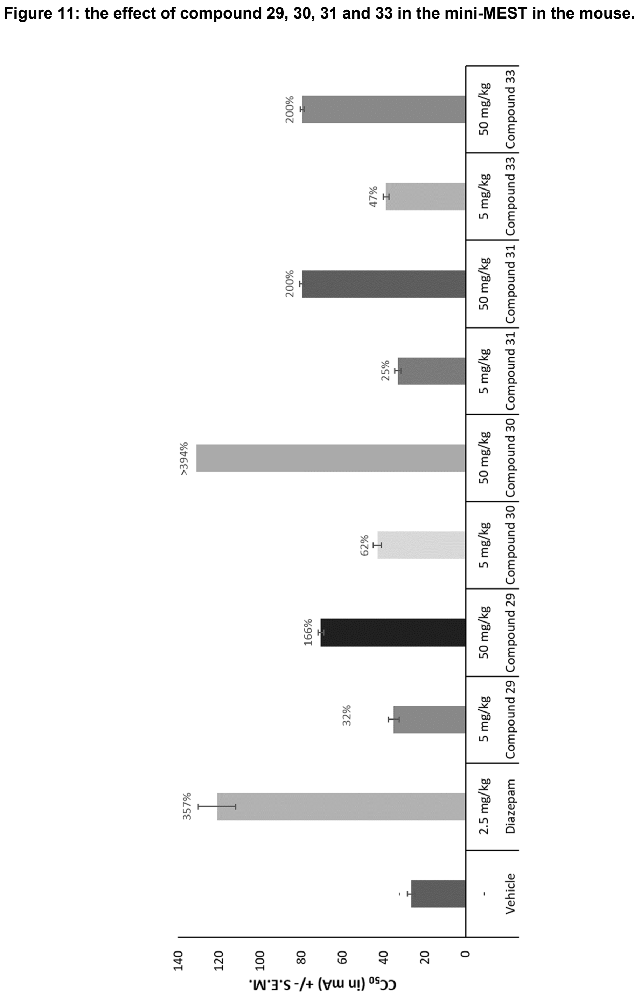

**Figure 11** shows the effect of compound **29, 30, 31** and **33** in the mini-MEST in the mouse.

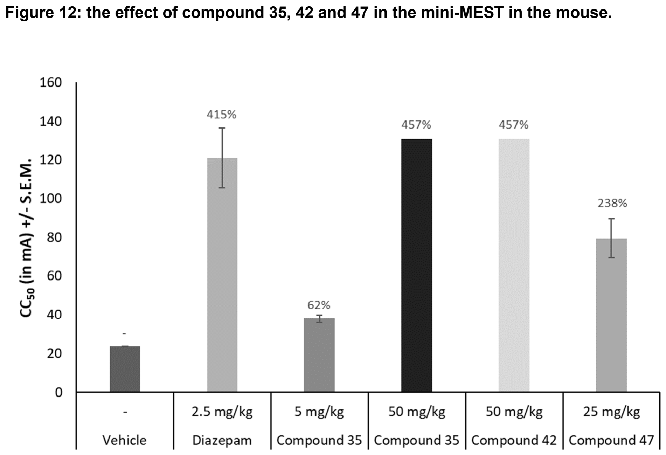

**Figure 12** shows the effect of compound **35, 42** and **47** in the mini-MEST in the mouse.

### DETAILED DESCRIPTION OF THE INVENTION

The present invention relates to invention relates to a group of resorcinol compounds which are biologically active and hence useful in the treatment of diseases.

### Resorcinols

### Formula (I)

In a first aspect of the invention there is provided a compound of formula (I) or a salt thereof: 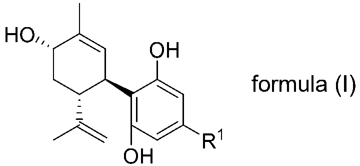 where:
R¹ is CH₃, CH₂CH₃, CI,

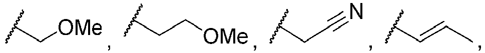

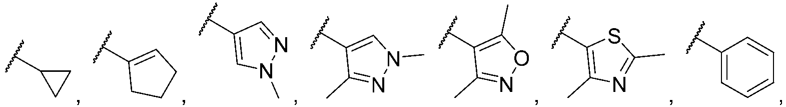

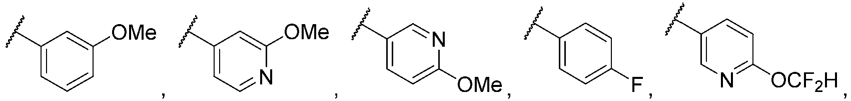

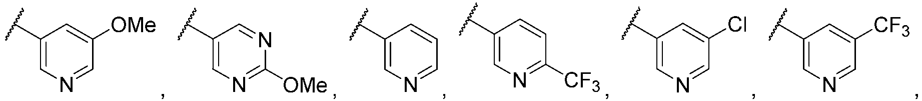

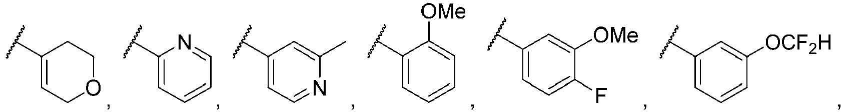

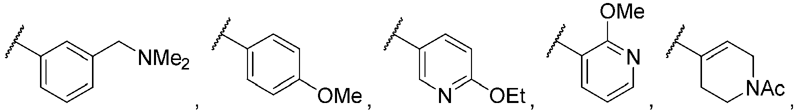

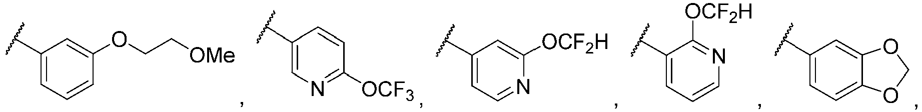

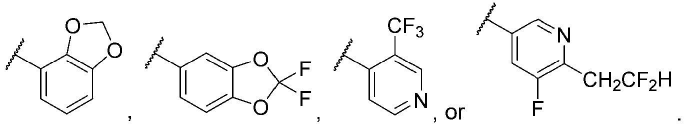

In preferred embodiments, R¹ is CH₃, CH₂CH₃, Cl,

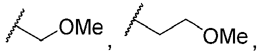

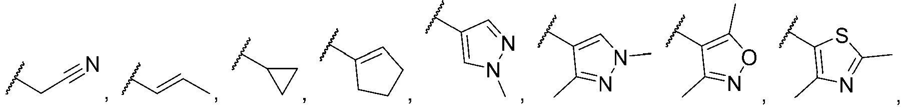

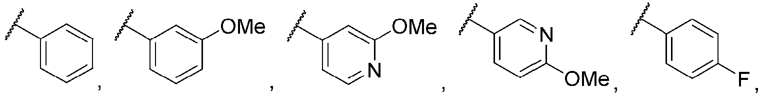

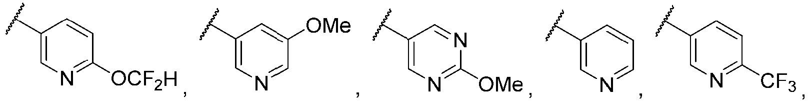

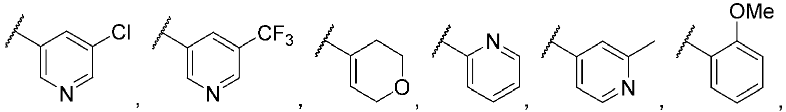

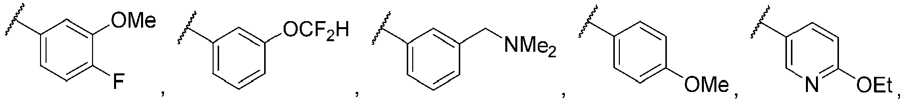

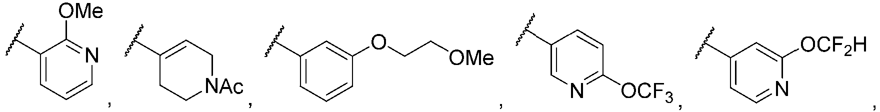

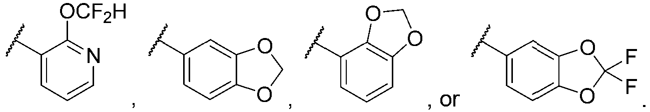

In more preferred embodiments, R¹ is CH₂CH₃, Cl,

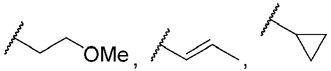

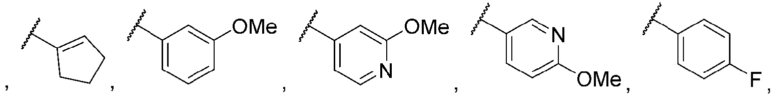

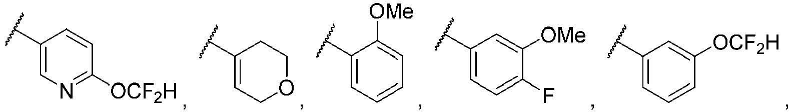

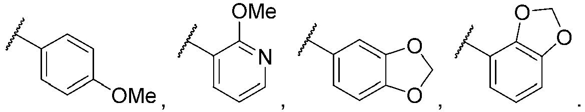

In even more preferred embodiments, R¹ is Cl,

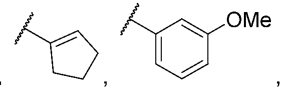

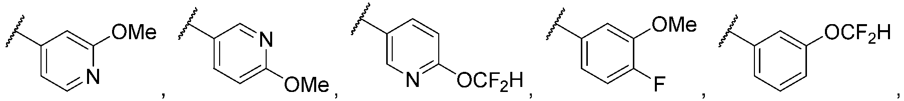

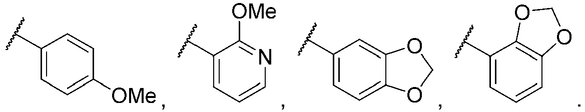

### Formula (II)

In a second aspect of the invention there is provided a compound of formula (II), or a salt thereof: 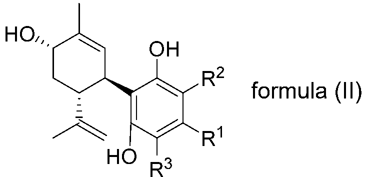 where:
R¹ is CH₂CH₂CH₃, Br, 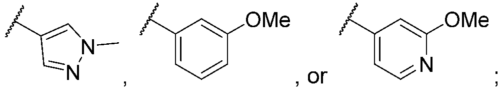
R² is CH₃, F or Cl; and
R³ is H or CH₃.

In preferred embodiments, R¹ is CH₂CH₂CH₃, Br, 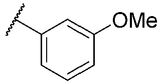 or

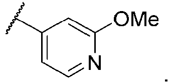

In preferred embodiments, R² is CH₃ or Cl.

In preferred embodiments, R³ is H.

### Formula (III)

In a third aspect of the invention there is provided a compound of formula (III), or a salt thereof: 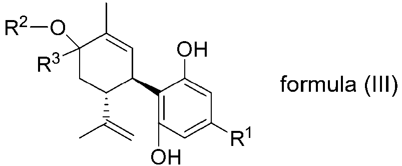 where:
R¹ is CH₂CH₂CH₃ or (CH₂)₄CH₃;
R² is H or CH₃; and
R³ is H or CH₃.

In preferred embodiments, R' is CH₂CH₂CH₃.

### Formula (IV)

In a fourth aspect of the invention there is provided a compound of formula (IV), or a salt thereof: 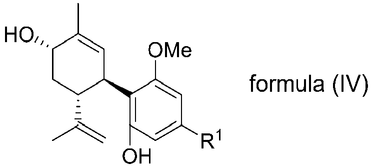 where:
R¹ is CH₂CH₂CH₃ or

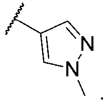

In preferred embodiments, R¹ is CH₂CH₂CH₃.

### Salts

In some embodiments, the compounds of formula (I), (II), (III) or (IV) are provided in free base form.

Alternatively, it may be convenient or desirable to prepare, purify, and/or handle a corresponding salt of the compound, for example, a pharmaceutically-acceptable salt. Examples of pharmaceutically acceptable salts are discussed in "Pharmaceutical Salts: Properties, Selection, and Use", 2nd Edition, 2002, Stahl and Wermuth (Eds), Wiley-VCH, Weinheim, Germany.

Accordingly, in some embodiments the compounds of formula (I), (II), (III) or (IV) are provided as salts, for example in a protonated form together with a suitable counter anion.

Suitable counter anions include both organic and inorganic anions. Example of suitable inorganic anions include those derived from inorganic acids, including chloride (Cl⁻), bromide (Br⁻), iodide (I⁻), sulfate (SO₄²⁻), sulfite (SO₃²⁻), nitrate (NO₃⁻), nitrite (NO₂⁻), phosphate (PO₄³⁻), and phosphite (PO₃³⁻). Examples of suitable organic anions include 2-acetoxybenzoate, acetate, ascorbate, aspartate, benzoate, camphorsulfonate, cinnamate, citrate, edetate, ethanedisulfonate, ethanesulfonate, formate, fumarate, gluconate, glutamate, glycolate, hydroxymalate, carboxylate, lactate, laurate, lactate, maleate, malate, methanesulfonate, oleate, oxalate, palmitate, phenylacetate, phenylsulfonate, propionate, pyruvate, salicylate, stearate, succinate, sulfanilate, tartarate, toluenesulfonate, and valerate. Examples of suitable polymeric organic anions include those derived from tannic acid and carboxymethyl cellulose.

Alternatively, in some embodiments the compounds of formula (I), (II), (III) or (IV) are provided as salts, for example in a deprotonated form together with a suitable counter cation.

Suitable counter cations include both organic and inorganic cations. Examples of suitable inorganic cations include alkali metal ions such as Na⁺ and K⁺, alkaline earth cations such as Ca²⁺ and Mg²⁺, and other cations such as Al³⁺. Examples of suitable organic cations include the ammonium ion (i.e., NH₄⁺) and substituted ammonium ions (e.g., NH₃R⁺, NH₂R₂⁺, NHR₃⁺, NR₄⁺). Examples of substituted ammonium ions include those derived from ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

### Solvates

In some embodiments, the compounds of formula (I), (II), (III) or (IV) are provided in desolvated form, for example, in dehydrated form.

Alternatively, it may be convenient or desirable to prepare, purify, and/or handle a corresponding solvate of the compound.

Accordingly, in some embodiments the compounds of formula (I), (II), (III) or (IV) are provided in the form of a solvate (a complex of solute (e.g., compound, salt of compound) and solvent). Examples of solvates include hydrates, for example, a monohydrate, a di-hydrate and a tri-hydrate.

### Certain Isomers

Certain compounds of formula (I), (II), (III) or (IV) may exist in one or more particular optical, enantiomeric, diasteriomeric, epimeric, stereoisomeric, tautomeric, or conformational forms, including but not limited to, D- and L-forms; d- and l-forms; (+) and (-) forms; syn- and anti-forms; axial and equatorial forms; boat-, chair-, twistboat-, envelope-, and halfchairforms; and combinations thereof, hereinafter collectively referred to as "isomers" or "isomeric forms".

Specifically excluded from the term "isomers," as used herein, are structural (or constitutional) isomers (i.e., isomers which differ in the connections between atoms rather than merely by the position of atoms in space). For example, a reference to a methoxy group, -OCH₃, is not to be construed as a reference to its structural isomer, a hydroxymethyl group, -CH₂OH.

The above exclusion does not pertain to tautomeric forms, for example, keto-, enol-, and enolate-forms, as in, for example, the following tautomeric pairs: keto/enol, imine/enamine, amide/imino alcohol, nitroso/oxime, and lactam/lactim.

Included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including (wholly or partially) racemic and other mixtures thereof.

### Methods of Synthesis

### Synthesis of Resorcinols

PCT/GB2020/052942 discloses a method of synthesising a 6-hydroxy analogue of cannabidiol (CBD). PCT/GB2020/052944 discloses a method of synthesising a 6-hydroxy analogue of cannabidivarin. Both methods involve the oxidation of the natural cannabinoid using a chromium oxidising agent.

The inventors have found an improved synthetic method that allows the production of the 6-hydroxy compounds with greater yield and reliability. Moreover, the method of the invention allows for the use of a late-stage cross coupling reaction to install a variety of functional groups at the resorcinol tail portion. Finally, the method avoids the use of toxic, chromium-based oxidising agents.

### Coupling Step (step i):

The invention provides a method of preparing a compound of formula (I), the method comprising:
i) reacting a compound of formula (V) with a compound of formula (VI): 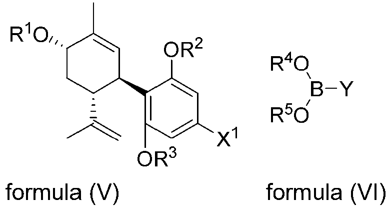 where:
   R¹ is H, Ac, Piv, Bz or PMB;
   R² and R³ are independently H, Ac or Piv;
   R⁴ and R⁵ are OH, or R⁴ and R⁵ together form -C(Me)₂C(Me)₂C-;
   X¹ is Br, I or OTf; and
   Y is selected from:

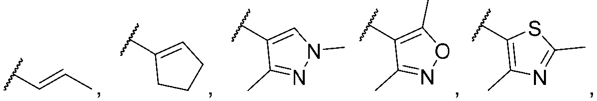

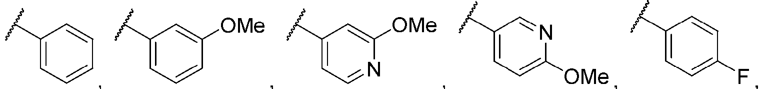

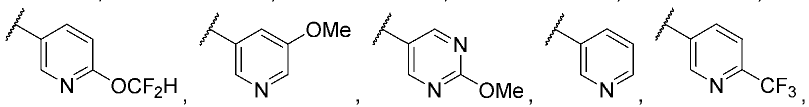

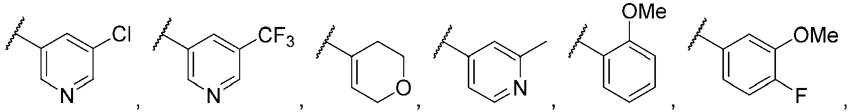

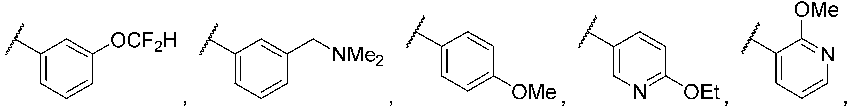

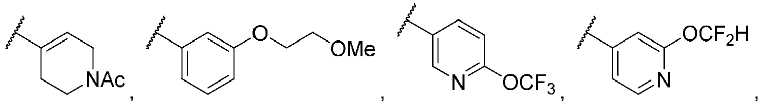

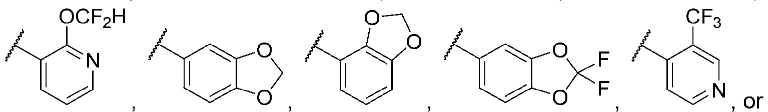

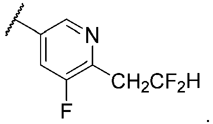

Step (i) may be known as the coupling step.

In preferred embodiments, R¹ is H, Ac or Piv. In more preferred embodiments, R¹ is H.

In preferred embodiments, R² and R³ are H.

In preferred embodiments, X¹ is Br or OTf. In more preferred embodiments, X¹ is OTf.

Typically, step (i) comprises reacting a compound of formula (V) with a compound of formula (VI) and a palladium catalyst. Suitable palladium catalysts include Pd(dppf)Cl₂, Pd(PPh₃)₄, and SPhos-Pd-G2 (Chloro(2-dicyclohexylphosphino-2',6'-dimethoxy-1,1'-biphenyl)[2-(2'-amino-1,1'-biphenyl)]palladium(II)).

In preferred embodiments, step (i) further comprises reacting a compound of formula (V) with a compound of formula (VI) and a base. Suitable bases include caesium fluoride (CsF), sodium carbonate (Na2CO3), caesium carbonate (Cs2CO3)

Typically, step (i) is carried out in a solvent. Suitable solvents include dioxane, tetrahydrofuran (THF), dimethylformamide (DMF), water and mixtures thereof.

Step (i) is typically performed at elevated temperature (above ambient temperature; approximately 20 °C). Methods for providing heat during the reaction are known and include, for example, using a reaction vessel having an external heating jacket or using microwave heating.

Typically, step (i) comprises reacting a compound of formula (V) with a compound of formula (VI) at a temperature of from 60 °C to 140 °C, preferably 80 °C to 140 °C, more preferably 80 °C to 120 °C.

Step (i) may be performed for sufficient time to allow a desired quantity of the coupling product to form. Typically, the step (i) is performed until substantially all of the compound of formula (QQ) has been consumed.

Typically, the step (1a) comprises reacting a compound of formula (II) with a compound of formula (III) for 1 hour to 12 hours.

### Triflation Step (step ii):

The invention also provides a method of preparing a compound of formula (V), the method comprising:
ii) reacting a compound of formula (VII) with a triflation reagent: 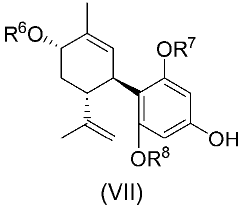 where:
R⁶ is H, Bz or PMB;
R⁷ and R⁸ are independently H, Ac or Piv.

Step (ii) may be known as the triflation step.

In preferred embodiments, R⁶ is H or Bz. In more preferred embodiments, R⁶ is Bz.

In preferred embodiments, R⁷ and R⁶ are OH.

Suitable triflation reagents include trifluoromethanesulfonic anhydride (Tf₂O), N-phenylbis(trifluoromethanesulfonimide) (PhNTf₂), Comins' reagent, 1-(trifluoromethanesulfonyl)-1H-benzotriazole, 1-(trifluoromethanesulfonyl)imidazole and N-(2-pyridyl)-bis(trifluoromethanesulfonimide). In preferred embodiments, Tf₂O or PhNTf₂ are use. In more preferred emdbodiments, PhNTf₂is used.

In preferred embodiments, step (ii) comprises reacting a compound of formula (VII) with a triflation reagent and a base. Suitable bases include organic bases such as pyridine, leutidine, dimethylbenzylamine imidazole, benzimidazole, methylimidazole, triethylamine, tributylamine, diisopropylethylamine, tetramethylethylenediamine, DABCO. In preferred embodiments, triethylamine or lutidine is used. In more preferred embodiments, triethylamine is used.

Typically, step (ii) is carried out in a solvent. Suitable solvents include dichloromethane.

Step (ii) is typically performed at lowered temperature (below ambient temperature; approximately 20 °C). Methods for removing heat during the reaction are known and include, for example, using a reaction vessel having an external cooling jacket.

Typically, step (ii) comprises reacting a compound of formula (VII) with a triflation reagent at a temperature of from -40 °C to 25 °C, preferably -5 °C to 20 °C, more preferably 0 °C to 20 °C.

Step (ii) may be performed for sufficient time to allow a desired quantity of the triflation product to form. Typically, the step (ii) is performed until substantially all of the compound of formula (VII) has been consumed.

Optionally, step (ii) may further comprise:
ii-a) reacting a compound of formula (VIII) with a halogen exchange reagent 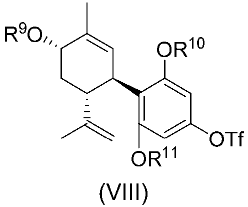 where:
R⁹ is independently H, Bz or PMB;
R¹⁰ and R¹¹ are independently H, Ac or Piv.

In preferred embodiments, R⁹ is Bz or PMB. In more preferred embodiments, R⁹ is Bz.

In preferred embodiments, R¹⁰ and R¹¹ are Ac or Piv. In more preferred embodiments R¹⁰ and R¹¹ Piv.

Step (ii-a) may take place over one (direct halogen exchange) or multiple (indirect halogen exchange) steps.

Suitable halogen exchange reagents for direct halogen exchange include [Cp*Ru(MeCN)₃OTf] in combination with lithium bromide.

Suitable halogen exchange reagents for indirect halogen exchange include B₂Pin₂ in combination with a palladium catalyst such as Pd(POAc)₂, and optionally a ligand such as dppf, followed by copper(ll) bromide. Typically, elevated temperatures are used, such as from 60 °C to 90 °C. Indirect halogen exchange may proceed through an intermediate of formula (VIIIa): 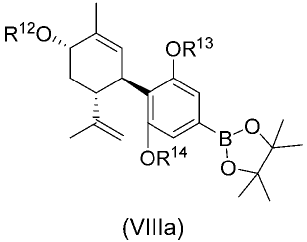 where:
R¹² is independently H, Bz or PMB;
R¹³ and R¹⁴ are independently H, Ac or Piv.

### Friedel Crafts (step iii):

The invention also provides a method of preparing a compound of formula (VII), the method comprising:
iii) reacting a compound of formula (IX) with phloroglucinol (benzene-1,3,5-triol) 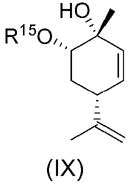 where:
R¹⁵ is H, Bz or PMB.

Step (iii) may be known as the Friedel Crafts step.

In preferred embodiments, R¹⁵ is Bz or PMB. In more preferred embodiments, R¹⁵ is Bz.

In preferred embodiments, step (iii) comprises reacting a compound of formula (IX) with phloroglucinol and an acid. Suitable acids Lewis acids such as boron trifluoride and Bronsted acids such as triflic acid. In preferred embodiments, triflic acid is used.

Step (iii) is typically performed at lowered temperature (below ambient temperature; approximately 20 °C). Typically, step (ii) comprises reacting a compound of formula (IX) with phloroglucinol at a temperature of from -15 °C to 20 °C, preferably 0 °C to 15 °C, more preferably 5 °C to 15 °C.

Typically, step (ii) is carried out in a solvent. Suitable solvents include tetrahydrofuran and isopropyl acetate.

### Preferred embodiments:

In a preferred embodiment, there is provided a method comprising:
iii) a Friedel Crafts step as set out in paragraphs [0079] to [0084];
ii) a triflation step, as set out in paragraphs [0063] to [0078]; and optionally
i) a coupling step, as set out in paragraphs [0051] to [0062].

### Synthesis of Key Intermediate

The inventors have found the 6-hydroxy compounds can be produced by a Friedel Crafts reaction from key intermediate of formula (IX). This method provides the compounds with greater yield and reliability in comparison to a late-stage oxidation method. Moreover, the method of the invention allows for the use of a late-stage cross coupling reaction to install a variety of functional groups at the resorcinol tail portion.

### Allylic Oxidation (step iv):

The invention also provides a method for preparing a compound of formula (IX), the method comprising:
iv-a) reacting a compound of formula (X) with diphenyl diselenide to produce a compound of formula (XI); and
iv-b) reacting the compound of formula (XI) with an oxidising agent, 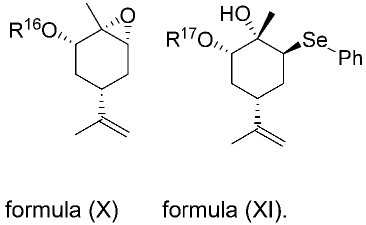 where:
   R¹⁶ and R¹⁷ are independently H, Bz or PMB.

Step (iv-a) and (iv-b) may together be known as the allylic oxidation step.

In preferred embodiments, step (iv-a) comprises reacting a compound of formula (X) with diphenyl diselenide and a reducing agent. Suitable reducing agents include sodium borohydride.

In preferred embodiments, step (iv-a) comprises reacting a compound of formula (X) with diphenyl diselenide and a base. Suitable base include sodium hydroxide.

Suitable oxidising agents include hydrogen peroxide. In preferred embodiments, hydrogen peroxide is used.

Step (iv-b) may be performed at elevated temperature (above ambient temperature; approximately 20 °C). Typically, step (iv-b) comprises reacting a compound of formula (XI) with an oxidising agent at a temperature of from 0 °C to 20 °C for an initial period (selenium oxidation), and then raising the temperature to from 20 °C to 60 °C for a second period (allylic oxidation).

Typically, step (iv-a) and step (iv-b) are carried out in a solvent. Suitable solvents include tetrahydrofuran, 2-mthyl tetrahydrofuran and ethanol.

### Epoxidation (step v):

The invention also provides a method for preparing a compound of formula (X), the method comprising:
v) reacting a compound of formula (XII), which may be known as (+)-cis-carveol, with a peroxycarboxylic acid (peracid)

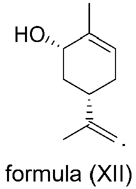

Step (v) may be known as the epoxidation step.

Suitable peroxycarboxylic acids include meta-chloroperoxybenzoic acid (mCPBA) and perbenzoic acid. In preferred emdboiments, mCPBA is used.

Step (v) is typically performed at lowered temperature (below ambient temperature; approximately 20 °C). Typically, step (v) comprises reacting a compound of formula (QQ) with a peroxycarboxylic acid at a temperature of from -40 °C to 0 °C, preferably -20 °C to 0 °C.

Typically, step (v) is carried out in a solvent. Suitable solvents include dichloromethane.

Step (v) produces a compound of formula (Xa), which may be known as epoxy-carveol

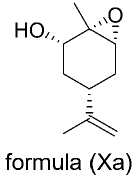

Optionally, step (v) may further comprise:
v-a) reacting a compound of formula (Xa) with p-methoxybenzyl chloride or benzoyl chloride.

In preferred embodiments, step (v-a) further comprises reacting a compound of formula (Xa) with p-methoxybenzyl chloride or benzoyl chloride and a base. Suitable bases include sodium hydride and triethylamine.

### Reduction (step vi):

The invention also provides a method for preparing a compound of formula (Xa), the method comprising:
vi) reacting S-carvone with a reducing agent,

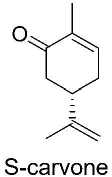

Step (vi) may be know as the reduction step.

Suitable reducing agents include lithium aluminium hydride, sodium bis(2-methoxyethoxy)aluminium hydride (red-AI), diborane and sodium borohydride. In preferred embodiments, sodium borohydride is used.

In some preferred, optional additives such as cerium(III) chloride are used.

Step (vi) is typically performed at lowered temperature (below ambient temperature; approximately 20 °C). Typically, step (v) comprises reacting S-carvone with a reducing agent at a temperature of from -80 °C to 20 °C, preferably -40 °C to 0 °C, more preferably -20 °C to -10 °C.

Typically, step (v) is carried out in a solvent. Suitable solvents include tetrahydrofuran, 2-methyl tetrahydrofuran, ethanol and methanol.

### Preferred embodiments:

In a preferred embodiment, there is provided a method comprising:
vi) a reduction step, as set out in paragraphs to [0102] to [0107];
v) an epoxidation step, as set out in paragraphs [0094] to [0101]; and optionally
iv) an allylic oxidation step, as set out in paragraphs [0087] to [0093].

### Other embodiments:

In one embodiment, there is provided a process for the production of a compound of formula (I) to (IV) comprising the following steps:
i) Reducing carvone a to carveol b followed by epoxidation to produce intermediate c and reaction with any suitable protecting group to produce the corresponding epoxide **d;**
ii) Reacting epoxide **d** with diphenyl diselenide in sodium borohydride and treating with hydrogen peroxide to give the crude **e,** and subsequent heating of **e** to form corresponding allylic alcohol **f;** or alternatively Refluxing epoxide **d** in the presence of dimethylamine to give crude **g,** subsequently oxidising **g** to the N-oxide **h** by treatment with hydrogen peroxide, and refluxing to provide **f;** and
iii) Reacting allylic alcohol **f** with phloroglucinol **i** to give compound **j,** treating **j** with acetic anhydride, followed by triflic anhydride in the presence of 2,6-lutidine to produce compound **k,** followed by bromination of **k** to give **I,** deprotection of **l** using cerium (IV) ammonium nitrate followed by sodium borohydride to give **m,** followed by subsequent metal catalysed coupling using **k** or **m** to access compounds of Formulae I to V; or alternatively Reacting allylic alcohol **f** and phenol **n** to give product **o,** decarboxylation of **o** in the presence of aqueous sodium hydroxide, followed by treatment with citric acid to give phenol **p,** deprotection of the protecting group using cerium (IV) ammonium nitrate to produce compounds of Formulae I to V.

Preferably the embodiment in paragraph [0109] uses one of the protecting groups: PMB, silicon based protecting groups (TMS, TES, TBS, TBDPS, SEM), ethers (MOM, MEM, BOM, Bn, PMB, DMB, allyl), esters (Piv, Bz, Ac), carbamates and carbonates (Troc, dimethylaminocarbamate) or no protecting group.

In one embodiment, there is provide a method comprising:
vi) a reduction step, as set out in paragraphs to [0102] to [0107];
v) an epoxidation step, as set out in paragraphs [0094] to [0101];
iv) an allylic oxidation step, as set out in paragraphs [0087] to [0093].
iii) a Friedel Crafts step as set out in paragraphs [0079] to [0084];
ii) a triflation step, as set out in paragraphs [0063] to [0078]; and optionally
i) a coupling step, as set out in paragraphs [0051] to [0062].

### Intermediates

The invention also provides an intermediate selected from compounds of formula (V) and (IX): 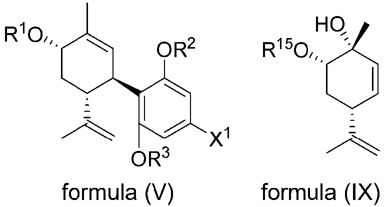 where:
R¹ is H, Ac, Piv, Bz or PMB;
R² and R³ are independently H, Ac or Piv;
X¹ is Br, I or OTf; and
R¹⁵ is H, Bz or PMB.

In a preferred embodiment, R¹ is H, Ac or Piv. In more preferred embodiments, R¹ is H.

In preferred embodiments, R² and R³ are H.

In preferred embodiments, X¹ is Br or OTf. In more preferred embodiments, X¹ is OTf.

In preferred embodiments, R¹⁵ is Bz or PMB. In more preferred embodiments, R¹⁵ is Bz.

In more preferred embodiments, the intermediate is selected from:

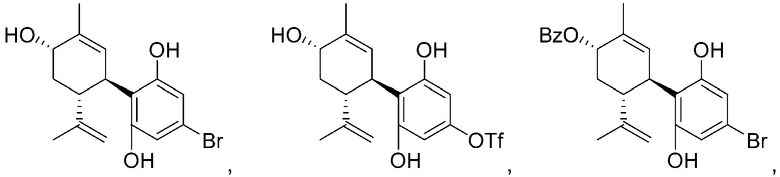

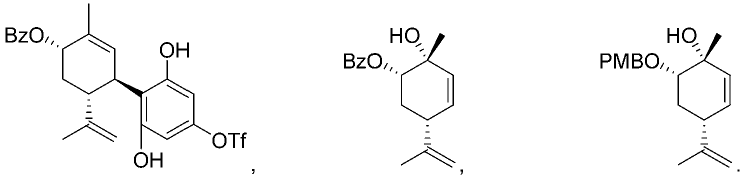

### Pharmaceutical Compositions

While it is possible for the compound of formula (I), (II), (III) or (IV) to be administered alone, it is preferable to administer a pharmaceutical composition (e.g., a formulation, preparation, or medicament) comprising a compound of formula (I), (II), (III) or (IV) together with one or more other pharmaceutically acceptable ingredients.

Accordingly, the invention provides a pharmaceutical composition comprising a compound of formula (I), (II), (III) or (IV), or a salt thereof, together with one or more pharmaceutically acceptable ingredients.

Suitable pharmaceutically acceptable ingredients (e.g. carriers, diluents, excipients, etc.) can be found in standard pharmaceutical texts, for example, Remington: The Science and Practice of Pharmacy, 20th Edition, 2000, pub. Lippincott, Williams & Wilkins; and Handbook of Pharmaceutical Excipients, 9th edition, 2020, pub. Pharmaceutical Press.

Examples of suitable pharmaceutically acceptable ingredients include pharmaceutically acceptable carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

In preferred embodiments, the pharmaceutical compositions comprises one or more of: an excipient selected among a carrier, an oil, a disintegrant, a lubricant, a stabilizer, a flavouring agent, an antioxidant, a diluent and another pharmaceutically effective compound

The pharmaceutical composition may be in any suitable form. Examples of suitable forms include liquids, solutions (e.g., aqueous, nonaqueous), suspensions (e.g., aqueous, non-aqueous), emulsions (e.g., oil-in-water, water-in-oil), syrups, electuaries, mouthwashes, drops, tablets (including, e.g., coated tablets), granules, powders, losenges, pastilles, capsules (including, e.g., hard and soft gelatin capsules), cachets, pills, ampoules, boluses, suppositories, pessaries, tinctures, gels, pastes, ointments, creams, lotions, oils, foams, sprays, and aerosols.

In preferred embodiments, the pharmaceutical composition is selected from a tablet, a capsule, a granule, an oral solution, a powder for inhalation, a sprinkle, an oral solution and a suspension

### Medical Treatment

The inventors have found that compounds of formula (I), (II), (III) and (IV) are biologically active. The worked examples demonstrate that compound of formula (I), (II), (III) and (IV) display anticonvulsant activity in a mouse model of seizure. As such, the compounds of formula (I), (II), (III) and (IV), and their salts, as well as pharmaceutical compositions comprising the compounds of formula (I), (II), (III) or (IV), or their salts, will be useful in medical treatment.

Accordingly, the invention provides a compound of formula (I), (II), (III) or (IV), or a salt thereof, for use in a method of treatment, for example for use in a method of treatment of the human or animal body by therapy (i.e. a method of therapy).

The invention also provides a compound of formula (I), (II), (III) or (IV), or a salt thereof, for use as a medicament.

The invention also provides a method of treatment comprising administering to a subject in need of treatment a therapeutically effective amount of a compound of formula (I), (II), (III) or (IV), or a salt thereof.

The invention also provides the use of a compound of formula (I), (II), (III) or (IV), or a salt thereof, for the manufacture of a medicament.

The invention also provides use of a compound of formula (I), (II), (III) or (IV), or a salt thereof, in a method of treatment.

### Conditions Treated

The inventors have found that the compounds of formula (I), (II), (III) and (IV) display anticonvulsant activity in a mouse model of generalised seizure. Accordingly, the compounds of formula (I), (II), (III) and (IV), their salts, as well as pharmaceutical compositions comprising compounds of formula (I), (II), (III) and (IV), or their salts, will be useful in the treatment of certain conditions associated with seizure.

Similarly, the compounds of formula (I), (II), (III) and (IV), their salts, as well as pharmaceutical compositions comprising a compound of formula (I), (II), (III) and (IV), or their salts, will be useful as medicaments for treating (and in the manufacture of medicaments for treating) certain conditions associated with seizure.

In a preferred embodiment, the condition associated with seizure is epilepsy.

In one embodiment, the condition associated with seizure is generalised seizure, such as generalised seizure associated with epilepsy.

In one embodiment, the condition associated with seizure is focal-onset seizures, such as focal-onset seizure associated with epilepsy.

In one embodiment, the condition associated with seizure is tonic-clonic seizures, such as tonic-clonic seizures associated with epilepsy.

### The Subject/Patient

The method of treatment typically comprises administering a compound of formula (I), (II), (III) and (IV), or a salt thereof, to a subject or patient.

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orang-utan, gibbon), or a human.

The subject/patient may be any of its forms of development, for example, the subject/patient may be an infant or child.

In a preferred embodiment, the subject/patient is a human, more preferably an adult human.

The subject/patient may also be a non-human mammal used in laboratory research, such as a rodent. Rodents include rats, mice, guinea pigs and chinchillas.

### Routes of Administration

The method of treatment may comprise administering a compound of formula (I), (II), (III) or (IV), or a salt thereof, to a subject by any convenient route of administration, whether systemically/peripherally or topically (i.e., at the site of desired action).

The route of administration may be oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection or infusion, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; or by implant of a depot or reservoir, for example, subcutaneously or intramuscularly.

### Dosages

The method of treatment typically comprises administering a therapeutically effective amount of a compound of formula (I), (II), (III) or (IV), or a salt thereof, to a subject.

Appropriate dosages of the compounds of formula (I), (II), (III) and (IV), their salts, as well as pharmaceutical compositions comprising the compound of formula (I), (II), (III) or (IV), or their salts, can vary from patient to patient. Determining the optimal dosage will generally involve balancing the level of therapeutic benefit against any risk or deleterious side effects. The selected dosage level will depend on a variety of factors including, but not limited to, the activity of the particular compound of formula (I), (II), (III) or (IV), the route of administration, the time of administration, the rate of excretion of the compound, the duration of the treatment, other active agents, compounds, and/or materials used in combination, the severity of the condition, and the species, sex, age, weight, condition, general health, and prior medical history of the patient. The dosage and route of administration will ultimately be at the discretion of the clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Single or multiple administrations can be carried out with the dose level and pattern being selected by the treating clinician.

### Other Aspects and Embodiments

Each and every compatible combination of the embodiments described above is explicitly discloses herein, as if each and every combination was individually and explicitly recited.

Carious further aspects and embodiment of the present invention will be apparent to those skilled in the arti in view of the present disclosure.

Where used, "and/or" is to be taken as a specific disclosure of each of the relevant components or features alone as well as a specific disclosure of the combination of the components or features. For example, "A and/or B" is to be taken as specific disclosure of each of i) A, ii) B, and ii) A and B, just as if each were set out individually.

Unless context dictates otherwise, the descriptions and definitions of the features set out above are not limited to any particular aspect or embodiment of the invention and apply equally to all aspects ad embodiments which are described.

The invention may be defined according to any one of the following numbered clauses:
1. a compound of formula (I) or a salt thereof: 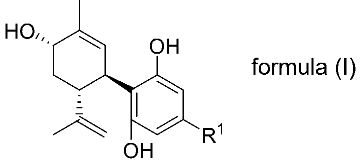 where:
   R¹ is CH₃, CH₂CH₃, Cl, 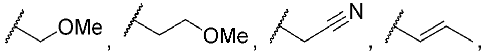 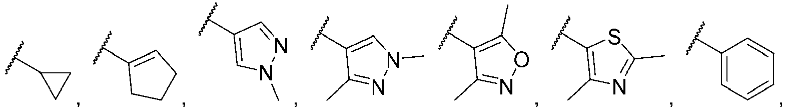 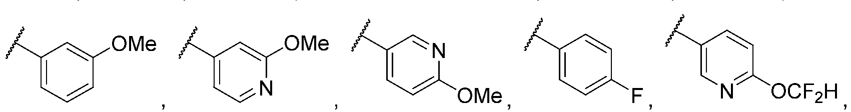 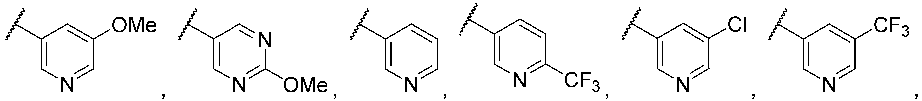 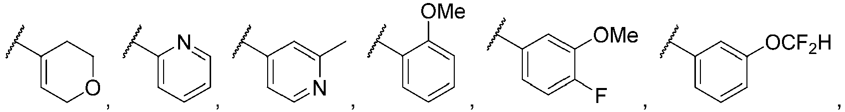 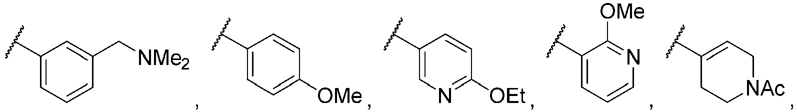 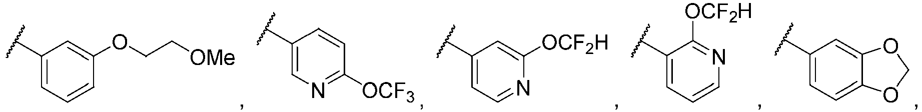 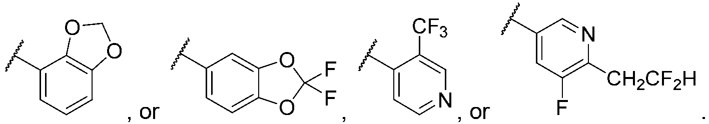
2. The compound of clause 1, where R¹ is CH₂CH₃, Cl, 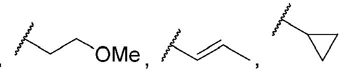 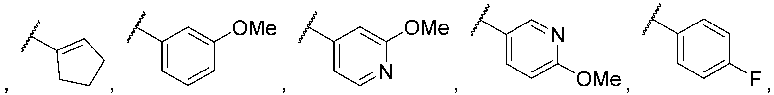 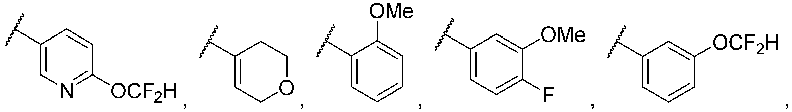 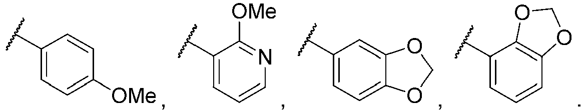
3. A compound of formula (II), or a salt thereof: 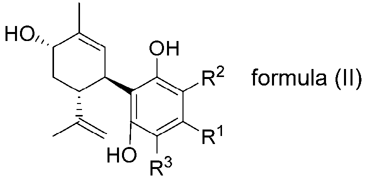 where:
   R¹ is CH₂CH₂CH₃, Br, 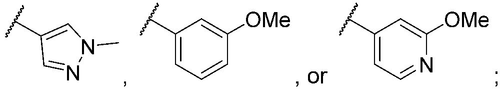
   R² is CH₃, F or Cl; and
   R³ is H or CH₃.
4. The compound of clause 3, where R¹ is CH₂CH₂CH₃.
5. The compound of clause 3 or 4, where R² is F or Cl.
6. The compound of any of clauses 3 to 5, where R³ is H.
7. a compound of formula (III), or a salt thereof: 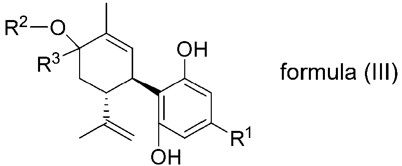 where:
   R¹ is CH₂CH₂CH₃ or (CH₂)₄CH₃;
   R² is H or CH₃; and
   R³ is H or CH₃.
8. The compound of clause 7, where R¹ is CH₂CH₂CH₃.
9. A compound of formula (IV), or a salt thereof: 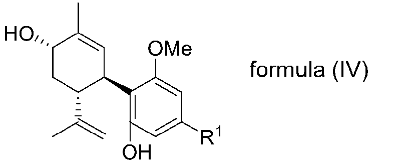 where:
   R¹ is CH₂CH₂CH₃ or 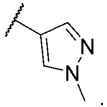
10. The compound of clause 9, where R¹ is CH₂CH₂CH₃.
11. A pharmaceutical composition comprising the compound of any of clauses 1 to 10 together with one or more additional ingredients selected from carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.
12. The pharmaceutical composition of clause 11 in a form selected from a liquid, a solution, a suspension, an emulsion, a syrup, an electuary, a mouthwash, a drop, a tablet, a granule, a powder, a lozenge, a pastille, a capsule, a cachet, a pill, an ampoule, a bolus, a suppository, a pessary, a tincture, a gel, a paste, an ointment, a cream, a lotion, an oil, a foam, a spray, and an aerosol.
13. The compound of any one of clauses 1 to 10, or the pharmaceutical composition of clauses 11 or 12, for use in a method of treatment.
14. The compound of any one of clauses 1 to 10, or the pharmaceutical composition of clauses 11 or 12, for use in a method of treating epilepsy.
15. The compound of any one of clauses 1 to 10, or the pharmaceutical composition of clauses 11 or 12, for use in a method of treating generalised seizure, focal onset seizure or tonic-colic seizure.
16. The compound of any one of clauses 1 to 10, or the pharmaceutical composition of clauses 11 or 12, for use as a medicament.
17. The compound of any one of clauses 1 to 10, or the pharmaceutical composition of clauses 11 or 12, for use as a medicament for treating epilepsy.
18. The compound of any one of clauses 1 to 10, or the pharmaceutical composition of clauses 11 or 12, for use as a medicament for treating generalised seizure, focal onset seizure or tonic-colic seizure.
19. A method of treatment comprising administering to a subject in need of treatment a therapeutically effective amount of the compound of any one of clauses 1 to 10, or the pharmaceutical composition of clauses 11 or 12.
20. A method of treating epilepsy comprising administering to a subject in need of treatment a therapeutically effective amount of the compound of any one clauses 1 to 10, or the pharmaceutical composition of clauses 11 or 12.
21. A method of treating generalised seizure, focal onset seizure or tonic-colic seizure comprising administering to a subject in need of treatment a therapeutically effective amount of the compound of any one of clauses 1 to 10, or the pharmaceutical composition of clauses 11 or 12.
22. A method of preparing a compound of formula (I), the method comprising:
   i) reacting a compound of formula (V) with a compound of formula (VI): 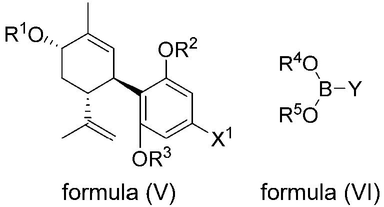 where:
      R¹ is H, Ac, Piv, Bz or PMB;
      R² and R³ are independently H, Ac or Piv;
      R⁴ and R⁵ are OH, or R⁴ and R⁵ together form -C(Me)₂C(Me)₂C-;
      X¹ is Br, I or OTf; and
      Y is selected from: 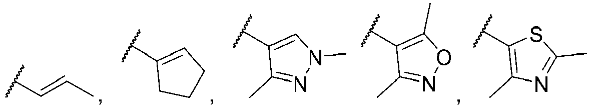 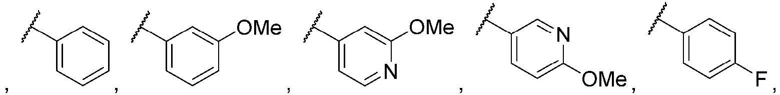 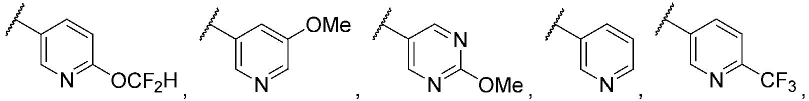 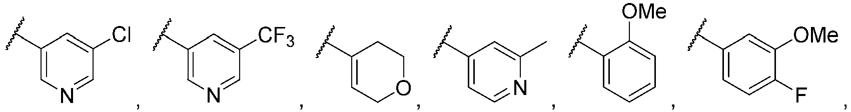 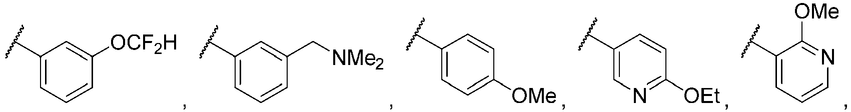 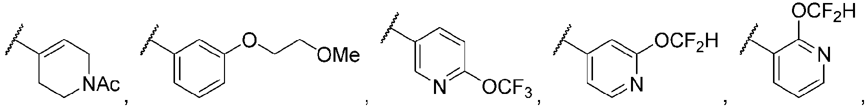 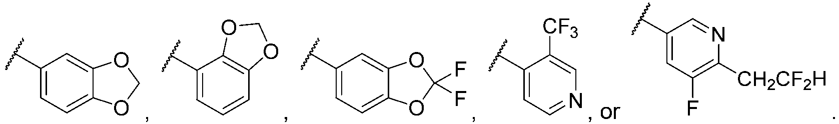
23. The method of clause 22, wherein R¹ is H, Ac or Piv.
24. The method of clause 22 or clause 23, wherein R² and R³ are H.
25. The method of any of clauses 22 to 24, wherein X¹ is Br or OTf.
26. The method of any of clauses 22 to 25, wherein step (i) comprises reacting a compound of formula (V) with a compound of formula (VI) and a palladium catalyst, such as include Pd(dppf)Cl₂, Pd(PPh₃)₄, and SPhos-Pd-G2 (Chloro(2-dicyclohexylphosphino-2',6'-dimethoxy-1,1'-biphenyl)[2-(2'-amino-1,1'-biphenyl)]palladium(II)).
27. The method of any of clauses 22 to 26, wherein step (i) further comprises reacting a compound of formula (V) with a compound of formula (VI) and a base, such as a base selected from caesium fluoride (CsF), sodium carbonate (Na2CO3), caesium carbonate (Cs2CO3).
28. A method of preparing a compound of formula (V), the method comprising:
   ii) reacting a compound of formula (VII) with a triflation reagent: 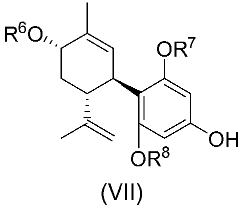 where:
   R⁶ is H, Bz or PMB; and
   R⁷ and R⁸ are independently H, Ac or Piv.
29. The method of clause 28, wherein R⁶ is H or Bz.
30. The method of clause 28 or clause 29, wherein R⁷ and R⁸ are OH.
31. The method of any of clauses 28 to 30, wherein the reagent is selected from trifluoromethanesulfonic anhydride (Tf₂O), N-phenylbis(trifluoromethanesulfonimide) (PhNTf₂), Comins' reagent, 1-(trifluoromethane-sulfonyl)-1H-benzotriazole, 1-(trifluoromethanesulfonyl)imidazole and N-(2-pyridyl)-bis(trifluoromethanesulfonimide).
32. The method of any of clauses 28 to 31, wherein step (ii) comprises reacting a compound of formula (VII) with a triflation reagent and an organic base, such as an organic base selected from pyridine, leutidine, dimethylbenzylamine imidazole, benzimidazole, methylimidazole, triethylamine, tributylamine, diisopropylethylamine, tetramethylethylenediamine, DABCO.
33. A method of preparing a compound of formula (VII), the method comprising:
   iii) reacting a compound of formula (IX) with phloroglucinol (benzene-1,3,5-triol) 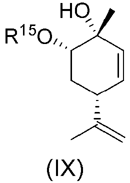 where:
   R¹⁵ is H, Bz or PMB.
34. The method of clause 33, wherein R¹⁵ is Bz or PMB.
35. The method of clause 33 or clause 34, wherein step (iii) comprises reacting a compound of formula (IX) with phloroglucinol and an acid, such as an acid selected from boron trifluoride and triflic acid.
36. A method of preparing a compound of formula (I), the method comprising:
   iii) a Friedel Crafts step as set out in any of clauses 33 to 35;
   ii) a triflation step as set out in any of clause 28 to 32; and optionally
   i) a coupling step as set out in any of clause 22 to 27.
37. A method for preparing a compound of formula (IX), the method comprising:
   iv-a) reacting a compound of formula (X) with diphenyl diselenide to produce a compound of formula (XI); and
   iv-b) reacting the compound of formula (XI) with an oxidising agent, 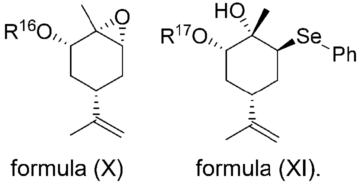 where:
      R¹⁶ and R¹⁷ are independently H, Bz or PMB.
38. The method of clause 37, wherein step (iv-a) comprises reacting a compound of formula (X) with diphenyl diselenide and a reducing agent, such as sodium borohydride.
39. The method of clause 37 or clause 38, wherein step (iv-a) comprises reacting a compound of formula (X) with diphenyl diselenide and a base, such as sodium hydroxide.
40. The method of any of clauses 37 to 39, wherein the oxidising agent us hydrogen peroxide.
41. A method for preparing a compound of formula (X), the method comprising:
   v) reacting a compound of formula (XII) with a peroxycarboxylic acid (peracid) 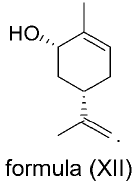
42. The method of clause 41, wherein the peroxycarboxylic acids is selected from chloroperoxybenzoic acid (mCPBA) and perbenzoic acid.
43. A method for preparing a compound of formula (Xa), the method comprising:
   vi) reacting S-carvone with a reducing agent, 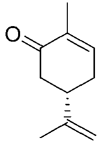
44. The method of clause 44, wherein the reducing agent is selected from lithium aluminium hydride, sodium bis(2-methoxyethoxy)aluminium hydride (red-AI), diborane and sodium borohydride.
45. A method of preparing a compound of formula (V), the method comprising:
   vi) a reduction step as set out in clause 43 or clause 44;
   v) an epoxidation step as set out in clauses 41 or clause 42;
   iv) an allylic oxidation step as set out in any of clauses 37 to 40.
46. A method of preparing a compound of formula (I), the method comprising:
   vi) a reduction step as set out in clause 43 or clause 44;
   v) an epoxidation step as set out in clauses 41 or clause 42;
   iv) an allylic oxidation step as set out in any of clauses 37 to 40.
   iii) a Friedel Crafts step as set out in any of clauses 33 to 35;
   ii) a triflation step as set out in any of clause 28 to 32; and optionally
   i) a coupling step as set out in any of clause 22 to 27.
47. An intermediate selected from compounds of formula (V) and (IX): 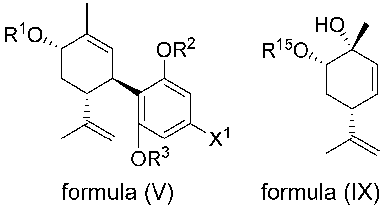 where:
   R¹ is H, Ac, Piv, Bz or PMB;
   R² and R³ are independently H, Ac or Piv;
   X¹ is Br, I or OTf; and
   R¹⁵ is H, Bz or PMB.
48. The intermediate of clause 47, wherein the intermediate is selected from:

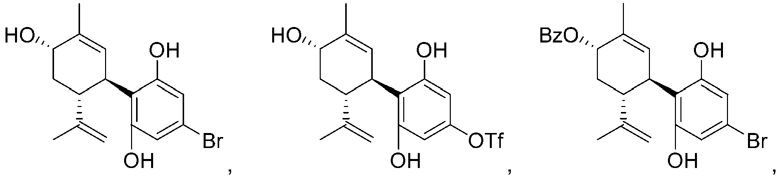

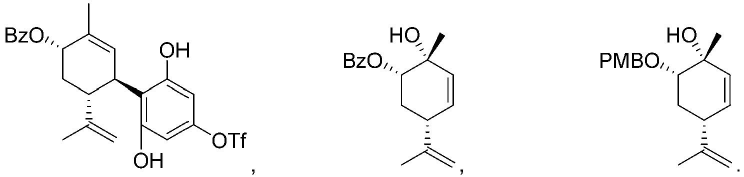

### Definitions

The following definitions are provided in order to aid understanding of the invention.

Epilepsy is considered to be a disease of the brain defined by any of the following conditions: (1) At least two unprovoked (or reflex) seizures occurring >24 h apart; (2) one unprovoked (or reflex) seizure and a probability of further seizures similar to the general recurrence risk (at least 60%) after two unprovoked seizures, occurring over the next 10 years; (3) diagnosis of an epilepsy syndrome (A practical clinical definition of epilepsy by the International League Against Epilepsy (ILAE), 2014).

The term "focal seizure" ("focal onset seizure") refers to seizures originating within networks limited to one hemisphere. They may be discretely localized or more widely distributed. Focal seizures may originate in subcortical structures (Operational Classification of Seizure Types by the ILAE, 2017).

The term "generalized seizure" ("generalized onset seizures") refers to seizures conceptualized as originating at some point within the brain and rapidly engaging bilaterally distributed networks (Operational Classification of Seizure Types by the ILAE, 2017).

The term "pharmaceutically acceptable" pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each ingredient (e.g. carrier, diluent, excipient, etc.) must also be "acceptable" in the sense of being compatible with the other ingredients of the composition.

A "resorcinol" is a compound with the following structure, or any super-structure containing such a structure within it:

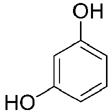

The term "therapeutically-effective amount" pertains to that amount of a compound, or a material, composition or dosage form comprising a compound, which is effective for producing some desired therapeutic effect, commensurate with a reasonable benefit/risk ratio, when administered in accordance with a desired treatment regimen.

A "tonic-clonic seizure" occurs in two phases, a tonic phase typically involving muscle stiffening and loss of consciousness, and a clonic phase typically involving rhythmically jerking of the limbs.

A bond terminating in a wavey line 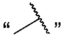 is used to indicate an attachment point.

Ac is an acetyl group (-C(=O)CH₃). Bz is a benzoyl group (-C(=O)Ph). Piv is a pivaloyl group (-C(=O)C(CH₃)₃). PMB is a para-methoxybenzyl group (-CH₂-C₆H₄-OCH₃). Tf is a triflate group (-SO₂CF₃).

### WORKED EXAMPLES

Certain aspects and embodiments of the invention will not be illustrated by way of example and with reference to the figures described above.

### Analytical Methods

### NMR

Bruker Avance 400MHz, 5mm QNP probe H, C, F, P, single Z gradient, two channel instrument running TopSpin 2.1

Bruker Avance III 400MHz, 5mm BBFO Plus probe, single Z gradient, two channel instrument running TopSpin 3.1.

### LCMS

| | | | | |
|---|---|---|---|---|
| Instrumentation | UPLC + Waters DAD + Waters SQD2, single quadrapole UPLC-MS | | | |
| Column | Acquity UPLC HSS C18 1.8um 100 x 2.1mm. (Plus guard cartridge), maintained at temp | | | |
| Mobile Phase A | Water (High purity via PureLab Option unit) with 0.1% formic acid | | | |
| Mobile Phase B | Acetonitrile (Far UV grade) with 0.1% (V/V) formic acid | | | |
| Flow | 0.4ml/min | | | |
| Gradient Program | Time (mins) | % A | | %B |
| | 0.0 | 95 | | 05 |
| | 0.4 | 95 | | 05 |
| | 6.0 | 05 | | 95 |
| | 6.8 | 05 | | 95 |
| | 7.0 | 95 | | 05 |
| | 8.0 | 95 | | 05 |
| Sample | 0.5-2ul (concentration ~ 0.2 -1mg/ml). | | | |
| Detectors | UV, diode array 210nm-400nm | | Resolution 1.2nm | |
| | Other wavelength traces are extracted from the DAD data | | | |
| | MS, mass 100-700 (or -1500 for HM method) in ES+ & ES-(300µl/min split to MS) | | | |

| | | | | |
|---|---|---|---|---|
| Instrumentation | UPLC + Waters DAD + Waters SQD2, single quadrapole UPLC-MS | | | |
| Column | Acquity UPLC BEH C18 1.7um 100 x 2.1mm. (Plus guard cartridge), maintained at temp | | | |
| Mobile Phase A | Water (High purity via PureLab Option unit) with 10mM ammonium bicarbonate (ammonium hydrogen carbonate) | | | |
| Mobile Phase B | Acetonitrile (Far UV grade) | | | |
| Flow | 0.4ml/min | | | |
| Gradient Program | Time (mins) | % A | | %B |
| | 0.0 | 95 | | 05 |
| | 0.4 | 95 | | 05 |
| | 6.0 | 05 | | 95 |
| | 6.8 | 05 | | 95 |
| | 7.0 | 95 | | 05 |
| | 8.0 | 95 | | 05 |
| Sample | 0.5-2ul (concentration ~ 0.2 -1mg/ml). | | | |
| Detectors | UV, diode array 210nm-400nm | | Resolution 1.2nm | |
| | Other wavelength traces are extracted from the DAD data | | | |
| | MS, mass 100-700 (or -1500 for HM method) in ES+ & ES-(300µl/min split to MS) | | | |

### Example 1: Synthtic Production Method for Novel Compounds

This example describes novel methods of synthesis which were used to produce novel resorcinols (**1-58**) which demonstrated pharmacological activity.

The Schemes 1-3 below are numbered to represent each stage of synthesis. Scheme 1 presents the first stage to prepare intermediate **d;** Schemes 2A and 2B are alternative routes of the second stage to prepare intermediate **f** using intermediate **d** from Scheme 1; Schemes 3A and 3B describe the third final stage to prepare the novel compounds (Compounds I-V) of the present invention using intermediate f from either of Schemes 2A or 2B, each also being equally alternative routes.

It will be appreciated that any combination of routes are within the scope of the present invention: Scheme 1 followed by Scheme 2A followed by 3A; Scheme 1 followed by Scheme 2A followed by 3B; Scheme 1 followed by Scheme 2B followed by 3A; Scheme 1 followed by Scheme 2B followed by 3B.

It will be appreciated that any suitable leaving group may be used in place of the p-Methoxybenzyl (PMB) group, and thus the present invention is not limited to such leaving group. Alternative protecting groups include silicon based protecting groups (TMS, TES, TBS, TBDPS, SEM), ethers (MOM, MEM, BOM, Bn, PMB, DMB, allyl), esters (Piv, Bz, Ac), carbamates and carbonates (Troc, dimethylaminocarbamate) or no protecting group (OH).

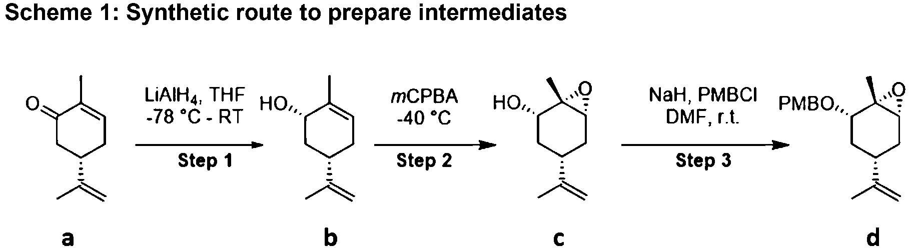

The (S)-(+)-Carvone **a** was reduced using sodium borohydride and cerium (III) chloride at 0 °C to provide the desired (+)-cis-carveol **b.** The endo-cyclic alkene of the (+)-cis-carveol b was then selectively epoxidized using m-CPBA in DCM at -40 °C, yielding the epoxide c. Finally, the secondary alcohol **c** was protected in the presence of NaH and PMBCI in THF to obtain the target material **d** after column chromatography.

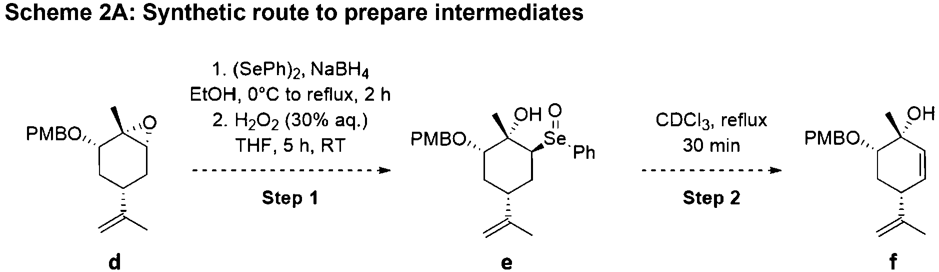

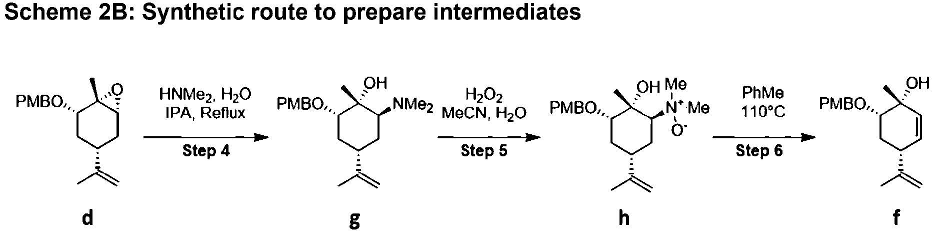

Scheme 2A: The epoxide **d** was reacted with diphenyl diselenide in the presence of sodium borohydride in ethanol at RT. It was then subsequently treated with an aqueous solution of hydrogen peroxide in ethanol to form give the crude **e.** Subsequent heating of **e** in ethanol formed the corresponding allylic alcohol **f.**

Scheme 2B: The epoxide **d** was refluxed in a mixture of IPA/water in the presence of dimethylamine to give crude **g.** It was subsequently oxidised to the N-oxide **h** by treatment with hydrogen peroxide in acetonitrile and water. Ultimately, **h** was refluxed in toluene for 30 minutes to provide **f.**

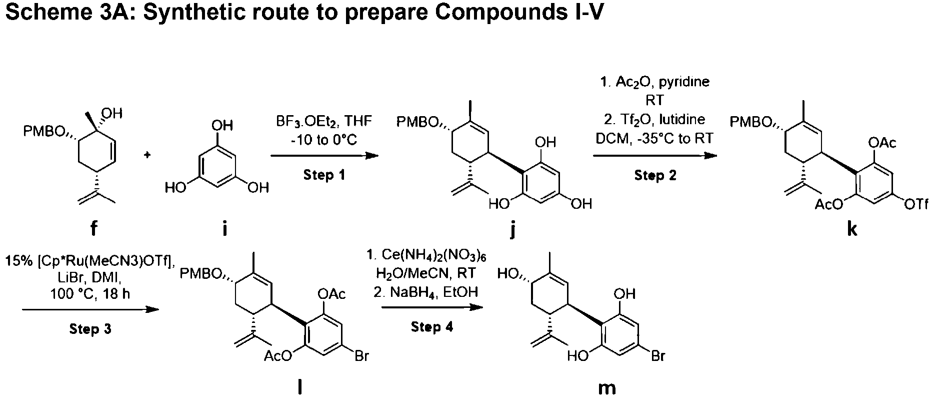

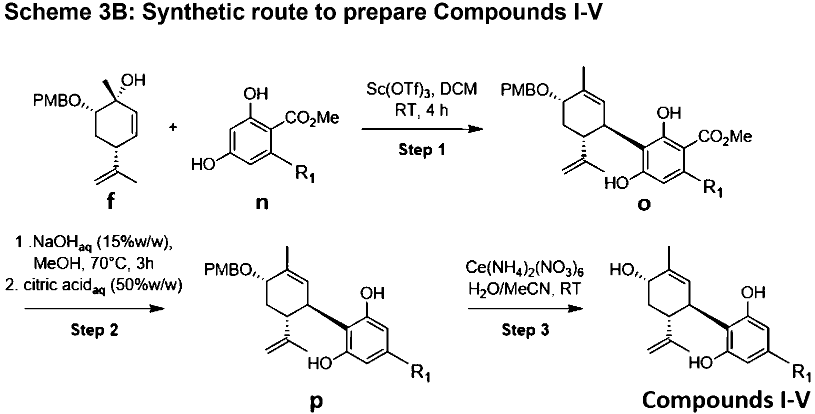

Scheme 3A: The allylic alcohol **f** was stirred in DCM at RT in the presence of phloroglucinol **i** and catalytic amount of Sc(OTf)₃ gave the desired compound **j,** which was subsequently treated with acetic anhydride in pyridine, followed by triflic anhydride in DCM in the presence of 2,6-lutidine to access compound **k.** Ruthenium catalysed bromination of **k** yielded **I.** Final deprotection using cerium (IV) ammonium nitrate followed by sodium borohydride in ethanol afforded the desired product **m.** Subsequent metal catalysed coupling (using Pd, Pt, Ni, Ru, Rh, Au, Mg, Zn etc.) can be performed using **k** or **m** to access further analogues.of the present invention.

Secheme 3B: The allylic alcohol **f** and phenol **n** were stirred at RT in the presence of catalytic amount of Sc(OTf)₃ to give product **o**. Subsequent decarboxylation of **o** in the presence of aqueous sodium hydroxide at 70 °C, followed by treatment with citric acid yielded the desired phenol **p.** Finally deprotection of the PMB protecting group using cerium (IV) ammonium nitrate in a mixture of water and acetonitrile produces compounds of the present invention.

Chemical names of the intermediate compounds **a** to **p** are listed below in Table 1.

**Table 1: Names of intermediate compounds**

| **Intermediate** | **Compound name** |
|---|---|
| **a** | 2-methyl-5-(prop-1-en-2-yl)cyclohex-2-en-1-one |
| **b** | 2-methyl-5-(prop-1-en-2-yl)cyclohex-2-en-1-ol |
| **c** | 1-methyl-4-(prop-1-en-2-yl)-7-oxabicyclo[4.1.0]heptan-2-ol |
| **d** | 2-((4-methoxybenzyl)oxy)-1-methyl-4-(prop-1-en-2-yl)-7-oxabicyclo[4.1.0]heptane |
| **e** | 2-((4-methoxybenzyl)oxy)-1-methyl-6-(phenylseleninyl)-4-(prop-1-en-2-yl)cyclohexan-1-ol |
| **f** | 6-((4-methoxybenzyl)oxy)-1-methyl-4-(prop-1-en-2-yl)cyclohex-2-en-1-ol |
| **g** | 2-(dimethylamino)-6-((4-methoxybenzyl)oxy)-1-methyl-4-(prop-1-en-2-yl)cyclohexan-1-ol |
| **h** | 2-hydroxy-3-((4-methoxybenzyl)oxy)-N,N,2-trimethyl-5-(prop-1-en-2-yl)cyclohexan-1-amine oxide |
| **i** | benzene-1,3,5-triol |
| **j** | 4'-((4-methoxybenzyl)oxy)-5'-methyl-2'-(prop-1-en-2-yl)-1',2',3',4'-tetrahydro-[1,1'-biphenyl]-2,4,6-triol |
| **k** | 4'-((4-methoxybenzyl)oxy)-5'-methyl-2'-(prop-1-en-2-yl)-4-(((trifluoromethyl)sulfonyl)oxy)-1',2',3',4'-tetrahydro-[1,1'-biphenyl]-2,6-diyl diacetate |
| **I** | 4-bromo-4'-((4-methoxybenzyl)oxy)-5'-methyl-2'-(prop-1-en-2-yl)-1',2',3',4'-tetrahydro-[1,1'-biphenyl]-2,6-diyl diacetate |
| **m** | 4-bromo-5'-methyl-2'-(prop-1-en-2-yl)-1',2',3',4'-tetrahydro-[1,1'-biphenyl]-2,4',6-triol |
| **n** | methyl 2,4-dihydroxy-6-propylbenzoate |
| **o** | methyl-2,6-dihyroxy-4'-((4-methoxybenzyl)oxy)-5'-methyl-2'-(prop-1-en-2-yl)-4-propyl-1',2',3',4'-tetrahydro-[1,1'-biphenyl]-3-carboxylate |
| **p** | 4'-((4-methoxybenzyl)oxy)-5'-methyl-2'-(prop-1-en-2-yl)-4-propyl-1',2',3',4'-tetrahydro-[1,1'-biphenyl]-2,6-diol |
| **n, o, p** exemplified using R₁= propyl | |

### Example 1-1: Improved Synthetic Production Method for Intermediates

### Synthesis of Coupling Partner

### Stage 1: Friedel Crafts

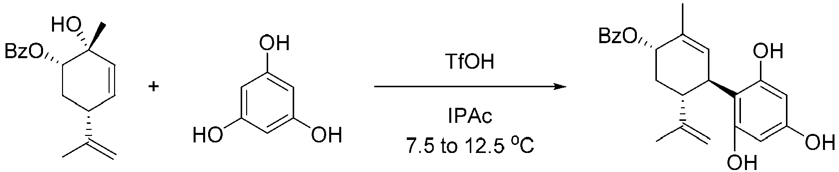

(1S,2R,5R)-2-hydroxy-2-methyl-5-(prop-1-en-2-yl)cyclohex-3-en-1-yl benzoate (6-benzolyloxomethandienol) was reacted with phloroglucinol (10.0 equiv) and triflic acid (0.125 equiv.) in isopropyl acetate (35 volumes). The temperature was maintained between 7.5 and 12.5 °C.

### Stage 2: Triflation

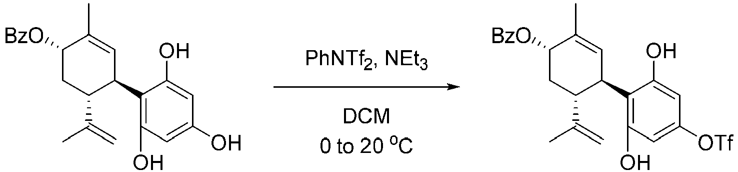

(1R,2R,4S)-2',4',6'-trihydroxy-5-methyl-2-(prop-1-en-2-yl)-1,2,3,4-tetrahydro-[1,1'-biphenyl]-4-yl benzoate was reacted with N-phenylbis(trifluoromethanesulfonimide) (1.20 equiv.) and triethylamine (4.5 equiv) in DCM (15 volumes). The temperature was maintained between 0 °C and 20 °C.

### Stage 3: Pivylation

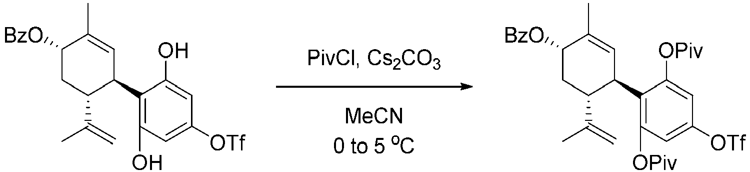

(1R,2R,4S)-2',6'-dihydroxy-5-methyl-2-(prop-1-en-2-yl)-4'-(((trifluoromethyl)sulfonyl)oxy)-1,2,3,4-tetrahydro-[1,1'-biphenyl]-4-yl benzoate was reacted with pivaloyl chloride (2.05 equiv.) and caesium carbonate (2.2 equiv.) in acetonitrile (10 volumes). The temperature was maintained at 0 °C to 5 °C.

### Stage 4: Borylation

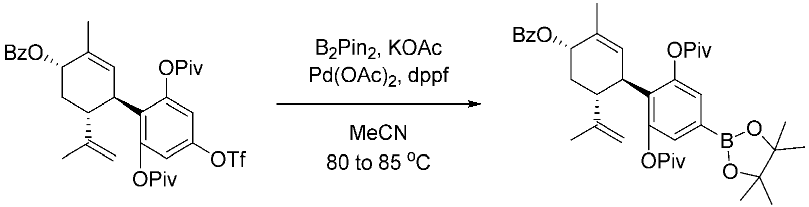

(1'R,2'R,4'S)-4'-(benzoyloxy)-5'-methyl-2'-(prop-1-en-2-yl)-4-(((trifluoromethyl)sulfonyl)oxy)-1',2',3',4'-tetrahydro-[1,1'-biphenyl]-2,6-diyl bis(2,2-dimethylpropanoate) was reacted with bis(pinacolato)diboron (2.0 equiv), pallaidum(II) acetate (0.05 equiv), potassium acetate (3.0 equiv) and dppf (0.1 equiv) in acetonitrile (10 volumes). The temperature was maintained between 80 °C and 85 °C.

### Stage 5: Bromination

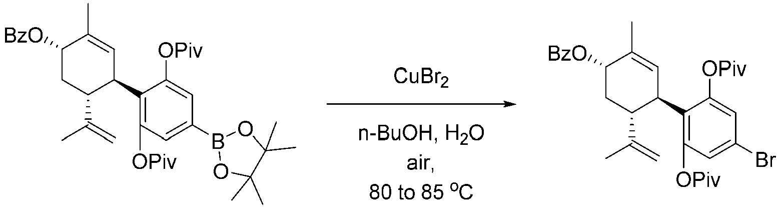

(1'R,2'R,4'S)-4'-(benzoyloxy)-5'-methyl-2'-(prop-1-en-2-yl)-4-(4,4,5,5-tetramethyl-1,3,2-dioxaborolan-2-yl)-1',2',3',4'-tetrahydro-[1,1'-biphenyl]-2,6-diyl bis(2,2-dimethylpropanoate) was reacted with copper (II) bromide (1.0 equiv.) in butanol (10 equiv.) and water (5 equiv.) in air. The temperature was maintained between 80 °C and 85 °C.

### Stable 6: Deprotection

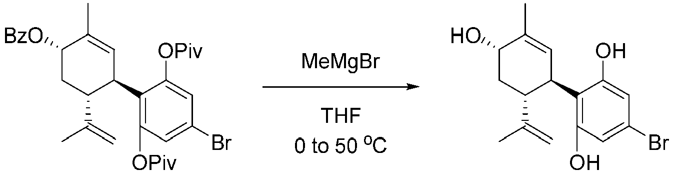

(1'R,2'R,4'S)-4'-(benzoyloxy)-4-bromo-5'-methyl-2'-(prop-1-en-2-yl)-1',2',3',4'-tetrahydro-[1,1'-biphenyl]-2,6-diyl bis(2,2-dimethylpropanoate) was reacted with methylmagnesium bromide (10.0 equiv.) in THF (10 volumes). The reaction mixture was heated from 0 to 50 °C.

### Synthesis of 6-benzolyloxomethandienol

### Stage 1: Reduction

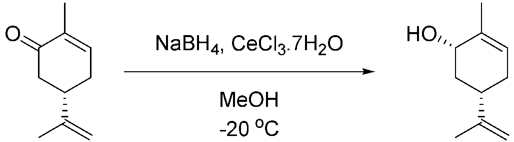

S-carvone was reacted with sodium borohydride and cerium (III) chloride heptahydrate in methanol. The temperature was maintained at -20 °C.

### Stage 2: Epoxidation

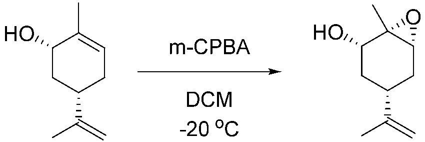

(1S,5S)-2-methyl-5-(prop-1-en-2-yl)cyclohex-2-en-1-ol ((+)-cis-carveol) was reacted with m-CPBA in dichloromethane. The temperature was maintained at -20 °C.

### Stable 3: Selenation

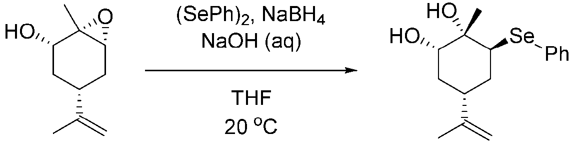

(1S,2S,4R,6R)-1-methyl-4-(prop-1-en-2-yl)-7-oxabicyclo[4.1.0]heptan-2-ol was reacted with diphenyl diselenide, sodium borohydride and sodium hydroxide in tetrahydrofuran. The temperature was maintained at 20 °C.

### Stage 4: Protection

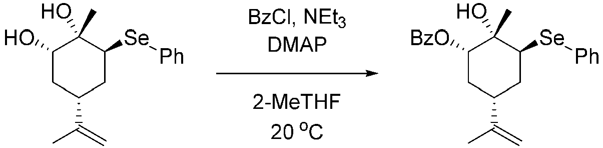

(1S,2S,4R,6S)-1-methyl-6-(phenylselanyl)-4-(prop-1-en-2-yl)cyclohexane-1,2-diol was reacted with benzoyl chloride and DMAP in 2-methyl tetrahydrofuran. The temperature was maintained at 20 °C.

### Stage 5: Allylic oxidation

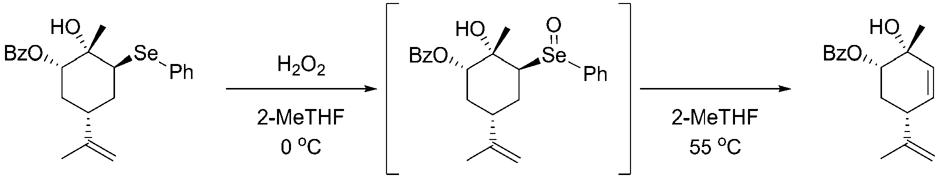

(1S,2S,3S,5R)-2-hydroxy-2-methyl-3-(phenylselanyl)-5-(prop-1-en-2-yl)cyclohexyl benzoate was reacted with hydrogen peroxide in 2-methyl tetrahydrofuran. The temperature was maintained at 0 °C. The temperature was raised to 55 °C.

### Example 1-2: Alternative Synthtic Production Method for Novel Compounds

### Synthesis of Intermediates

### Intermediate 1: 5-(2-Methoxyethyl)benzene-1,3-diol

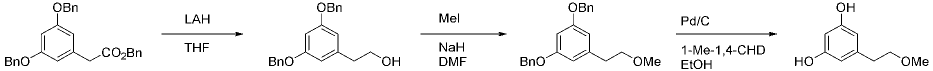

### 2-(3,5-Dibenzyloxyphenyl)ethanol

Benzyl 2-(3,5-dibenzyloxyphenyl)acetate (12.3 g, 28.0 mmol) in tetrahydrofuran (178 mL) under a nitrogen atmosphere was cooled in an ice/water bath and treated with 1 M lithium aluminum hydride (56 mL, 56.1 mmol) dropwise. The reaction mixture was allowed to warm to room temperature overnight, then cooled in an ice/water bath. The reaction was diluted with diethyl ether and quenched with sequential dropwise addition of 2.1 mL of water, 2.1 mL of 15% aqueous sodium hydroxide solution and 6.3 mL of water. The mixture was allowed to warm to room temperature and stirred for 15 minutes. Magnesium sulfate was added and the reaction mixture was stirred for a further 15 minutes. The solids were removed by filtration and the filtrate was concentrated *in vacuo.* The residue was purified by column chromatography on silica, eluting with 0-100% diethyl ether in cyclohexane, to give the title compound as a white solid (5.44 g, 58%).

¹H NMR (400 MHz, DMSO) d 7.47 - 7.32 (m, 10H), 6.52 - 6.49 (m, 3H), 5.06 (s, 4H), 4.63 (t, J=5.3 Hz, 1H), 3.62 - 3.56 (m, 2H), 2.66 (dd, J=7.2, 7.2 Hz, 2H).

### 1,3-Dibenzyloxy-5-(2-methoxyethyl)benzene

A suspension of sodium hydride (60% dispersion in mineral oil, 1.30 g, 32.5 mmol) in *N,N*-dimethylformamide (80 mL) was cooled in an ice/water bath under a nitrogen atmosphere and treated with a solution of 2-(3,5-dibenzyloxyphenyl)ethanol (5.44 g, 16.3 mmol) in *N,N*-dimethylformamide (20 mL) followed by the addition of iodomethane (2.0 mL, 32.5 mmol). The reaction mixture was warmed to room temperature, stirred for 3 hours then quenched with saturated aqueous ammonium chloride (30 mL) and partitioned between water (40 mL) and diethyl ether (100 mL). The aqueous layer was extracted with diethyl ether (2 x 50 mL) and the combined organic layers were washed with water (50 mL), dried (MgSO₄) and concentrated *in vacuo* to give the title compound as a yellow oil (5.88 g, quantitative crude yield). This was used in the next step without further purification.

¹H NMR (400 MHz, DMSO) d 7.46 - 7.32 (m, 10H), 6.54 - 6.50 (m, 3H), 5.07 (s, 4H), 3.52 (t, J=6.9 Hz, 2H), 3.24 (s, 3H), 2.74 (t, J=7.0 Hz, 2H).

### 5-(2-Methoxyethyl)benzene-1,3-diol

A solution of 1,3-dibenzyloxy-5-(2-methoxyethyl)benzene (5.80 g, 16.6 mmol) and 1-methyl-1,4-cyclohexadiene (15 mL, 0.133 mol) in ethanol (100 mL) was treated with 10% palladium on carbon (1.8 g) and heated to 75°C and stirred for 3 hours. The reaction mixture was filtered through a pad of celite and concentrated *in vacuo.* The residue was purified column chromatography on silica, eluting with 0-100% ethyl acetate in cyclohexane, to give the title compound as a yellow oil (2.98 g, NMR shows presence of ethyl acetate, 88% yield accounting for ethyl acetate content).

¹H NMR (400 MHz, DMSO) d 9.06 (s, 2H), 6.07 (d, J=2.2 Hz, 2H), 6.05 (t, J=2.2 Hz, 1H), 4.04 (q, J=7.1 Hz, 1H), 3.46 (t, J=7.0 Hz, 2H), 3.24 (s, 3H), 2.61 (t, J=6.9 Hz, 2H).

### Intermediate 2: 4-Methyl-5-propylbenzene-1,3-diol

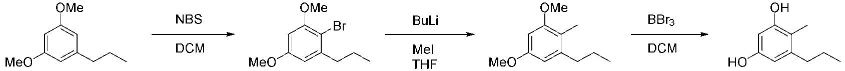

### 2-Bromo-1,5-dimethoxy-3-propyl-benzene

1,3-Dimethoxy-5-propyl-benzene (2.50 g, 13.9 mmol) in dichloromethane (30 mL) was cooled in an ice/water bath and treated with *N*-bromosuccinimide (2.47 g, 13.9 mmol). The reaction mixture was stirred for 2 hours then diluted with ethyl acetate (100 mL), washed with half-saturated sodium thiosulfate solution (100 mL), 2 M aqueous sodium hydroxide solution (100 mL) and water (100 mL). The organic layer was dried (hydrophobic filter paper) and concentrated *in vacuo* to give the title compound as a colourless oil (3.75 g, quantitative crude yield). This was used without further purification in the next step.

¹H NMR (400 MHz, CDCl₃) d 6.40 (d, J=2.9 Hz, 1H), 6.35 (d, J=2.8 Hz, 1H), 3.86 (s, 3H), 3.79 (s, 3H), 2.73 - 2.68 (m, 2H), 1.69 - 1.59 (m, 2H), 0.98 (t, J=7.3 Hz, 3H).

### 1,5-Dimethoxy-2-methyl-3-propyl-benzene

2-Bromo-1,5-dimethoxy-3-propyl-benzene (3.46 g, 13.4 mmol) in tetrahydrofuran (100 mL) was cooled in a dry ice-acetone bath and treated with 2.5 M *n*-butyllithium solution (12 mL, 29.4 mmol) in portions over 10 minutes. The reaction mixture was stirred for 15 minutes then treated with iodomethane (2.5 mL, 40.1 mmol) and allowed to warm to room temperature overnight. The reaction was quenched with saturated ammonium chloride solution (10 mL) and treated with 7 M ammonia in methanol (5 ml). The mixture was stirred for 2 hours, diluted with ethyl acetate (250 mL), washed with water (2 x 250 mL), dried (hydrophobic filter paper) and concentrated *in vacuo.* The crude product was combined with that of a test reaction which had been run on 250mg scale under the same conditions, and purified by column chromatography on silica, eluting with 0-60 % dichloromethane in cyclohexane, to give the title compound as a colourless oil (2.42 g, 100%)

¹H NMR (400 MHz, CDCl₃) d 6.32 (s, 2H), 3.79 (s, 3H), 3.79 (s, 3H), 2.58 - 2.52 (m, 2H), 2.09 (s, 3H), 1.61 - 1.54 (m, 2H), 0.97 (t, J=7.3 Hz, 3H).

### 4-Methyl-5-propylbenzene-1,3-diol

1,5-Dimethoxy-2-methyl-3-propyl-benzene (2.4 g, 12.5 mmol) in dichloromethane (75 mL) was cooled in an ice/water bath and treated with 1 M boron tribromide (31 mL, 31.4 mmol). The mixture was allowed to warm to room temperature overnight. The reaction was quenched with methanol (10mL) and saturated aqueous sodium hydrogen carbonate solution (10 mL) and diluted with dichloromethane (75 mL). The two phases were separated and the organic phase was washed with half-saturated aqueous sodium hydrogen carbonate solution (100 mL). The aqueous layer was extracted with ethyl acetate (2 x 100 mL) and the combined organic layers were dried (hydrophobic filter paper) and concentrated *in vacuo* to give a brown gum (1.73 g, 83%).

¹H NMR (400 MHz, DMSO) d 8.92 (s, 1H), 8.76 (s, 1H), 6.14 (d, J=2.4 Hz, 1H), 6.03 (d, J=2.1 Hz, 1H), 2.42 - 2.37 (m, 2H), 1.94 (s, 3H), 1.52 - 1.42 (m, 2H), 0.92 (t, J=7.3 Hz, 3H).

### General Method A

General Method A is illustrated by the synthesis of Intermediate 3: (1'*R*,2'*R*)-4-(Methoxymethyl)-5'-methyl-2'-(prop-1-en-2-yl)-1',2',3',4'-tetrahydro-[1,1'-biphenyl]-2,6-diol

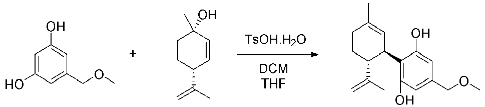

A solution of 5-(methoxymethyl)benzene-1,3-diol (2.68 g, 17.4 mmol) and p-toluenesulfonic acid monohydrate (248 mg, 1.30 mmol) in tetrahydrofuran (10 mL) and dichloromethane (120 mL) was cooled to 4°C in an ice/water bath. (4*R*)-4-isopropenyl-1-methyl-cyclohex-2-en-1-ol (2.60 mL, 16.0 mmol) was added dropwise over 60 minutes. The reaction mixture was stirred for a further 2 hours, then quenched with a solution of sodium hydrogen carbonate (450 mg) in water (50 mL). The layers were separated, the aqueous layer was extracted with dichloromethane (50 mL) and the combined organic layers were dried (hydrophobic frit paper) and concentrated *in vacuo.* The residue was purified by column chromatography on silica, eluting with 0-100% diethyl ether in cyclohexane, to give the title compound as a brown oil (1.08 g, 23%).

¹H NMR (400 MHz, DMSO) d 8.85 (s, 2H), 6.20 (s, 2H), 5.14 (s, 1H), 4.55 - 4.52 (m, 1H), 4.46 (s, 1H), 4.21 (s, 2H), 3.94 - 3.87 (m, 1H), 3.28 (s, 3H), 3.14 - 3.06 (m, 1H), 2.20 - 2.11 (m, 1H), 2.02 - 1.94 (m, 1H), 1.78 - 1.67 (m, 2H), 1.66 (s, 3H), 1.64 (s, 3H).

General Method A, using the appropriate resorcinol, was used to generate the following compounds.

### Intermediate 4: (1'R,2'R)-4-Bromo-5'-methyl-2'-(prop-1-en-2-yl)-1',2',3',4'-tetrahydro-[1,1'-biphenyl]-2,6-diol

¹H NMR (400 MHz, DMSO) d 9.37 (s, 2H), 6.38 (s, 2H), 5.08 (s, 1H), 4.49 (d, J=2.8 Hz, 1H), 4.44 (dd, J=1.6, 2.8 Hz, 1H), 3.86 - 3.83 (m, 1H), 3.06 - 2.98 (m, 1H), 2.12 - 2.07 (m, 1H), 1.96 - 1.92 (m, 1H), 1.63 - 1.59 (m, 8H).

### Intermediate 5: (1'R,2'R)-4-(2-Methoxyethyl)-5'-methyl-2'-(prop-1-en-2-yl)-1',2',3',4'-tetrahydro-[1,1'-biphenyl]-2,6-diol

¹H NMR (400 MHz, DMSO) d 8.73 (s, 2H), 6.06 (s, 2H), 5.09 (s, 1H), 4.51 (d, J=2.8 Hz, 1H), 4.44 - 4.41 (m, 1H), 3.87 - 3.83 (m, 1H), 3.45 (t, J=6.9 Hz, 2H), 3.24 (s, 3H), 3.09 - 3.01 (m, 1H), 2.56 (t, J=6.8 Hz, 2H), 2.16 - 2.06 (m, 1H), 1.97 - 1.89 (m, 1H), 1.69 - 1.63 (m, 2H), 1.62 (s, 3H), 1.61 (s, 3H).

### Intermediate 6: (1'R,2'R)-3,5'-dimethyl-2'-(prop-1-en-2-yl)-4-propyl-1',2',3',4'-tetrahydro-[1,1'-biphenyl]-2,6-diol

¹H NMR (400 MHz, DMSO) d 8.53 (s, 1H), 7.27 (s, 1H), 6.09 (s, 1H), 5.15 (s, 1H), 4.48 (d, J=2.2 Hz, 1H), 4.42 (dd, J=1.4, 2.7 Hz, 1H), 3.94 - 3.88 (m, 1H), 3.03 - 2.94 (m, 1H), 2.39 - 2.32 (m, 2H), 2.16 - 2.09 (m, 1H), 2.00 - 1.92 (m, 1H), 1.96 (s, 3H), 1.73 - 1.66 (m, 2H), 1.64 (s, 3H), 1.60 (s, 3H), 1.51 - 1.40 (m, 2H), 0.91 (t, J=7.3 Hz, 3H).

### Intermediate 7: (1'R,2'R)-4-bromo-3,5,5'-trimethyl-2'-(prop-1-en-2-yl)-1',2',3',4'-tetrahydro-[1,1'-biphenyl]-2,6-diol

¹H NMR (400 MHz, DMSO) d 8.16 (s, 1H), 7.71 (s, 1H), 5.18 (s, 1H), 4.47 (d, J=2.5 Hz, 1H), 4.43 (dd, J=1.4, 2.5 Hz, 1H), 4.03 - 3.99 (m, 1H), 3.01 (dt, J=3.3, 11.0 Hz, 1H), 2.18 (s, 6H), 2.16 - 2.11 (m, 1H), 2.04 - 1.96 (m, 1H), 1.75 - 1.67 (m, 2H), 1.65 (s, 3H), 1.60 (s, 3H).

### General Method B

General Method B is illustrated by the preparation of Intermediate 6: (1'*R*,2'*R*,4'*S*)-4-Bromo-5'-methyl-2'-(prop-1-en-2-yl)-1',2',3',4'-tetrahydro-[1,1'-biphenyl]-2,4',6-triol

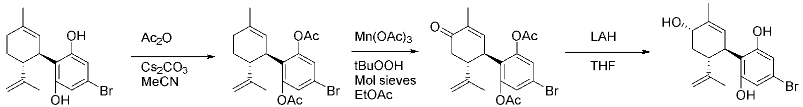

### [3-Acetoxy-5-bromo-2-[(1R,6R)-6-isopropenyl-3-methyl-cyclohex-2-en-1-yl]phenyl] acetate

5-Bromo-2-[(1*R*,6*R*)-6-isopropenyl-3-methyl-cyclohex-2-en-1-yl]benzene-1,3-diol (1305 mg, 4.04 mmol) in acetonitrile (30 mL) was treated with cesium carbonate (3289 mg, 10.1 mmol) followed by acetic anhydride (0.95 mL, 10.1 mmol). The reaction mixture was stirred for 1 hour then partitioned between ethyl acetate (100 mL) and water (100 mL). The layers were separated, the aqueous layer was extracted with ethyl acetate (100 mL) and the combined organic phases were dried (hydrophobic filter paper) and concentrated *in vacuo* to give the title compound as an orange gum (1.60 g, 93.4%).

¹H NMR (400 MHz, DMSO) d 7.33 (s, 2H), 4.98 (s, 1H), 4.55 - 4.52 (m, 1H), 4.43 (d, J=1.5 Hz, 1H), 3.59 - 3.52 (m, 1H), 2.70 - 2.61 (m, 1H), 2.26 - 2.25 (m, 7H), 2.03 (d, J=16.9 Hz, 1H), 1.79 - 1.72 (m, 2H), 1.67 (s, 3H), 1.62 (s, 3H).

### [3-Acetoxy-5-bromo-2-[(1R,6R)-6-isopropenyl-3-methyl-4-oxo-cyclohex-2-en-1-yl]phenyl] acetate

[3-Acetoxy-5-bromo-2-[(1*R*,6*R*)-6-isopropenyl-3-methyl-cyclohex-2-en-1-yl]phenyl] acetate (1560 mg, 3.83 mmol) and molecular sieves (3Å, 5 g) in ethyl acetate (31.92 mL) were treated with manganese(lll) acetate dihydrate (103 mg, 0.383 mmol) followed by 5.5 M tert-butyl hydroperoxide solution in nonane (3.5 mL, 19.2 mmol) The reaction mixture was stirred at 20°C overnight then diluted with ethyl acetate (50 mL) and filtered through a pad of celite. The filtrate was washed with saturated aqueous sodium thiosulfate (30 mL) and water (30 mL), separated, dried (MgSO₄), filtered and concentrated *in vacuo.* The residue was purified by column chromatography on silica, eluting with 0-100% diethyl ether in cyclohexane, to give the title compound as an off-white solid (561 mg, 34.8%).

¹H NMR (400 MHz, DMSO) d 7.39 (s, 2H), 6.38 (t, J=1.8 Hz, 1H), 4.56 (s, 1H), 4.50 (s, 1H), 4.04 (td, J=2.3, 10.6 Hz, 1H), 3.20 - 3.11 (m, 1H), 2.85 - 2.76 (m, 1H), 2.35 - 2.19 (m, 7H), 1.71 (dd, J=1.5, 2.5 Hz, 3H), 1.62 (s, 3H).

### (1'R,2'R,4'S)-4-Bromo-5'-methyl-2'-(prop-1-en-2-yl)-1',2',3',4'-tetrahydro-[1,1'-biphenyl]-2,4',6-triol

To a solution of [3-acetoxy-5-bromo-2-[(1*R*,6*R*)-6-isopropenyl-3-methyl-4-oxo-cyclohex-2-en-1-yl]phenyl] acetate (1.10 g, 2.61 mmol) in methyl alcohol (25.0 mL) at 0°C was added sodium borohydride (0.22 g, 5.74 mmol) in four equal portions over a period of 30 minutes and the mixture was allowed to warm to room temperature overnight. The reaction mixture was concentrated *in vacuo* and the residue partitioned between ethyl acetate (75 mL) and water (75 mL). The layers were separated and the organic phase was washed with brine (50 mL), dried (MgSO₄) and concentrated *in vacuo.* The residue was purified by column chromatography on silica, eluting with 0-80% ethyl acetate in cyclohexane, to give the title compound (508 mg, 57.4%) as a white solid.

¹H NMR (400 MHz, DMSO) d 9.53 (s, 1H), 9.32 (s, 1H), 6.43 - 6.36 (m, 2H), 5.07 - 5.05 (m, 1H), 4.62 (d, J=6.7 Hz, 1H), 4.49 - 4.44 (m, 2H), 4.12 - 4.06 (m, 1H), 3.88 - 3.84 (m, 1H), 3.20 - 3.12 (m, 1H), 1.90 (ddd, J=2.4, 5.7, 12.0 Hz, 1H), 1.66 - 1.63 (m, 3H), 1.63 - 1.55 (m, 4H).

### General Method C

General Method C is illustrated by the synthesis of Intermediate 7: (1'*R*,2'*R*,4'*S*)-2,4',6-Trihydroxy-5'-methyl-2'-(prop-1-en-2-yl)-1',2',3',4'-tetrahydro-[1,1'-biphenyl]-4-yl trifluoromethanesulfonate

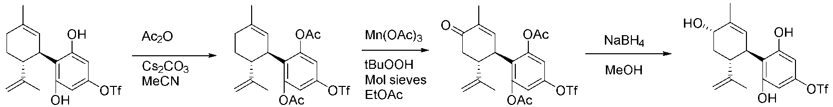

### (1'R,2'R)-5'-Methyl-2'-(prop-1-en-2-yl)-4-(((trifluoromethyl)sulfonyl)oxy)-1',2',3',4'-tetrahydro-[1,1'-biphenyl]-2,6-diyl diacetate

A solution of (1'*R*,2'*R*)-2,6-dihydroxy-5'-methyl-2'-(prop-1-en-2-yl)-1',2',3',4'-tetrahydro-[1,1'-biphenyl]-4-yl trifluoromethanesulfonate (10.0 g, 25.5 mmol) in acetonitrile (200 mL) was treated with cesium carbonate (20.76 g, 63.7 mmol) and acetic anhydride (6.0 mL, 63.7 mmol). The reaction mixture was stirred at room temperature over the weekend. The mixture was diluted with ethyl acetate (200 mL) and washed with water (100 mL) and brine (100 mL). The organic layer was dried (phase separation paper) and concentrated *in vacuo.* The residue was purified by column chromatography on silica, eluting with 0-10% ethyl acetate in cyclohexane, to give the title compound as a pale yellow oil (8.08 g, 67%).

¹H NMR (400 MHz, CDCl₃) d 6.91 (s, 2H), 5.16 (s, 1H), 4.55 (s, 1H), 4.41 (s, 1H), 3.61 - 3.56 (m, 1H), 2.67 - 2.59 (m, 1H), 2.20 (s, 6H), 2.15 - 2.01 (m, 2H), 1.85 - 1.70 (m, 2H), 1.68 (s, 3H), 1.57 (s, 3H).

### (1'R,2'R)-5'-Methyl-4'-oxo-2'-(prop-1-en-2-yl)-4-(((trifluoromethyl)sulfonyl)oxy)-1',2',3',4'-tetrahydro-[1,1'-biphenyl]-2,6-diyl diacetate

A solution of (1'*R*,2'*R*)-5'-methyl-2'-(prop-1-en-2-yl)-4-(((trifluoromethyl)sulfonyl)oxy)-1',2',3',4'-tetrahydro-[1,1'-biphenyl]-2,6-diyl diacetate (4.0 g, 8.40 mmol) in ethyl acetate (80 mL) was treated with 3Å mol sieves (4 g) followed by manganese(III) acetate dihydrate (270 mg, 1.01 mmol) and tert-butyl hydroperoxide (5.5 M solution in nonane, 8.5 mL, 47.0 mmol). The reaction mixture was stirred at room temperature overnight. The mixture was filtered through a pad of celite washing with ethyl acetate (80 mL) and the organic layer was washed with saturated sodium thiosulphate solution (50 mL) and brine (50 mL), dried (phase separation paper) and concentrated *in vacuo.* The residue was purified by column chromatography on silica, eluting with 10-25% ethyl acetate in cyclohexane, to give the title compound as a yellow oil (1.26 g, 30%)

¹H NMR (400 MHz, CDCl₃) d 6.99 (s, 2H), 6.51 - 6.48 (m, 1H), 4.66 - 4.63 (m, 1H), 4.52 (s, 1H), 3.95 - 3.90 (m, 1H), 3.25 - 3.16 (m, 1H), 2.65 - 2.53 (m, 2H), 2.24 (s, 6H), 1.83 - 1.80 (m, 3H), 1.61 (s, 3H).

### (1'R,2'R,4'S)-2,4',6-Trihydroxy-5'-methyl-2'-(prop-1-en-2-yl)-1',2',3',4'-tetrahydro-[1,1'-biphenyl]-4-yl trifluoromethanesulfonate

A solution of (1'*R*,2'*R*)-5'-methyl-4'-oxo-2'-(prop-1-en-2-yl)-4-(((trifluoromethyl)sulfonyl)oxy)-1',2',3',4'-tetrahydro-[1,1'-biphenyl]-2,6-diyl diacetate (1.26 g, 2.57 mmol) in methanol (20 mL) cooled to 0°C was treated with sodium borohydride (243 mg, 6.42 mmol) in portions. The reaction mixture was warmed to room temperature and stirred overnight. The mixture was diluted with ethyl acetate (100 mL) and washed with saturated sodium hydrogen carbonate solution (50 mL). The aqueous layer was extracted with ethyl acetate (50 mL) and the organic layers were combined, washed with brine (50 mL), dried (phase separation paper) and concentrated *in vacuo.* The residue was purified by column chromatography on silica, eluting with 10-40% ethyl acetate in cyclohexane, to give the title compound as a white solid (0.5 g, 48%).

¹H NMR (400 MHz, DMSO) d 9.93 (s, 1H), 9.74 (s, 1H), 6.29 (s, 2H), 5.13 - 5.10 (m, 1H), 4.66 (d, J=6.8 Hz, 1H), 4.51 - 4.49 (m, 2H), 4.19 - 4.11 (m, 1H), 3.97 - 3.92 (m, 1H), 3.24 - 3.16 (m, 1H), 1.95 (ddd, J=2.4, 5.7, 12.0 Hz, 1H), 1.70 (s, 3H), 1.64 (s, 3H), 1.63 - 1.57 (m, 1H).

### Synthesis of Final Compounds

### General Method D

General Method D is illustrated by the synthesis of (1'*R*,2'*R*,4'*S*)-4-(5-Methoxypyridin-3-yl)-5'-methyl-2'-(prop-1-en-2-yl)-1',2',3',4'-tetrahydro-[1,1'-biphenyl]-2,4',6-triol **compound 20**

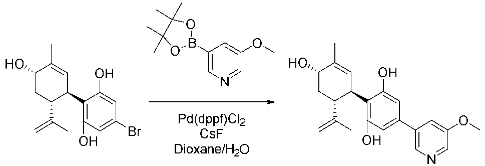

5-Bromo-2-[(1*R*,4*S*,6*R*)-4-hydroxy-6-isopropenyl-3-methyl-cyclohex-2-en-1-yl]benzene-1,3-diol (70 mg, 0.206 mmol), 5-methoxy-3-pyridineboronic acid pinacol ester (73 mg, 0.310 mmol) and cesium fluoride (94 mg, 0.619 mmol) in 1,4-dioxane (2.0 mL) and water (0.50 mL) were degassed with nitrogen and treated with [1,1'-bis(diphenylphosphino)ferrocene]-dichloropalladium(ll) (7.7 mg, 0.0103 mmol). The reaction mixture was heated at 80°C for 2 hours. The reaction mixture was cooled to room temperature, poured into water (20 mL) and extracted with dichloromethane (2 x 20 mL). The combined organic layers were dried (hydrophobic frit) and concentrated *in vacuo.* The residue was purified by column chromatography on silica, eluting with 20-80% ethyl acetate in cyclohexane, and lyophilised from acetonitrile/water to give the title compound as a white solid (35.2 mg, 46%).

¹H NMR (400 MHz, DMSO) d 9.34 (s, 1H), 9.11 (s, 1H), 8.28 (d, J=1.9 Hz, 1H), 8.25 (d, J=2.8 Hz, 1H), 7.39 (dd, J=1.9, 2.7 Hz, 1H), 6.50 (s, 2H), 5.11 (q, J=1.7 Hz, 1H), 4.62 (d, J=6.5 Hz, 1H), 4.55 (d, J=2.5 Hz, 1H), 4.46 (q, J=1.4 Hz, 1H), 4.17 - 4.10 (m, 1H), 4.00 - 3.93 (m, 1H), 3.89 (s, 3H), 3.31 - 3.23 (m, 1H), 1.93 (ddd, J=2.2, 5.6, 12.1 Hz, 1H), 1.67 (s, 3H), 1.65 (s, 3H), 1.63 - 1.55 (m, 1H). MS (ESI): m / z 368 (M+1). HPLC purity: 99.7%.

General Method D, using the appropriate boronic acid or boronic ester, was used to generate the following compounds.

### (1'R,2'R,4'S)-4-(2-Methoxypyrimidin-5-yl)-5'-methyl-2'-(prop-1-en-2-yl)-1',2',3',4'-tetrahydro-[1,1'-biphenyl]-2,4',6-triol (compound 21)

¹H NMR (400 MHz, DMSO) d 9.38 (s, 1H), 9.14 (s, 1H), 8.70 (s, 2H), 6.44 (s, 2H), 5.12 - 5.09 (m, 1H), 4.62 (d, J=6.2 Hz, 1H), 4.54 (d, J=2.7 Hz, 1H), 4.45 (q, J=1.4 Hz, 1H), 4.17 - 4.10 (m, 1H), 4.00 - 3.94 (m, 4H), 3.30 - 3.22 (m, 1H), 1.93 (ddd, J=2.4, 5.8, 12.1 Hz, 1H), 1.66 (s, 3H), 1.64 (s, 3H), 1.62 - 1.55 (m, 1H). MS (ESI): m / z 369 (M+1). HPLC purity: 99.3%.

### (1'R,2'R,4'S)-4-(1,3-Dimethyl-1H-pyrazol-4-yl)-5'-methyl-2'-(prop-1-en-2-yl)-1',2',3',4'-tetrahydro-[1,1'-biphenyl]-2,4',6-triol (compound 11)

¹H NMR (400 MHz, DMSO) d 9.03 (s, 1H), 8.80 (s, 1H), 7.64 (s, 1H), 6.29 (s, 2H), 5.12 - 5.09 (m, 1H), 4.59 (d, J=6.6 Hz, 1H), 4.54 (d, J=2.4 Hz, 1H), 4.45 (q, J=1.4 Hz, 1H), 4.15 - 4.09 (m, 1H), 3.94 - 3.88 (m, 1H), 3.77 (s, 3H), 3.28 - 3.19 (m, 1H), 2.25 (s, 3H), 1.92 (ddd, J=2.3, 5.6, 11.9 Hz, 1H), 1.66 (s, 3H), 1.63 (s, 3H), 1.61 - 1.54 (m, 1H). MS (ESI): m / z 355 (M+1). HPLC purity: 95%.

### (1'R,2'R,4'S)-4-(2-Methoxypyridin-4-yl)-5'-methyl-2'-(prop-1-en-2-yl)-1',2',3',4'-tetrahydro-[1,1'-biphenyl]-2,4',6-triol (compound 16)

¹H NMR (400 MHz, DMSO) d 9.38 (s, 1H), 9.16 (s, 1H), 8.19 (dd, J=0.6, 5.4 Hz, 1H), 7.07 (dd, J=1.5, 5.4 Hz, 1H), 6.82 (dd, J=0.7, 1.6 Hz, 1H), 6.54 (s, 2H), 5.12 - 5.09 (m, 1H), 4.62 (d, J=6.5 Hz, 1H), 4.53 (d, J=2.5 Hz, 1H), 4.45 (q, J=1.5 Hz, 1H), 4.17 - 4.10 (m, 1H), 3.98 - 3.94 (m, 1H), 3.89 (s, 3H), 3.32 - 3.22 (m, 1H), 1.93 (ddd, J=2.2, 5.7, 11.9 Hz, 1H), 1.66 (s, 3H), 1.64 (s, 3H), 1.62 - 1.55 (m, 1H). MS (ESI): m / z 368 (M+1). HPLC purity: 99.2%.

### (1R,2R,4S)-3"-Methoxy-5-methyl-2-(prop-1-en-2-yl)-1,2,3,4-tetrahydro-[1,1':4',1"-terphenyl]-2',4,6'-triol (compound 15)

¹H NMR (400 MHz, DMSO) d 9.24 (s, 1H), 9.03 (s, 1H), 7.38 (t, J=8.0 Hz, 1H), 7.08 (d, J=7.6 Hz, 1H), 7.02 (t, J=2.0 Hz, 1H), 6.93 (ddd, J=0.8, 2.5, 8.3 Hz, 1H), 6.52 (s, 2H), 5.15 (q, J=1.6 Hz, 1H), 4.64 (d, J=6.6 Hz, 1H), 4.59 (d, J=2.4 Hz, 1H), 4.51 - 4.48 (m, 1H), 4.21 - 4.12 (m, 1H), 4.03 - 3.96 (m, 1H), 3.84 (s, 3H), 3.35 - 3.26 (m, 1H), 1.97 (ddd, J=2.3, 5.6, 11.9 Hz, 1H), 1.70 (s, 3H), 1.68 (s, 3H), 1.67 - 1.58 (m, 1H). MS (ESI): m / z 367 (M+1). HPLC purity: 97.3%.

### (1'R,2'R,4'S)-4-(6-Methoxypyridin-3-yl)-5'-methyl-2'-(prop-1-en-2-yl)-1',2',3',4'-tetrahydro-[1,1'-biphenyl]-2,4',6-triol (compound 17)

¹H NMR (400 MHz, DMSO) d 9.29 (s, 1H), 9.07 (s, 1H), 8.31 (dd, J=0.6, 2.6 Hz, 1H), 7.82 (dd, J=2.5, 8.6 Hz, 1H), 6.92 (dd, J=0.7, 8.6 Hz, 1H), 6.47 (s, 2H), 5.16 - 5.14 (m, 1H), 4.64 (d, J=6.6 Hz, 1H), 4.58 (d, J=2.6 Hz, 1H), 4.49 (q, J=1.3 Hz, 1H), 4.21 - 4.14 (m, 1H), 4.01 - 3.98 (m, 1H), 3.93 (s, 3H), 3.34 - 3.26 (m, 1H), 1.97 (ddd, J=2.2, 5.6, 11.9 Hz, 1H), 1.70 (s, 3H), 1.68 (s, 3H), 1.66 - 1.59 (m, 1H). MS (ESI): m / z 368 (M+1). HPLC purity: 98.9%.

### (1'R,2'R,4'S)-5'-Methyl-2'-(prop-1-en-2-yl)-4-(pyridin-3-yl)-1',2',3',4'-tetrahydro-[1,1'-biphenyl]-2,4',6-triol (compound 22)

¹H NMR (400 MHz, DMSO) d 9.35 (s, 1H), 9.12 (s, 1H), 8.68 (dd, J=0.9, 2.4 Hz, 1H), 8.53 (dd, J=1.6, 4.7 Hz, 1H), 7.85 (ddd, J=1.7, 2.4, 8.0 Hz, 1H), 7.45 (dd, J=4.8, 8.3 Hz, 1H), 6.49 (s, 2H), 5.13 - 5.11 (m, 1H), 4.62 (d, J=6.5 Hz, 1H), 4.55 (d, J=2.5 Hz, 1H), 4.46 (s, 1H), 4.14 - 4.11 (m, 1H), 3.98 - 3.95 (m, 1H), 3.32 - 3.23 (m, 1H), 1.93 (ddd, J=2.3, 5.7, 12.1 Hz, 1H), 1.67 (s, 3H), 1.65 (s, 3H), 1.63 - 1.55 (m, 1H). MS (ESI): m / z 338 (M+1). HPLC purity: 99.1%.

### (1'R,2'R,4'S)-5'-Methyl-2'-(prop-1-en-2-yl)-4-(6-(trifluoromethyl)pyridin-3-yl)-1',2',3',4'-tetrahydro-[1,1'-biphenyl]-2,4',6-triol (compound 23)

¹H NMR (400 MHz, DMSO) d 9.47 (s, 1H), 9.25 (s, 1H), 8.88 (d, J=2.1 Hz, 1H), 8.13 (dd, J=1.9, 8.2 Hz, 1H), 7.95 (d, J=7.9 Hz, 1H), 6.54 (s, 2H), 5.13 - 5.11 (m, 1H), 4.63 (d, J=6.5 Hz, 1H), 4.55 (d, J=2.5 Hz, 1H), 4.46 (q, J=1.3 Hz, 1H), 4.17 - 4.11 (m, 1H), 4.06 - 3.97 (m, 1H), 3.32 - 3.23 (m, 1H), 1.94 (ddd, J=2.4, 5.6, 12.1 Hz, 1H), 1.68 (s, 3H), 1.65 (s, 3H), 1.63 - 1.56 (m, 1H). MS (ESI): m / z 406 (M+1). HPLC purity: 98.5%.

### (1'R,2'R,4'S)-4-(5-Chloropyridin-3-yl)-5'-methyl-2'-(prop-1-en-2-yl)-1',2',3',4'-tetrahydro-[1,1'-biphenyl]-2,4',6-triol (compound 24)

¹H NMR (400 MHz, DMSO) d 9.43 (s, 1H), 9.18 (s, 1H), 8.64 (d, J=1.9 Hz, 1H), 8.59 (d, J=2.3 Hz, 1H), 7.95 (t, J=2.1 Hz, 1H), 6.49 (s, 2H), 5.11 (d, J=1.5 Hz, 1H), 4.63 (d, J=6.7 Hz, 1H), 4.54 (d, J=2.5 Hz, 1H), 4.47 - 4.44 (m, 1H), 4.16 - 4.10 (m, 1H), 3.99 - 3.95 (m, 1H), 3.32 - 3.23 (m, 1H), 1.94 (ddd, J=2.3, 5.5, 12.0 Hz, 1H), 1.67 (s, 3H), 1.65 (s, 3H), 1.62 - 1.56 (m, 1H). MS (ESI): m / z 372 (M+1). HPLC purity: 96.2%.

### (1'R,2'R,4'S)-5'-Methyl-4-(2-methylthiazol-5-yl)-2'-(prop-1-en-2-yl)-1',2',3',4'-tetrahydro-[1,1'-biphenyl]-2,4',6-triol (compound 13)

¹H NMR (400 MHz, DMSO) d 9.33 (s, 1H), 9.14 (s, 1H), 7.70 (s, 1H), 6.43 (s, 2H), 5.10 - 5.08 (m, 1H), 4.62 (d, J=6.7 Hz, 1H), 4.51 (d, J=2.6 Hz, 1H), 4.44 (q, J=1.4 Hz, 1H), 4.14 - 4.09 (m, 1H), 3.94 - 3.90 (m, 1H), 3.30 - 3.19 (m, 1H), 2.65 (s, 3H), 1.91 (ddd, J=2.3, 5.7, 12.1 Hz, 1H), 1.65 (s, 3H), 1.62 (s, 3H), 1.61 - 1.54 (m, 1H). MS (ESI): m / z 358 (M+1). HPLC purity: 93.9%.

### (1'R,2'R,4'S)-4-(2,2-Difluorobenzo[d][1,3]dioxol-5-yl)-5'-methyl-2'-(prop-1-en-2-yl)-1',2',3',4'-tetrahydro-[1,1'-biphenyl]-2,4',6-triol (compound 43)

¹H NMR (400 MHz, DMSO) d 9.29 - 9.25 (m, 1H), 9.11 - 9.07 (m, 1H), 7.48 - 7.43 (m, 2H), 7.30 - 7.26 (m, 1H), 6.43 - 6.42 (m, 2H), 5.11 (d, J=1.5 Hz, 1H), 4.62 (d, J=6.5 Hz, 1H), 4.54 (d, J=2.6 Hz, 1H), 4.47 - 4.45 (m, 1H), 4.13 (d, J=5.6 Hz, 1H), 3.97 - 3.92 (m, 1H), 3.30 - 3.22 (m, 1H), 1.96 - 1.89 (m, 1H), 1.67 - 1.63 (m, 6H), 1.63 - 1.55 (m, 1H). MS (ESI): m / z 417 (M+1). HPLC purity: 96.3%.

### (1'R,2'R,4'S)-5'-Methyl-4-(2-methylpyridin-4-yl)-2'-(prop-1-en-2-yl)-1',2',3',4'-tetrahydro-[1,1'-biphenyl]-2,4',6-triol (compound 28)

¹H NMR (400 MHz, DMSO) d 9.37 - 9.36 (m, 1H), 9.14 - 9.12 (m, 1H), 8.45 (d, J=5.3 Hz, 1H), 7.32 (s, 1H), 7.25 (dd, J=1.4, 5.2 Hz, 1H), 6.56 (s, 2H), 5.11 (d, J=1.5 Hz, 1H), 4.63 (d, J=6.7 Hz, 1H), 4.53 (d, J=2.6 Hz, 1H), 4.45 (dd, J=1.4, 2.5 Hz, 1H), 4.13 (d, J=4.4 Hz, 1H), 3.99 - 3.95 (m, 1H), 3.29 - 3.22 (m, 1H), 2.53 (s, 3H), 1.96 - 1.90 (m, 1H), 1.68 - 1.63 (m, 6H), 1.62 - 1.55 (m, 1H). MS (ESI): m / z 352 (M+1). HPLC purity: 98.4%.

### (1R,2R,4S)-2"-Methoxy-5-methyl-2-(prop-1-en-2-yl)-1,2,3,4-tetrahydro-[1,1':4',1"-terphenyl]-2',4,6'-triol (compound 29)

¹H NMR (400 MHz, DMSO) d 9.06 (s, 1H), 8.88 - 8.79 (m, 1H), 7.35 - 7.30 (m, 1H), 7.22 (dd, J=1.8, 7.6 Hz, 1H), 7.10 (d, J=7.6 Hz, 1H), 7.04 - 7.00 (m, 1H), 6.41 - 6.39 (m, 2H), 5.16 (d, J=1.5 Hz, 1H), 4.65 - 4.62 (m, 2H), 4.53 - 4.51 (m, 1H), 4.18 - 4.14 (m, 1H), 4.02 - 3.97 (m, 1H), 3.80 (s, 3H), 3.35 - 3.29 (m, 1H), 2.05 - 1.94 (m, 1H), 1.70 - 1.69 (m, 6H), 1.66 - 1.58 (m, 1H). MS (ESI): m / z 367 (M+1). HPLC purity: 99.5%.

### (1R,2R,4S)-4"-Fluoro-3"-methoxy-5-methyl-2-(prop-1-en-2-yl)-1,2,3,4-tetrahydro-[1,1':4',1"-terphenyl]-2',4,6'-triol (compound 30)

¹H NMR (400 MHz, DMSO) d 9.26 (s, 1H), 9.07 - 8.98 (m, 1H), 7.32 - 7.21 (m, 2H), 7.04 (ddd, J=1.8, 4.1, 8.4 Hz, 1H), 6.53 - 6.49 (m, 2H), 5.15 (s, 1H), 4.66 - 4.57 (m, 2H), 4.49 (s, 1H), 4.16 (d, J=5.3 Hz, 1H), 4.00 - 3.96 (m, 1H), 3.95 - 3.93 (m, 3H), 3.34 - 3.26 (m, 1H), 1.99 - 1.95 (m, 1H), 1.72 - 1.67 (m, 6H), 1.66 - 1.59 (m, 1H). MS (ESI): m / z 385 (M+1). HPLC purity: 97.4%.

### (1R,2R,4S)-3"-(Difluoromethoxy)-5-methyl-2-(prop-1-en-2-yl)-1,2,3,4-tetrahydro-[1,1':4',1"-terphenyl]-2',4,6'-triol (compound 31)

¹H NMR (400 MHz, DMSO) d 9.36 - 9.28 (m, 1H), 9.14 - 9.09 (m, 1H), 7.55 - 7.15 (m, 5H), 6.58 - 6.53 (m, 2H), 5.17 - 5.14 (m, 1H), 4.65 (d, J=6.6 Hz, 1H), 4.58 (s, 1H), 4.49 (s, 1H), 4.19 - 4.14 (m, 1H), 4.01 - 3.98 (m, 1H), 3.34 - 3.27 (m, 1H), 1.98 ( dd, J=5.8, 9.9 Hz, 1H), 1.72 - 1.67 (m, 6H), 1.67 - 1.59 (m, 1H). MS (ESI): m / z 403 (M+1). HPLC purity: 95.1%.

### (1R,2R,4S)-3"-(Aminomethyl)-5-methyl-2-(prop-1-en-2-yl)-1,2,3,4-tetrahydro-[1,1':4',1"-terphenyl]-2',4,6'-triol (compound 32)

¹H NMR (400 MHz, DMSO) d 9.10 (s, 1H), 8.87 (t, J=12.8 Hz, 1H), 7.36 - 7.28 (m, 3H), 7.15 - 7.13 (m, 1H), 6.46 - 6.39 (m, 2H), 5.05 (d, J=1.3 Hz, 1H), 4.54 - 4.45 (m, 2H), 4.37 (d, J=1.3 Hz, 1H), 4.09 - 4.01 (m, 1H), 3.90 - 3.86 (m, 1H), 3.36 (s, 2H), 3.24 - 3.15 (m, 1H), 2.11 - 2.09 (m, 6H), 1.88 - 1.78 (m, 1H), 1.60 - 1.55 (m, 6H), 1.55 - 1.48 (m, 1H). MS (ESI): m / z 394 (M+1). HPLC purity: 92.7%.

### (1R,2R,4S)-4"-Methoxy-5-methyl-2-(prop-1-en-2-yl)-1,2,3,4-tetrahydro-[1,1':4',1"-terphenyl]-2',4,6'-triol (compound 33)

¹H NMR (400 MHz, DMSO) d 9.26 - 9.19 (m, 1H), 9.05 - 8.99 (m, 1H), 7.51 - 7.48 (m, 2H), 7.10 - 7.07 (m, 2H), 6.52 - 6.51 (m, 2H), 5.22 - 5.20 (m, 1H), 4.71 - 4.63 (m, 2H), 4.55 - 4.54 (m, 1H), 4.25 - 4.18 (m, 1H), 4.05 - 4.02 (m, 1H), 3.89 - 3.88 (m, 3H), 3.39 - 3.32 (m, 1H), 2.06 - 1.99 (m, 1H), 1.77 - 1.73 (m, 6H), 1.71 - 1.64 (m, 1H). MS (ESI): m / z 367 (M+1). HPLC purity: 94.8%.

### (1R,2R,4S)-5-Methyl-2-(prop-1-en-2-yl)-1,2,3,4-tetrahydro-[1,1':4',1"-terphenyl]-2',4,6'-triol (compound 14)

¹H NMR (400 MHz, DMSO) d 9.29 - 9.20 (m, 1H), 9.03 (s, 1H), 7.53 - 7.44 (m, 4H), 7.38 - 7.33 (m, 1H), 6.53 - 6.51 (m, 2H), 5.16 (d, J=1.5 Hz, 1H), 4.66 - 4.58 (m, 2H), 4.51 - 4.49 (m, 1H), 4.19 - 4.13 (m, 1H), 4.01 - 3.96 (m, 1H), 3.34 - 3.27 (m, 1H), 2.00 - 1.94 (m, 1H), 1.72 - 1.68 (m, 6H), 1.67 - 1.59 (m, 1H). MS (ESI): m / z 337 (M+1). HPLC purity: 99.2%.

### (1'R,2'R,4'S)-4-(6-Ethoxypyridin-3-yl)-5'-methyl-2'-(prop-1-en-2-yl)-1',2',3',4'-tetrahydro-[1,1'-biphenyl]-2,4',6-triol (compound 34)

¹H NMR (400 MHz, DMSO) d 9.31 - 9.20 (m, 1H), 9.07 - 8.98 (m, 1H), 8.24 (d, J=2.5 Hz, 1H), 7.76 (dd, J=2.5, 8.7 Hz, 1H), 6.86 - 6.83 (m, 1H), 6.43 - 6.40 (m, 2H), 5.11 (s, 1H), 4.61 (d, J=6.5 Hz, 1H), 4.54 (s, 1H), 4.45 (s, 1H), 4.33 (q, J=7.0 Hz, 2H), 4.15 - 4.11 (m, 1H), 3.96 - 3.93 (m, 1H), 3.30 - 3.26 (m, 1H), 1.95 - 1.90 (m, 1H), 1.68 - 1.63 (m, 6H), 1.62 - 1.55 (m, 1H), 1.34 (t, J=7.1 Hz, 3H). MS (ESI): m / z 382 (M+1). HPLC purity: 93.3%.

### (1'R,2'R,4'S)-4-(2-Methoxypyridin-4-yl)-3,5,5'-trimethyl-2'-(prop-1-en-2-yl)-1',2',3',4'-tetrahydro-[1,1'-biphenyl]-2,4',6-triol (compound 52)

¹H NMR (400 MHz, DMSO) d 8.20 (dd, J=0.6, 5.2 Hz, 1H), 7.89 (s, 1H), 7.51 (s, 1H), 6.73 - 6.67 (m, 1H), 6.50 (s, 1H), 5.18 (s, 1H), 4.57 (s, 1H), 4.48 (dd, J=1.4, 2.5 Hz, 1H), 4.17 - 4.10 (m, 2H), 3.89 (s, 3H), 3.31 (t, J=11.7 Hz, 1H), 1.95 (ddd, J=2.3, 5.3, 11.9 Hz, 1H), 1.74 (s, 3H), 1.71 (s, 3H), 1.69 (s, 3H), 1.65 (s, 3H), 1.67 - 1.61 (m, 1H). (OH not observed) MS (ESI): m / z 396 (M+1). HPLC purity: 99%.

### General Method E

General Method E is illustrated by the synthesis of (1'*R*,2'*R*,4'*S*)-5'-Methyl-2'-(prop-1-en-2-yl)-4-(5-(trifluoromethyl)pyridin-3-yl)-1',2',3',4'-tetrahydro-[1,1'-biphenyl]-2,4',6-triol **(compound 25)**

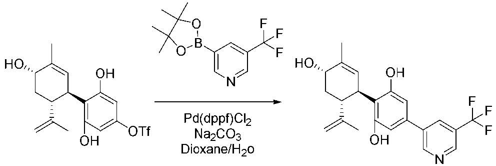

A mixture of [1,1'-bis(diphenylphosphino)ferrocene]dichloropalladium(II) (7.3 mg, 9.79 µmol), (1'*R*,2'*R*,4'*S*)-2,4',6-trihydroxy-5'-methyl-2'-(prop-1-en-2-yl)-1',2',3',4'-tetrahydro-[1,1'-biphenyl]-4-yl trifluoromethanesulfonate (80 mg, 0.196 mmol), sodium carbonate (83 mg, 0.784 mmol) and 3-(4,4,5,5-tetramethyl-1,3,2-dioxaborolan-2-yl)-5-(trifluoromethyl)pyridine (59 mg, 0.215 mmol) in 1,4-dioxane (2.0 mL) and water (0.50 mL) was heated in a sealed tube at 100°C for 1 hour. The reaction mixture was diluted with ethyl acetate (50 mL) and washed with water (10 mL), brine (10 mL), dried (MgSO₄), filtered and concentrated *in vacuo.* The residue was purified by column chromatography on silica, eluting with 0-100% ethyl acetate in cyclohexane, followed by reverse phase preparative HPLC to give the title compound (35 mg, 44%) as an off-white solid.

¹H NMR (400 MHz, DMSO) d 9.44 (s, 1H), 9.21 (s, 1H), 8.99 (d, J=2.0 Hz, 1H), 8.94 - 8.92 (m, 1H), 8.18 (t, J=2.0 Hz, 1H), 6.59 - 6.55 (m, 2H), 5.13 - 5.10 (m, 1H), 4.64 (d, J=6.8 Hz, 1H), 4.54 (d, J=2.7 Hz, 1H), 4.46 (dd, J=1.4, 2.7 Hz, 1H), 4.17 - 4.09 (m, 1H), 4.01 - 3.96 (m, 1H), 3.31 - 3.23 (m, 1H), 1.94 (ddd, J=2.4, 5.7, 11.9 Hz, 1H), 1.67 (s, 3H), 1.65 (s, 3H), 1.63 - 1.56 (m, 1H). MS (ESI): m / z 406.3 (M+1). HPLC purity: 98.6%.

General Method E, using the appropriate boronic acid or boronic ester, was used to generate the following compounds.

### (1'R,2'R,4'S)-4-(3,6-Dihydro-2H-pyran-4-yl)-5'-methyl-2'-(prop-1-en-2-yl)-1',2',3',4'-tetrahydro-[1,1'-biphenyl]-2,4',6-triol (compound 26)

¹H NMR (400 MHz, DMSO) d 9.05 (s, 1H), 8.83 (s, 1H), 6.29 (s, 2H), 6.01 - 5.98 (m, 1H), 5.08 (q, J=1.5 Hz, 1H), 4.60 (d, J=6.8 Hz, 1H), 4.50 (d, J=2.4 Hz, 1H), 4.42 (q, J=1.4 Hz, 1H), 4.19 (q, J=2.7 Hz, 2H), 4.15 - 4.07 (m, 1H), 3.92 - 3.86 (m, 1H), 3.79 (t, J=5.4 Hz, 2H), 3.26 - 3.17 (m, 1H), 2.33 - 2.27 (m, 2H), 1.91 (ddd, J=2.3, 5.7, 12.1 Hz, 1H), 1.65 (s, 3H), 1.61 (s, 3H), 1.60 - 1.53 (m, 1H). MS (ESI): m / z 342.4 (M+1). HPLC purity: 94.4%.

### General Method F

General Method F is illustrated by the synthesis of (1'*R*,2'*R*,4'*S*)-4-(3,5-Dimethylisoxazol-4-yl)-5'-methyl-2'-(prop-1-en-2-yl)-1',2',3',4'-tetrahydro-[1,1'-biphenyl]-2,4',6-triol **(compound 12)**

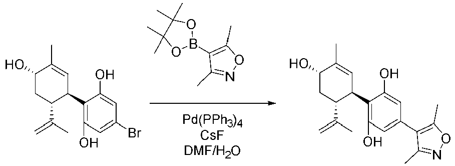

(1'*R*,2'*R*,4'*S*)-4-Bromo-5'-methyl-2'-(prop-1-en-2-yl)-1',2',3',4'-tetrahydro-[1,1'-biphenyl]-2,4',6-triol (100 mg, 0.295 mmol) was dissolved in dry N,N-dimethylformamide (1.70 mL) and water (0.30 mL). The mixture was degassed and 3,5-dimethylisoxazole-4-boronic acid pinacol ester (92 mg, 0.413 mmol), cesium fluoride (90 mg, 0.590 mmol) and tetrakis(triphenylphosphine)palladium(0) (34 mg, 0.0295 mmol) were added. The mixture was heated at 95°C overnight. The reaction was diluted with ethyl acetate (4 mL) and washed with water (2 mL) and brine (3 mL). The aqueous layer was extracted further with ethyl acetate (2 x 4 mL). The organic phases were combined, dried (hydrophobic frit) and concentrated *in vacuo.* The residue was purified by reverse phase preparative HPLC to give the title compound (12.0 mg, 10.7%) as an off-white solid.

¹H NMR (400 MHz, DMSO) d 9.24 (s, 1H), 9.01 (s, 1H), 6.21 (s, 2H), 5.13 - 5.10 (m, 1H), 4.61 (d, J=6.4 Hz, 1H), 4.55 (d, J=2.6 Hz, 1H), 4.46 (q, J=1.4 Hz, 1H), 4.16 - 4.09 (m, 1H), 3.96 - 3.92 (m, 1H), 3.29 - 3.21 (m, 1H), 2.39 (s, 3H), 2.21 (s, 3H), 1.93 (ddd, J=2.4, 5.7, 12.0 Hz, 1H), 1.66 (s, 3H), 1.64 (s, 3H), 1.62 - 1.54 (m, 1H). MS (ESI): m / z 355.4 (M+1). HPLC purity: 93.7%.

General Method F, using the appropriate boronic acid or boronic ester, was used to generate the following compound.

### (1R,2R,4S)-4"-fluoro-5-methyl-2-(prop-1-en-2-yl)-1,2,3,4-tetrahydro-[1,1':4',1"-terphenyl]-2',4,6'-triol (compound 18)

¹H NMR (400 MHz, DMSO) d 9.24 (s, 1H), 9.01 (s, 1H), 7.51 - 7.47 (m, 2H), 7.28 - 7.23 (m, 2H), 6.44 (s, 2H), 5.13 - 5.10 (m, 1H), 4.62 (d, J=6.7 Hz, 1H), 4.54 (d, J=2.6 Hz, 1H), 4.47 - 4.44 (m, 1H), 4.17 - 4.08 (m, 1H), 3.97 - 3.93 (m, 1H), 3.29 - 3.21 (m, 1H), 1.93 (ddd, J=2.3, 5.7, 11.9 Hz, 1H), 1.67 (s, 3H), 1.65 (s, 3H), 1.62 - 1.55 (m, 1H). MS (ESI): m / z 355 (M+1). HPLC purity: 98.3%.

### General Method G

General Method G is illustrated by the synthesis of (1'*R*,2'*R*,4'*S*)-4-(Benzo[d][1,3]dioxol-5-yl)-5'-methyl-2'-(prop-1-en-2-yl)-1',2',3',4'-tetrahydro-[1,1'-biphenyl]-2,4',6-triol **(compound 41)**

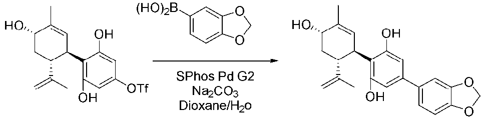

A solution of (1'*R*,2'*R*,4'*S*)-2,4',6-trihydroxy-5'-methyl-2'-(prop-1-en-2-yl)-1',2',3',4'-tetrahydro-[1,1'-biphenyl]-4-yl trifluoromethanesulfonate (80 mg, 0.196 mmol) in 1,4-dioxane (4.0 mL) and water (1.0 mL) was treated with 3,4-(methylenedioxy)phenylboronic acid (49 mg, 0.294 mmol) and sodium carbonate (62 mg, 0.588 mmol) followed by SPhos Pd G2 (14 mg, 0.0196 mmol). The reaction mixture was heated at 120°C under microwave irradiation for 1 hour. The mixture was diluted with water (5 mL) and extracted with ethyl acetate (30 mL). The organic layer was washed with brine (5 mL), dried (phase separation paper) and concentrated *in vacuo.* The residue was purified by reverse phase preparative HPLC to give the title compound (24 mg, 32%) as an off-white solid.

¹H NMR (400 MHz, DMSO) d 9.22 - 9.17 (m, 1H), 8.94 (s, 1H), 6.98 - 6.93 (m, 3H), 6.39 - 6.37 (m, 2H), 6.05 (s, 2H), 5.11 (d, J=1.4 Hz, 1H), 4.62 - 4.53 (m, 2H), 4.47 - 4.45 (m, 1H), 4.15 - 4.08 (m, 1H), 3.95 - 3.91 (m, 1H), 3.29 - 3.22 (m, 1H), 1.95 - 1.89 (m, 1H), 1.67 - 1.63 (m, 6H), 1.62 - 1.55 (m, 1H). MS (ESI): m / z 381 (M+1). HPLC purity: 99.1%.

General Method G, using the appropriate boronic acid or boronic ester, was used to generate the following compounds.

### (1'R,2'R,4'S)-4-(6-(Difluoromethoxy)pyridin-3-yl)-5'-methyl-2'-(prop-1-en-2-yl)-1',2',3',4'-tetrahydro-[1,1'-biphenyl]-2,4',6-triol (compound 19)

¹H NMR (400 MHz, DMSO) d 9.36 - 9.32 (m, 1H), 9.13 (s, 1H), 8.35 - 8.35 (m, 1H), 7.99 (dd, J=2.7, 8.7 Hz, 1H), 7.74 (t, J=72.9 Hz, 1H), 7.17 - 7.14 (m, 1H), 6.47 - 6.44 (m, 2H), 5.11 (d, J=1.5 Hz, 1H), 4.63 - 4.61 (m, 1H), 4.55 (d, J=2.5 Hz, 1H), 4.46 (dd, J=1.4, 2.6 Hz, 1H), 4.13 (d, J=5.4 Hz, 1H), 3.99 - 3.94 (m, 1H), 3.31 - 3.22 (m, 1H), 1.97 - 1.89 (m, 1H), 1.65 (d, J=7.0 Hz, 6H), 1.63 - 1.55 (m, 1H). MS (ESI): m / z 404 (M+1). HPLC purity: 99%.

### (1'R,2'R,4'S)-4-(Benzo[d][1,3]dioxol-4-yl)-5'-methyl-2'-(prop-1-en-2-yl)-1',2',3',4'-tetrahydro-[1,1'-biphenyl]-2,4',6-triol (compound 42)

¹H NMR (400 MHz, DMSO) d 9.24 - 9.18 (m, 1H), 9.00 (d, J=3.8 Hz, 1H), 7.00 - 6.90 (m, 3H), 6.64 (s, 2H), 6.09 (s, 2H), 5.16 (d, J=1.5 Hz, 1H), 4.65 - 4.57 (m, 2H), 4.49 (dd, J=1.4, 2.7 Hz, 1H), 4.17 - 4.13 (m, 1H), 4.02 - 3.96 (m, 1H), 3.34 - 3.26 (m, 1H), 2.00 - 1.94 (m, 1H), 1.71 - 1.67 (m, 6H), 1.66 - 1.59 (m, 1H). MS (ESI): m / z 381 (M+1). HPLC purity: 95.8%.

### (1'R,2'R,4'S)-4-(2-Methoxypyridin-3-yl)-5'-methyl-2'-(prop-1-en-2-yl)-1',2',3',4'-tetrahydro-[1,1'-biphenyl]-2,4',6-triol (compound 35)

¹H NMR (400 MHz, DMSO) d 9.10 - 8.99 (m, 1H), 8.82 (s, 1H), 8.03 (dd, J=1.9, 4.9 Hz, 1H), 7.52 (dd, J=1.8, 7.3 Hz, 1H), 6.96 (dd, J=5.1, 7.3 Hz, 1H), 6.33 (bs, 2H), 5.02 (d, J=1.5 Hz, 1H), 4.52 - 4.47 (m, 2H), 4.39 - 4.38 (m, 1H), 4.07 - 4.00 (m, 1H), 3.89 - 3.84 (m, 1H), 3.79 (s, 3H), 3.22 - 3.15 (m, 1H), 1.88 - 1.80 (m, 1H), 1.58 - 1.55 (m, 6H), 1.53 - 1.46 (m, 1H). MS (ESI): m / z 368 (M+1). HPLC purity: 99.2%.

### 1-(4-((1'R,2'R,4'S)-2,4',6-Trihydroxy-5'-methyl-2'-(prop-1-en-2-yl)-1',2',3',4'-tetrahydro-[1,1'-biphenyl]-4-yl)-3,6-dihydropyridin-1(2H)-yl)ethan-1-one (compound 36)

¹H NMR (400 MHz, DMSO) d 9.08 (s, 1H), 8.91 - 8.83 (m, 1H), 6.32 - 6.28 (m, 2H), 5.97 (d, J=3.8 Hz, 1H), 5.12 (d, J=1.3 Hz, 1H), 4.62 (d, J=6.6 Hz, 1H), 4.53 (d, J=2.3 Hz, 1H), 4.46 (s, 1H), 4.18 - 4.07 (m, 3H), 3.95 - 3.90 (m, 1H), 3.68 - 3.60 (m, 2H), 3.29 - 3.21 (m, 1H), 2.45 (s, 1H), 2.35 - 2.31 (m, 1H), 2.09 (d, J=15.9 Hz, 3H), 1.98 - 1.91 (m, 1H), 1.70 - 1.64 (m, 6H), 1.64 - 1.57 (m, 1H). MS (ESI): m / z 384 (M+1). HPLC purity: 97.1%.

### (1R,2R,4S)-3"-(2-Methoxyethoxy)-5-methyl-2-(prop-1-en-2-yl)-1,2,3,4-tetrahydro-[1,1':4',1"-terphenyl]-2',4,6'-triol (compound 37)

¹H NMR (400 MHz, DMSO) d 9.21 - 9.16 (m, 1H), 9.01 - 8.98 (m, 1H), 7.33 (t, J=8.0 Hz, 1H), 7.05 - 6.97 (m, 2H), 6.90 (dd, J=1.8, 8.2 Hz, 1H), 6.47 - 6.46 (m, 2H), 5.11 (d, J=1.5 Hz, 1H), 4.61 (d, J=6.7 Hz, 1H), 4.55 (d, J=2.5 Hz, 1H), 4.46 (d, J=1.3 Hz, 1H), 4.16 - 4.10 (m, 3H), 3.98 - 3.93 (m, 1H), 3.70 - 3.67 (m, 2H), 3.29 - 3.22 (m, 4H), 1.96 - 1.90 (m, 1H), 1.68 - 1.63 (m, 6H), 1.63 - 1.55 (m, 1H). MS (ESI): m / z 411 (M+1). HPLC purity: 97%.

### (1'R,2'R,4'S)-5'-Methyl-4-((E)-prop-1-en-1-yl)-2'-(prop-1-en-2-yl)-1',2',3',4'-tetrahydro-[1,1'-biphenyl]-2,4',6-triol (compound 7)

¹H NMR (400 MHz, DMSO) d 8.99 - 8.93 (m, 1H), 8.76 (s, 1H), 6.21 - 6.11 (m, 3H), 5.97 (ddd, J=4.5, 11.1, 17.7 Hz, 1H), 5.08 (d, J=1.5 Hz, 1H), 4.59 (d, J=6.7 Hz, 1H), 4.49 (d, J=2.6 Hz, 1H), 4.42 (dd, J=1.4, 2.6 Hz, 1H), 4.09 (d, J=5.1 Hz, 1H), 3.91 - 3.85 (m, 1H), 3.24 - 3.15 (m, 1H), 1.91 - 1.83 (m, 1H), 1.82 - 1.79 (m, 3H), 1.64 (s, 3H), 1.60 (s, 3H), 1.57 - 1.52 (m, 1H). MS (ESI): m / z 301 (M+1). HPLC purity: 97.4%.

### (1'R,2'R,4'S)-4-(Cyclopent-1-en-1-yl)-5'-methyl-2'-(prop-1-en-2-yl)-1',2',3',4'-tetrahydro-[1,1'-biphenyl]-2,4',6-triol (compound 9)

¹H NMR (400 MHz, DMSO) d 8.99 - 8.94 (m, 1H), 8.76 (s, 1H), 6.35 - 6.27 (m, 2H), 5.97 (d, J=1.9 Hz, 1H), 5.08 (d, J=1.5 Hz, 1H), 4.60 - 4.57 (m, 1H), 4.50 - 4.47 (m, 1H), 4.43 - 4.41 (m, 1H), 4.10 (d, J=5.4 Hz, 1H), 3.90 - 3.87 (m, 1H), 3.25 - 3.16 (m, 1H), 2.48 - 2.41 (m, 2H), 1.97 - 1.88 (m, 3H), 1.66 - 1.59 (m, 9H). MS (ESI): m / z 327 (M+1). HPLC purity: 97.3%.

### (1'R,2'R,4'S)-5'-Methyl-2'-(prop-1-en-2-yl)-4-(6-(trifluoromethoxy)pyridin-3-yl)-1',2',3',4'-tetrahydro-[1,1'-biphenyl]-2,4',6-triol (compound 38)

¹H NMR (400 MHz, DMSO) d 9.47 - 9.42 (m, 1H), 9.26 - 9.18 (m, 1H), 8.50 (d, J=2.0 Hz, 1H), 8.11 (dd, J=2.5, 8.6 Hz, 1H), 7.41 - 7.37 (m, 1H), 6.57 - 6.48 (m, 2H), 5.15 (d, J=1.5 Hz, 1H), 4.67 - 4.57 (m, 2H), 4.51 - 4.49 (m, 1H), 4.17 (dd, J=2.8, 5.1 Hz, 1H), 4.03 - 3.99 (m, 1H), 3.34 - 3.27 (m, 1H), 2.01 - 1.94 (m, 1H), 1.72 - 1.68 (m, 7H). MS (ESI): m / z 422 (M+1). HPLC purity: 97.8%.

### (1'R,2'R,4'S)-4-(2-(Difluoromethoxy)pyridin-4-yl)-5'-methyl-2'-(prop-1-en-2-yl)-1',2',3',4'-tetrahydro-[1,1'-biphenyl]-2,4',6-triol (compound 39)

¹H NMR (400 MHz, DMSO) d 9.47 - 9.40 (m, 1H), 9.28 - 9.20 (m, 1H), 8.28 (d, J=5.4 Hz, 1H), 7.75 (t, J=73.1 Hz, 1H), 7.36 (dd, J=1.6, 5.4 Hz, 1H), 7.08 (d, J=0.9 Hz, 1H), 6.59 - 6.53 (m, 2H), 5.11 (d, J=1.5 Hz, 1H), 4.64 - 4.62 (m, 1H), 4.53 (d, J=2.5 Hz, 1H), 4.45 (dd, J=1.4, 2.6 Hz, 1H), 4.14 - 4.10 (m, 1H), 4.00 - 3.96 (m, 1H), 3.30 - 3.22 (m, 1H), 1.97 - 1.90 (m, 1H), 1.68 - 1.63 (m, 7H). MS (ESI): m / z 404 (M+1). HPLC purity: 95.4%.

### (1'R,2'R,4'S)-4-(2-(Difluoromethoxy)pyridin-3-yl)-5'-methyl-2'-(prop-1-en-2-yl)-1',2',3',4'-tetrahydro-[1,1'-biphenyl]-2,4',6-triol (compound 40)

¹H NMR (400 MHz, DMSO) d 9.32 - 9.26 (m, 1H), 9.10 - 9.06 (m, 1H), 8.23 - 8.21 (m, 1H), 7.86 - 7.78 (m, 2H), 7.34 (dd, J=4.9, 7.5 Hz, 1H), 6.42 - 6.39 (m, 2H), 5.13 (d, J=1.5 Hz, 1H), 4.63 - 4.56 (m, 2H), 4.49 - 4.48 (m, 1H), 4.13 (d, J=5.5 Hz, 1H), 3.99 - 3.96 (m, 1H), 3.32 - 3.25 (m, 1H), 1.97 - 1.90 (m, 1H), 1.68 - 1.64 (m, 7H). MS (ESI): m / z 404 (M+1). HPLC purity: 98.9%.

### (1'R,2'R,4'S)-5'-Methyl-2'-(prop-1-en-2-yl)-4-(2-(trifluoromethyl)pyridin-4-yl)-1',2',3',4'-tetrahydro-[1,1'-biphenyl]-2,4',6-triol (compound 58)

¹H NMR (400 MHz, DMSO) d 9.49 - 9.45 (m, 1H), 9.27 - 9.25 (m, 1H), 8.79 (d, J=5.1 Hz, 1H), 7.88 (s, 1H), 7.80 (dd, J=1.4, 5.1 Hz, 1H), 6.66 (d, J=1.9 Hz, 2H), 5.11 (d, J=1.5 Hz, 1H), 4.64 (d, J=6.7 Hz, 1H), 4.53 (d, J=2.5 Hz, 1H), 4.45 (dd, J=1.4, 2.6 Hz, 1H), 4.16 - 4.09 (m, 1H), 4.01 - 3.97 (m, 1H), 3.31 - 3.24 (m, 1H), 1.97 - 1.90 (m, 1H), 1.68 - 1.63 (m, 7H). MS (ESI): m / z 406 (M+1). HPLC purity: 99.2%.

### (1'R,2'R,4'S)-4-(6-(2,2-Difluoroethyl)-5-fluoropyridin-3-yl)-5'-methyl-2'-(prop-1-en-2-yl)-1',2',3',4'-tetrahydro-[1,1'-biphenyl]-2,4',6-triol (compound 59)

¹H NMR (400 MHz, DMSO) d 9.47 - 9.20 (m, 2H), 8.23 - 8.22 (m, 1H), 8.07 - 7.97 (m, 1H), 7.63 - 7.61 (m, 1H), 6.57 - 6.50 (m, 2H), 5.14 (d, J=1.5 Hz, 1H), 4.67 - 4.64 (m, 1H), 4.58 (d, J=2.5 Hz, 1H), 4.50 (d, J=1.3 Hz, 1H), 4.17 (d, J=5.6 Hz, 1H), 4.04 - 4.00 (m, 1H), 3.34 - 3.26 (m, 1H), 2.01 - 1.93 (m, 1H), 1.72 - 1.67 (m, 7H). MS (ESI): m / z 422 (M+1). HPLC purity: 95.2%.

General Method B (3 step oxidation with LAH reduction), using the appropriate 4-substituted (1'R,2'R)-5'-methyl-2'-(prop-1-en-2-yl)-1',2',3',4'-tetrahydro-[1,1'-biphenyl]-2,6-diol, was used to generate the following compounds.

### (1'R,2'R,4'S)-5'-Methyl-4-(1-methyl-1H-pyrazol-4-yl)-2'-(prop-1-en-2-yl)-1',2',3',4'-tetrahydro-[1,1'-biphenyl]-2,4',6-triol (compound 10)

¹H NMR (400 MHz, DMSO) d 9.06 (s, 1H), 8.82 (s, 1H), 7.83 (s, 1H), 7.54 (s, 1H), 6.34 (s, 2H), 5.12 - 5.11 (m, 1H), 4.60 (d, J=6.7 Hz, 1H), 4.53 - 4.50 (m, 1H), 4.45 - 4.43 (m, 1H), 4.15 - 4.08 (m, 1H), 3.94 - 3.87 (m, 1H), 3.86 - 3.85 (m, 3H), 3.25 - 3.19 (m, 1H), 1.91 (ddd, J=2.3, 5.8, 12.0 Hz, 1H), 1.67 - 1.61 (m, 6H), 1.57 (d, J=22.7 Hz, 1H). MS (ESI): m / z 341 (M+1). HPLC purity: 98.6%.

### (1'R,2'R,4'S)-4-(Methoxymethyl)-5'-methyl-2'-(prop-1-en-2-yl)-1',2',3',4'-tetrahydro-[1,1'-biphenyl]-2,4',6-triol (compound 4)

¹H NMR (400 MHz, DMSO) d 9.00 (s, 1H), 8.78 (s, 1H), 6.18 (s, 2H), 5.08 (q, J=1.9 Hz, 1H), 4.58 (d, J=6.7 Hz, 1H), 4.49 (d, J=2.7 Hz, 1H), 4.42 (q, J=1.4 Hz, 1H), 4.17 (s, 2H), 4.14 - 4.06 (m, 1H), 3.93 - 3.86 (m, 1H), 3.24 (s, 3H), 3.23 - 3.17 (m, 1H), 1.91 (ddd, J=2.4, 5.6, 12.0 Hz, 1H), 1.65 (s, 3H), 1.60 (s, 3H), 1.60 - 1.54 (m, 1H). MS (ESI): m / z 303 (M-1) (only negative ion observed). HPLC purity: 92.5%.

### (1'R,2'R,4'S)-4-(2-Methoxyethyl)-5'-methyl-2'-(prop-1-en-2-yl)-1',2',3',4'-tetrahydro-[1,1'-biphenyl]-2,4',6-triol (compound 5)

¹H NMR (400 MHz, DMSO) d 8.89 (s, 1H), 8.66 (s, 1H), 6.08 (s, 2H), 5.08 - 5.05 (m, 1H), 4.58 (d, J=7.0 Hz, 1H), 4.50 (d, J=2.6 Hz, 1H), 4.42 (q, J=1.4 Hz, 1H), 4.13 - 4.05 (m, 1H), 3.91 - 3.85 (m, 1H), 3.45 (t, J=6.9 Hz, 2H), 3.24 (s, 3H), 3.23 - 3.16 (m, 1H), 2.55 (t, J=6.9 Hz, 2H), 1.89 (ddd, J=2.5, 5.7, 12.0 Hz, 1H), 1.64 (s, 3H), 1.60 (s, 3H), 1.59 - 1.51 (m, 1H). MS (ESI): m / z 319 (M+1). HPLC purity: 95.6%.

### (1'R,2'R,4'S)-4,5'-Dimethyl-2'-(prop-1-en-2-yl)-1',2',3',4'-tetrahydro-[1,1'-biphenyl]-2,4',6-triol (compound 1)

¹H NMR (400 MHz, DMSO) d 8.86 (s, 1H), 8.62 (s, 1H), 6.00 (s, 2H), 5.07 (q, J=1.6 Hz, 1H), 4.58 (d, J=6.5 Hz, 1H), 4.49 (d, J=2.8 Hz, 1H), 4.41 (q, J=1.4 Hz, 1H), 4.14 - 4.05 (m, 1H), 3.89 - 3.83 (m, 1H), 3.24 - 3.15 (m, 1H), 2.05 (s, 3H), 1.89 (ddd, J=2.5, 5.8, 12.2 Hz, 1H), 1.64 (s, 3H), 1.60 (s, 3H), 1.59 - 1.52 (m, 1H). MS (ESI): m / z 275 (M+1). HPLC purity: 95.2%.

### (1'R,2'R,4'S)-4-Ethyl-5'-methyl-2'-(prop-1-en-2-yl)-1',2',3',4'-tetrahydro-[1,1'-biphenyl]-2,4',6-triol (compound 3)

¹H NMR (400 MHz, DMSO) d 8.87 (s, 1H), 8.64 (s, 1H), 6.04 (s, 2H), 5.08 - 5.05 (m, 1H), 4.58 (d, J=6.3 Hz, 1H), 4.50 (d, J=2.6 Hz, 1H), 4.42 (q, J=1.3 Hz, 1H), 4.13 - 4.06 (m, 1H), 3.89 - 3.84 (m, 1H), 3.24 - 3.16 (m, 1H), 2.35 (q, J=7.6 Hz, 2H), 1.90 (ddd, J=2.4, 5.6, 12.0 Hz, 1H), 1.64 (s, 3H), 1.61 (s, 3H), 1.59 - 1.52 (m, 1H), 1.09 (t, J=7.6 Hz, 3H). MS (ESI): m / z 289 (M+1). HPLC purity: 99.6%.

### 2-((1'R,2'R,4'S)-2,4',6-Trihydroxy-5'-methyl-2'-(prop-1-en-2-yl)-1',2',3',4'-tetrahydro-[1,1'-biphenyl]-4-yl)acetonitrile (compound 6)

¹H NMR (400 MHz, DMSO) d 9.32 - 9.27 (m, 1H), 9.06 - 9.04 (m, 1H), 6.22 (s, 2H), 5.12 - 5.10 (m, 1H), 4.64 - 4.62 (m, 1H), 4.53 (d, J=2.8 Hz, 1H), 4.46 (dd, J=1.4, 2.7 Hz, 1H), 4.14 (d, J=5.8 Hz, 1H), 3.98 - 3.91 (m, 1H), 3.84 (s, 2H), 3.29 - 3.20 (m, 1H), 1.98 - 1.91 (m, 1H), 1.70 - 1.63 (m, 6H), 1.62 - 1.58 (m, 1H). MS (ESI): m / z 298 (M-1) (only negative ion observed). HPLC purity: 93.4%.

### (1'R,2'R,4'S)-3,5'-Dimethyl-2'-(prop-1-en-2-yl)-4-propyl-1',2',3',4'-tetrahydro-[1,1'-biphenyl]-2,4',6-triol (compound 44)

¹H NMR (400 MHz, DMSO) d 8.69 - 8.41 (m, 1H), 7.70 - 7.26 (m, 1H), 6.13 - 6.05 (m, 1H), 5.15 - 5.07 (m, 1H), 4.61 - 4.47 (m, 2H), 4.41 (s, 1H), 4.12 (s, 1H), 3.93 - 3.89 (m, 1H), 3.25 - 3.12 (m, 1H), 2.38 - 2.32 (m, 2H), 1.99 - 1.92 (m, 3H), 1.91 - 1.87 (m, 1H), 1.68 - 1.57 (m, 7H), 1.48 - 1.43 (m, 2H), 0.94 - 0.89 (m, 3H). MS (ESI): m / z 317 (M+1). HPLC purity: 93.7%.

### (1'R,2'R,4'S)-4-bromo-3,5,5'-trimethyl-2'-(prop-1-en-2-yl)-1',2',3',4'-tetrahydro-[1,1'-biphenyl]-2,4',6-triol (compound 51)

¹H NMR (400 MHz, DMSO) d 8.21 (s, 1H), 7.85 (s, 1H), 5.13 (dd, J=1.5, 3.3 Hz, 1H), 4.67 (d, J=6.5 Hz, 1H), 4.46 (d, J=2.5 Hz, 1H), 4.42 (dd, J=1.4, 2.5 Hz, 1H), 4.15 - 4.11 (m, 1H), 4.06 - 4.02 (m, 1H), 3.22 (ddd, J=2.2, 10.7, 12.8 Hz, 1H), 2.19 (s, 3H), 2.16 (s, 3H), 1.92 (ddd, J=2.4, 5.7, 12.0 Hz, 1H), 1.67 (s, 3H), 1.65 - 1.61 (m, 1H), 1.60 (s, 3H). MS (ESI): m / z 367/369 (M+1). HPLC purity: 95.2%.

General Method C (3 step oxidation with NaBH₄ reduction), using the appropriate 4-substituted (1'*R*,2*'*R)-5'-methyl-2'-(prop-1-en-2-yl)-1',2',3',4'-tetrahydro-[1,1'-biphenyl]-2,6-diol, was used to generate the following compounds.

### (1'R,2'R,4'S)-4-Chloro-5'-methyl-2'-(prop-1-en-2-yl)-1',2',3',4'-tetrahydro-[1,1'-biphenyl]-2,4',6-triol (compound 2)

¹H NMR (400 MHz, DMSO) d 9.53 (s, 1H), 9.32 (s, 1H), 6.24 (s, 2H), 5.11 - 5.04 (m, 1H), 4.66 - 4.57 (m, 1H), 4.51 - 4.42 (m, 2H), 4.12 - 4.10 (m, 1H), 3.92 - 3.86 (m, 1H), 3.20 - 3.11 (m, 1H), 2.14 - 2.07 (m, 1H), 1.95 - 1.87 (m, 1H), 1.68 - 1.56 (m, 6H). Peak broadening possibly caused by atroposomerism. MS (ESI): m / z 293, 295 (M+1). HPLC purity: 99.2%.

### General Method H

General Method H is illustrated by the synthesis of (1'*R*,2'*R*,4'*S*)-3-fluoro-5'-methyl-2'-(prop-1-en-2-yl)-4-propyl-1',2',3',4'-tetrahydro-[1,1'-biphenyl]-2,4',6-triol **compound 45**

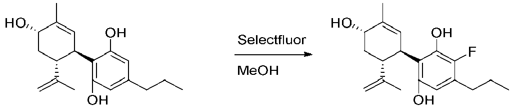

(1'*R*,2'*R*,4'*S*)-5'-methyl-2'-(prop-1-en-2-yl)-4-propyl-1',2',3',4'-tetrahydro-[1,1'-biphenyl]-2,4',6-triol (50 mg, 0.165 mmol) in methanol (2.0 mL) was treated with Selectfluor(R) fluorinating reagent (59 mg, 0.165 mmol) and stirred overnight at room temperature. The reaction mixture was poured into saturated ammonium chloride solution (10 mL) and extracted with dichloromethane (2 x 10 mL). The combined organic layers were dried (hydrophobic frit) and concentrated *in vacuo.* The residue was purified by column chromatography on silica, eluting with 10-80% ethyl acetate in cyclohexane, followed by reverse phase preparative HPLC to give the title compound as an off-white solid.

¹H NMR (400 MHz, DMSO) d 8.92 - 8.65 (m, 2H), 6.02 (s, 1H), 5.10 (s, 1H), 4.62 (d, J=6.7 Hz, 1H), 4.48 (d, J=2.0 Hz, 1H), 4.45 - 4.42 (m, 1H), 4.14 - 4.06 (m, 1H), 3.92 - 3.87 (m, 1H), 3.24 - 3.15 (m, 1H), 2.45 - 2.36 (m, 2H), 1.91 (ddd, J=2.2, 5.6, 12.0 Hz, 1H), 1.65 (s, 3H), 1.60 (s, 3H), 1.58 - 1.46 (m, 3H), 0.89 (t, J=7.3 Hz, 3H). MS (ESI): m / z 319 (M-1) (only negative ion observed). HPLC purity: 94.9%.

General Method H was used to generate the following compounds.

### (1'R,2'R,4'S)-3-Fluoro-5'-methyl-4-(1-methyl-1H-pyrazol-4-yl)-2'-(prop-1-en-2-yl)-1',2',3',4'-tetrahydro-[1,1'-biphenyl]-2,4',6-triol (compound 48)

¹H NMR (400 MHz, DMSO) d 9.14 - 8.83 (m, 2H), 7.94 (d, J=1.6 Hz, 1H), 7.64 (s, 1H), 6.38 (s, 1H), 5.15 - 5.11 (m, 1H), 4.64 (d, J=6.7 Hz, 1H), 4.51 (d, J=2.0 Hz, 1H), 4.46 - 4.43 (m, 1H), 4.15 - 4.09 (m, 1H), 3.95 - 3.90 (m, 1H), 3.89 (s, 3H), 3.26 - 3.18 (m, 1H), 1.93 (ddd, J=2.4, 5.9, 12.0 Hz, 1H), 1.66 (s, 3H), 1.62 (s, 3H), 1.60 - 1.55 (m, 1H). MS (ESI): m / z 359 (M+1). HPLC purity: 90.9%.

### General Method I

General Method I is illustrated by the synthesis of (1'*R*,2'*R*,4'*S*)-3-chloro-5'-methyl-2'-(prop-1-en-2-yl)-4-propyl-1',2',3',4'-tetrahydro-[1,1'-biphenyl]-2,4',6-triol **compound 46**

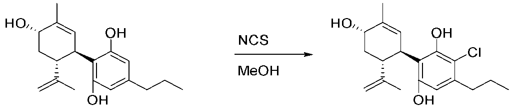

(1'*R*,2'*R*,4'*S*)-5'-Methyl-2'-(prop-1-en-2-yl)-4-propyl-1',2',3',4'-tetrahydro-[1,1'-biphenyl]-2,4',6-triol (50 mg, 0.165 mmol) in methanol (2.0 mL) was treated with N-chlorosuccinimide (22 mg, 0.165 mmol) and stirred overnight at room temperature. The reaction mixture was poured into water (25 ml) and extracted with ethyl acetate (2 x 25 mL). The combined organic layers were dried (hydrophobic filter paper) and concentrated *in vacuo.* The residue was purified by column chromatography on silica, eluting with 30-80 % diethyl ether in cyclohexane, to give the title compound as an off-white solid (30.2 mg, 54%).

¹H NMR (400 MHz, CD₃CN) d 6.93 - 6.48 (m, 2H), 6.32 (s, 1H), 5.29 - 5.26 (m, 1H), 4.52 - 4.49 (m, 2H), 4.32 - 4.25 (m, 1H), 4.04 - 3.98 (m, 1H), 3.13 - 3.06 (m, 1H), 2.89 (d, J=6.7 Hz, 1H), 2.63 - 2.55 (m, 2H), 2.04 (ddd, J=2.4, 5.7, 12.1 Hz, 1H), 1.76 (s, 3H), 1.74 - 1.67 (m, 4H), 1.66 - 1.56 (m, 2H), 0.97 (t, J=7.3 Hz, 3H). MS (ESI): m / z 335 (M-1) (only negative ion observed). HPLC purity: 96.2%.

General Method I was used to generate the following compounds.

### (1R,2R,4S)-3'-Chloro-3"-methoxy-5-methyl-2-(prop-1-en-2-yl)-1,2,3,4-tetrahydro-[1,1':4',1"-terphenyl]-2',4,6'-triol compound 49

Acetonitrile was used as solvent in place of methanol. ¹H NMR (400 MHz, DMSO) d 9.50 - 9.27 (m, 1H), 8.79 - 8.60 (m, 1H), 7.33 (t, J=7.9 Hz, 1H), 6.95 - 6.87 (m, 3H), 6.34 - 6.29 (m, 1H), 5.16 - 5.13 (m, 1H), 4.67 (d, J=6.5 Hz, 1H), 4.56 (d, J=2.4 Hz, 1H), 4.51 - 4.48 (m, 1H), 4.17 - 4.12 (m, 1H), 4.05 - 4.01 (m, 1H), 3.79 - 3.78 (m, 3H), 3.29 - 3.25 (m, 1H), 1.97 - 1.92 (m, 1H), 1.69 - 1.65 (m, 6H), 1.64 - 1.58 (m, 1H). MS (ESI): m / z 399 (M-1) (only negative ion observed). HPLC purity: 92.1%.

### (1'R,2'R,4'S)-4-bromo-3-chloro-5'-methyl-2'-(prop-1-en-2-yl)-1',2',3',4'-tetrahydro-[1,1'-biphenyl]-2,4',6-triol compound 47

¹H NMR (400 MHz, DMSO) d 9.80 - 9.58 (m, 1H), 9.20 - 9.03 (m, 1H), 6.68 - 6.62 (m, 1H), 5.12 - 5.07 (m, 1H), 4.67 (d, J=6.5 Hz, 1H), 4.46 (s, 2H), 4.11 (d, J=5.0 Hz, 1H), 3.96 - 3.95 (m, 1H), 3.21 - 3.15 (m, 1H), 1.94 - 1.90 (m, 1H), 1.67 - 1.60 (m, 6H), 1.59 - 1.56 (m, 1H). MS (ESI): m / z 371/373 (M-1) (only negative ion observed). HPLC purity: 93.3%.

### (1'R,2'R,4'S)-4'-Methoxy-5'-methyl-2'-(prop-1-en-2-yl)-4-propyl-1',2',3',4'-tetrahydro-[1,1'-biphenyl]-2,6-diol (compound 53)

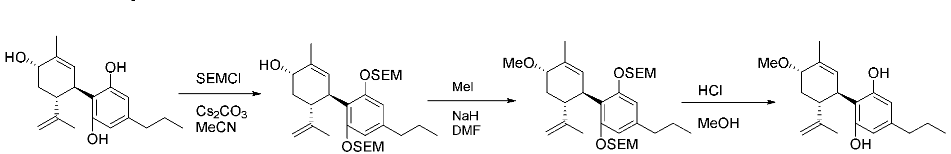

(1'*R*,2'*R*,4'*S*)-5'-methyl-2'-(prop-1-en-2-yl)-4-propyl-1',2',3',4'-tetrahydro-[1,1'-biphenyl]-2,4',6-triol (200 mg, 0.66 mmol) and cesium carbonate (474 mg, 1.45 mmol) in acetonitrile (10 mL) were treated with 2-(trimethylsilyl)ethoxymethyl chloride (0.25 mL, 1.39 mmol) dropwise over 20 minutes. The reaction mixture was stirred overnight, then poured into water (50 mL) and extracted with dichloromethane (3 x 50 mL) followed by ethyl acetate (3 x 50 ml). The organic layers were dried (hydrophobic filter paper) and concentrated *in vacuo* to give crude (1*R*,2*R*,4*S*)-5-Methyl-2-(prop-1-en-2-yl)-4'-propyl-2',6'-bis((2-(trimethylsilyl)ethoxy)methoxy)-1,2,3,4-tetrahydro-[1,1'-biphenyl]-4-ol as a colourless gum (316 mg, 85%). Without further purification a portion of this (233 mg, 0.41 mmol) was dissolved in *N*,*N*-dimethylformamide (3.0 mL), treated with sodium hydride (60% dispersion in mineral oil, 25 mg, 0.62 mmol) followed by iodomethane (39 mL, 0.62 mmol). The reaction mixture was stirred overnight, then treated with more sodium hydride (25 mg, 60% dispersion in mineral oil, 0.62 mmol) followed by iodomethane (39 mL, 0.62 mmol) and stirred over the weekend. The reaction mixture was quenched with saturated aqueous ammonium chloride solution (1 mL), diluted with ethyl acetate (20 mL) and washed with water (2 x 20 mL). The organic layer was dried (hydrophobic filter paper) and concentrated *in vacuo* to give crude ((((((1'*R*,2'*R*,4'*S*)-4'-methoxy-5'-methyl-2'-(prop-1-en-2-yl)-4-propyl-1',2',3',4'-tetrahydro-[1,1'-biphenyl]-2,6-diyl)bis(oxy))bis(methylene))bis(oxy))bis(ethane-2,1-diyl))bis(trimethylsilane) as a purple gum (148 mg, 62%). Without further purification a portion of this (120 mg, 0.21 mmol) in methanol (10 mL) was treated with aqueous 2 M hydrochloric acid (2 mL, 2 mmol) and stirred overnight. The reaction mixture was quenched with saturated sodium hydrogen carbonate solution (10 mL), diluted with ethyl acetate (50 mL), washed with water (2 x 50 mL) and concentrated *in vacuo.* The residue was purified by column chromatography on silica, eluting with 0-80% ethyl acetate in cyclohexane, followed by reverse phase preparative HPLC to give the title compound as an off-white solid (10.4 mg, 16%).

¹H NMR (400 MHz, DMSO) d 8.85 (s, 1H), 8.69 (s, 1H), 6.03 (s, 2H), 5.20 - 5.16 (m, 1H), 4.51 (d, J=2.5 Hz, 1H), 4.46 - 4.42 (m, 1H), 3.92 - 3.84 (m, 2H), 3.29 (s, 3H), 3.25 - 3.16 (m, 1H), 2.30 (t, J=7.6 Hz, 2H), 2.10 - 2.04 (m, 1H), 1.63 (s, 3H), 1.62 (s, 3H), 1.57 - 1.46 (m, 3H), 0.88 (t, J=7.3 Hz, 3H). MS (ESI): m / z 317 (M+1). HPLC purity: 97.2%.

### General Method J

General Method J is illustrated by the synthesis of (1'*R*,2*'*R)-4',5'-Dimethyl-2'-(prop-1-en-2-yl)-4-propyl-1',2',3',4'-tetrahydro-[1,1'-biphenyl]-2,4',6-triol **Compound 54**

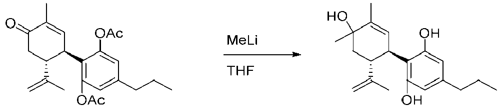

(1'R,2'R)-5'-Methyl-4'-oxo-2'-(prop-1-en-2-yl)-4-propyl-1',2',3',4'-tetrahydro-[1,1'-biphenyl]-2,6-diyl diacetate (184 mg, 0.479 mmol) in tetrahydrofuran (10 mL) was cooled to - 78°C and treated with 3.1 M methyllithium solution in dimethoxymethane (1.5 mL, 4.79 mmol). The reaction mixture was stirred at -78°C for 2 hours then allowed to warm to room temperature over 2 hours. The reaction mixture was quenched with saturated ammonium chloride (5 mL) and poured into water (50 mL) and extracted with dichloromethane (50 mL). The aqueous layer was acidified to pH2 with hydrochloric acid and extracted with further dichloromethane (2 x 50 mL). The combined organic layers were dried (hydrophobic frit) and concentrated *in vacuo.* The residue was purified by column chromatography on silica, eluting with 10-50% diethyl ether in cyclohexane followed by reverse phase preparative HPLC to give the title compound as an off-white solid (14.2 mg, 9.1%).

¹H NMR (400 MHz, DMSO) d 8.85 (s, 1H), 8.71 (s, 1H), 6.08 - 6.03 (m, 2H), 5.00 (s, 1H), 4.56 - 4.53 (m, 1H), 4.47 (s, 1H), 4.33 (s, 1H), 3.91 - 3.84 (m, 1H), 3.35 - 3.27 (m, 1H), 2.33 (t, J=7.9 Hz, 2H), 1.84 (t, J=12.9 Hz, 1H), 1.70 - 1.68 (m, 1H), 1.67 (s, 3H), 1.65 (s, 3H), 1.59 - 1.48 (m, 2H), 1.34 (s, 3H), 0.91 (t, J=7.3 Hz, 3H). MS (ESI): m / z 315 (M-1). HPLC purity: 96.8%.

General Method J, using the appropriate enone, was also used to generate the following compound.

### (1'R,2'R)-4',5'-dimethyl-4-pentyl-2'-(prop-1-en-2-yl)-1',2',3',4'-tetrahydro-[1,1'-biphenyl]-2,4',6-triol (compound 55):

¹H NMR (400 MHz, DMSO) d 8.82 (d, J=0.9 Hz, 1H), 8.67 (s, 1H), 6.04 - 5.98 (m, 2H), 4.97 - 4.94 (m, 1H), 4.50 (d, J=2.5 Hz, 1H), 4.45 - 4.42 (m, 1H), 4.30 (s, 1H), 3.86 - 3.80 (m, 1H), 3.31 - 3.23 (m, 1H), 2.30 (t, J=7.6 Hz, 2H), 1.80 (t, J=12.8 Hz, 1H), 1.65 - 1.64 (m, 1H), 1.64 - 1.62 (m, 3H), 1.61 (s, 3H), 1.52 - 1.43 (m, 2H), 1.33 - 1.23 (m, 7H), 0.87 (t, J=7.0 Hz, 3H). MS (ESI): *m* / z 343 (M-1). HPLC purity: 93.3%.

### (1'R,2'R,4'S)-4-Cyclopropyl-5'-methyl-2'-(prop-1-en-2-yl)-1',2',3',4'-tetrahydro-[1,1'-biphenyl]-2,4',6-triol (compound 8)

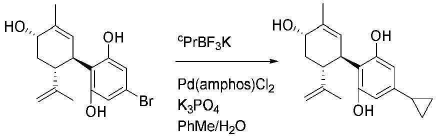

A solution of (1'*R*,2'*R*,4'*S*)-4-Bromo-5'-methyl-2'-(prop-1-en-2-yl)-1',2',3',4'-tetrahydro-[1,1'-biphenyl]-2,4',6-triol (60 mg, 0.177 mmol), potassium cyclopropyltrifluoroborate (39 mg, 0.265 mmol) and potassium phosphate tribasic (113 mg, 0.531 mmol) in toluene (2.0 mL) and water (0.5 mL) was degassed with nitrogen and treated with bis(di-*tert*-butyl(4-dimethylaminophenyl)phosphine)dichloropalladium(II) (6.3 mg, 8.84 µmol). The reaction mixture was heated at 100°C for 90 minutes then poured into water (20 mL) and extracted with ethyl acetate (2 x 30 mL). The combined organic layers were dried (hydrophobic filter paper) and concentrated *in vacuo.* The residue was purified by column chromatography on silica, eluting with 20-100% diethyl ether in cyclohexane, followed by reverse phase preparative HPLC to give the title compound as an off-white solid (0.78 mg, 1.5%).

¹H NMR (400 MHz, DMSO) d 8.84 (s, 1H), 8.63 (s, 1H), 5.91 (s, 2H), 5.07 - 5.04 (m, 1H), 4.57 (d, J=6.7 Hz, 1H), 4.49 (d, J=2.6 Hz, 1H), 4.43 - 4.40 (m, 1H), 4.13 - 4.05 (m, 1H), 3.87 - 3.82 (m, 1H), 3.22 - 3.15 (m, 1H), 1.89 (ddd, J=2.3, 5.6, 11.7 Hz, 1H), 1.63 (s, 3H), 1.59 (s, 3H), 1.59 - 1.52 (m, 2H), 0.86 - 0.80 (m, 2H), 0.49 - 0.45 (m, 2H). MS (ESI): m / z 301 (M+1). HPLC purity: 96.3%.

### (1'R,2'R,4'S)-5'-Methyl-2'-(prop-1-en-2-yl)-4-(pyridin-2-yl)-1',2',3',4'-tetrahydro-[1,1'-biphenyl]-2,4',6-triol (compound 27)

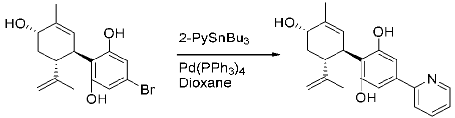

A solution of (1'*R*,2'*R*,4'*S*)-4-Bromo-5'-methyl-2'-(prop-1-en-2-yl)-1',2',3',4'-tetrahydro-[1,1'-biphenyl]-2,4',6-triol (100 mg, 0.295 mmol) in dry 1,4-dioxane (2.0 mL) was degassed with nitrogen, treated with 2-(tributylstannyl)pyridine (150 mL, 0.463 mmol) and tetrakis(triphenylphosphine)palladium(0) (34 mg, 0.0295 mmol) and heated at 95°C overnight. The reaction mixture was diluted with ethyl acetate (4 mL), water (2 mL) and brine (3 mL) and filtered. The aqueous layer was extracted further with ethyl acetate (2 x 4 mL). The combined organic layers were dried (hydrophobic frit) and concentrated *in vacuo.* The residue was purified by reverse phase preparative HPLC to give the title compound as an off-white solid (3.1 mg, 3.1%).

¹H NMR (400 MHz, DMSO) d 9.25 (s, 1H), 9.03 (s, 1H), 8.60 (ddd, J=0.9, 1.8, 4.8 Hz, 1H), 7.84 (dt, J=1.9, 7.8 Hz, 1H), 7.64 (d, J=7.4 Hz, 1H), 7.29 (ddd, J=1.0, 4.8, 7.5 Hz, 1H), 6.95 (s, 2H), 5.15 - 5.12 (m, 1H), 4.62 (d, J=6.8 Hz, 1H), 4.52 (d, J=2.6 Hz, 1H), 4.44 (dd, J=1.4, 2.6 Hz, 1H), 4.16 - 4.09 (m, 1H), 4.00 - 3.94 (m, 1H), 3.30 - 3.22 (m, 1H), 1.93 (ddd, J=2.3, 5.6, 12.1 Hz, 1H), 1.67 (s, 3H), 1.64 (s, 3H) 1.65 - 1.57 (m, 1H). MS (ESI): m / z 338 (M+1). HPLC purity: 99.5%.

(1R,2R,4S)-3'-Fluoro-3"-methoxy-5-methyl-2-(prop-1-en-2-yl)-1,2,3,4-tetrahydro-[1,1':4',1"-terphenyl]-2',4,6'-triol **(compound 50)**

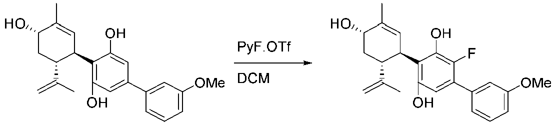

A solution of (1*R*,2*R*,4*S*)-3"-methoxy-5-methyl-2-(prop-1-en-2-yl)-1,2,3,4-tetrahydro-[1,1':4',1"-terphenyl]-2',4,6'-triol (130 mg, 0.355 mmol) in dry dichloromethane (12 mL) was cooled in an ice/water bath and treated with 1-fluoropyridinium triflate (100 mg, 0.405 mmol). The reaction mixture was allowed to warm to room temperature overnight was added and stirred as the ice melted and then at room temp overnight, then treated with further 1-fluoropyridinium triflate (100 mg, 0.405 mmol). After a further 24 hours the reaction was diluted with water (5 mL) and saturated aqueous sodium hydrogen carbonate solution (1 mL). The layers were separated and the aqueous layer was extracted further with dichloromethane (2 x 4 mL). The combined organic layers were dried (hydrophobic frit) and concentrated *in vacuo.* The residue was purified by column chromatography eluting with 0-100% diethyl ether in cyclohexane to give the title compound as an off-white solid (15.0 mg, 10%).

¹H NMR (400 MHz, DMSO) d 9.28 - 8.99 (m, 2H), 7.42 - 7.37 (m, 1H), 7.07 - 6.96 (m, 3H), 6.32 - 6.30 (m, 1H), 5.16 (s, 1H), 4.68 (d, J=6.6 Hz, 1H), 4.59 (d, J=1.8 Hz, 1H), 4.52 - 4.52 (m, 1H), 4.19 - 4.14 (m, 1H), 4.02 - 3.98 (m, 1H), 3.83 - 3.83 (m, 3H), 3.34 - 3.27 (m, 1H), 2.01 - 1.94 (m, 1H), 1.72 - 1.67 (m, 6H), 1.67 - 1.59 (m, 1H). MS (ESI): m / z 383 (M-1). HPLC purity: 92.0%.

### List of Compounds

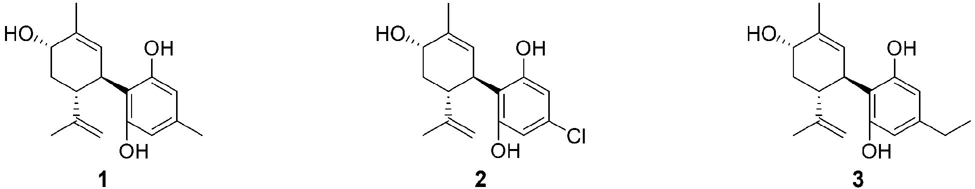

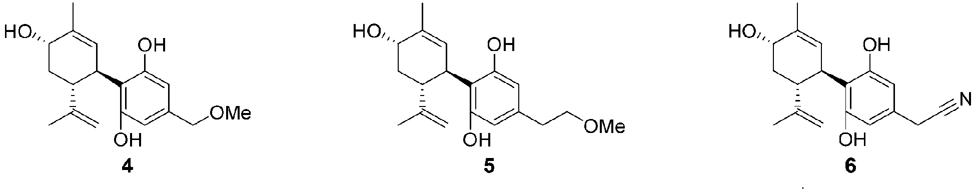

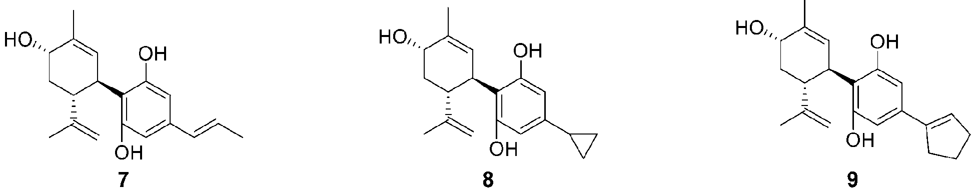

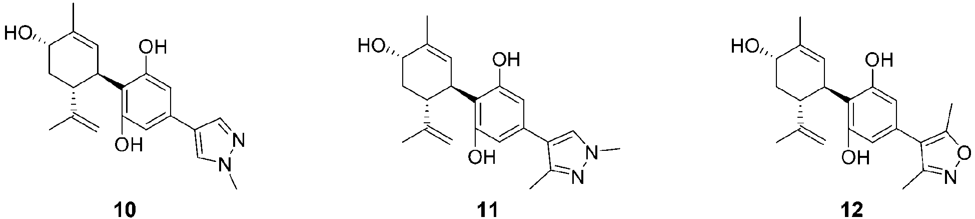

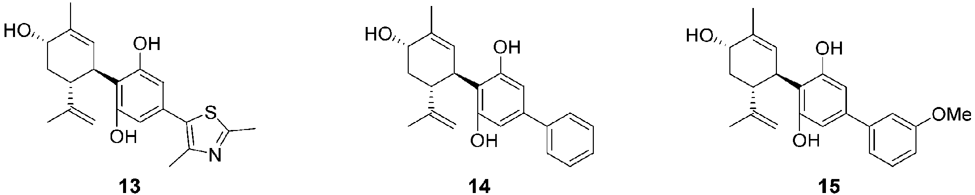

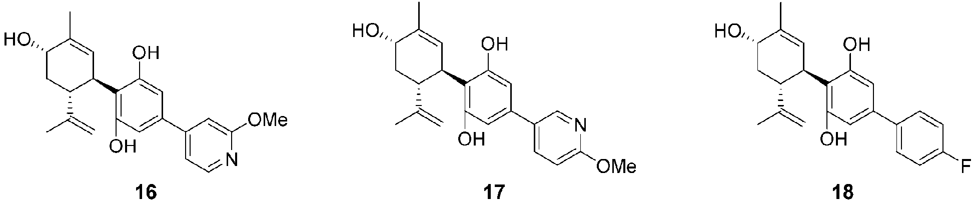

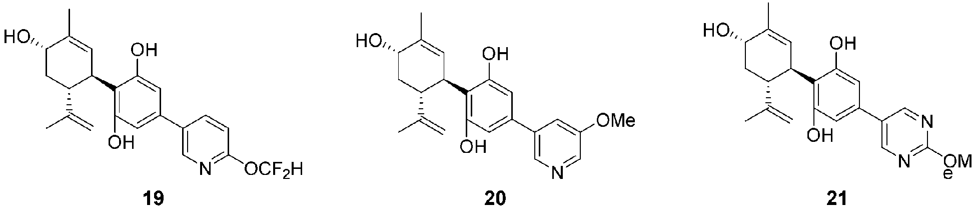

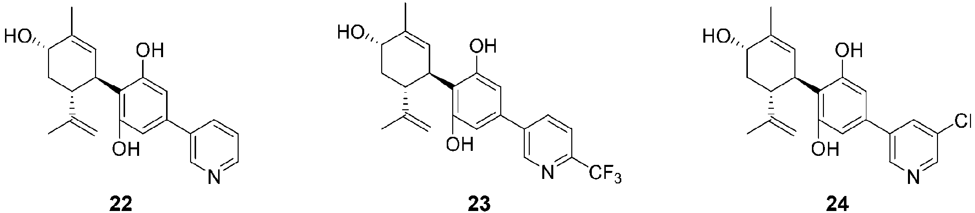

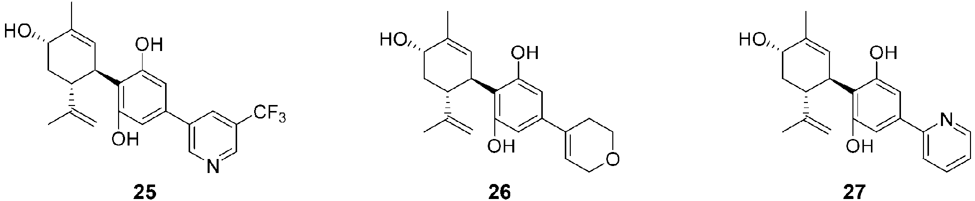

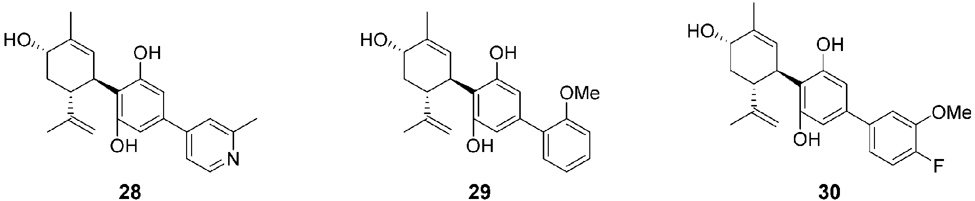

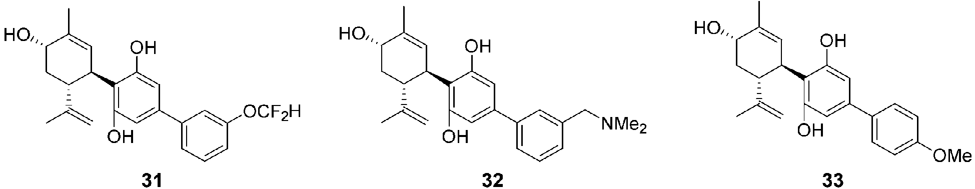

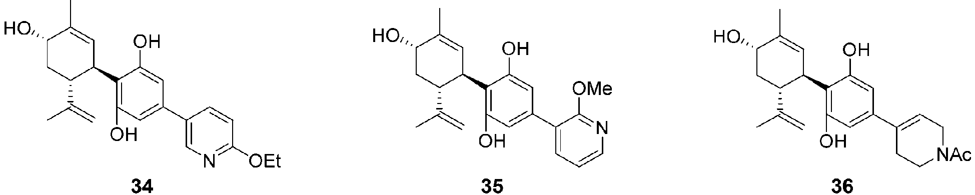

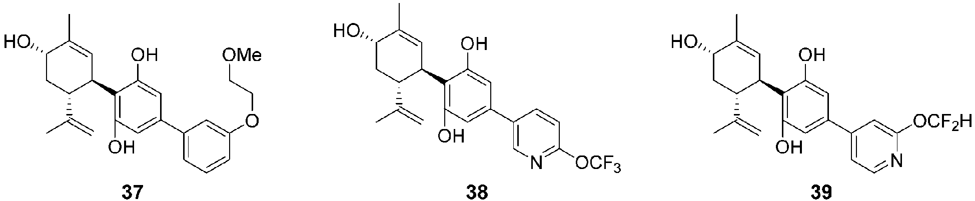

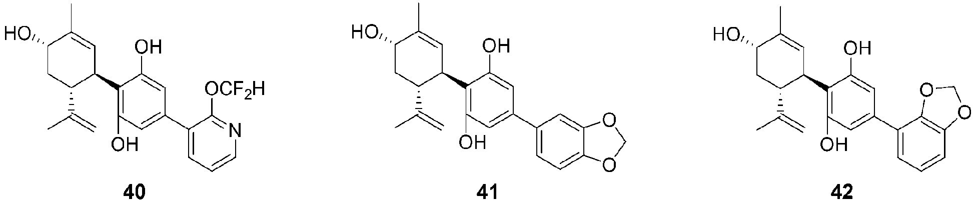

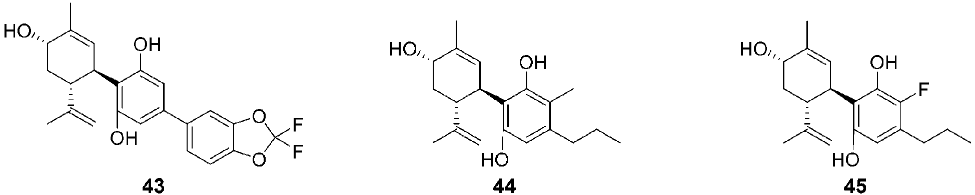

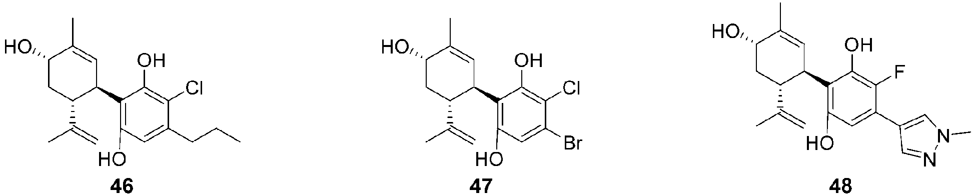

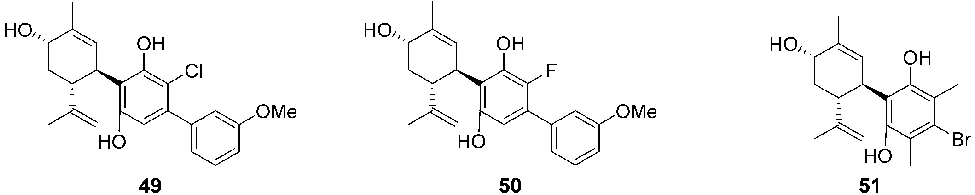

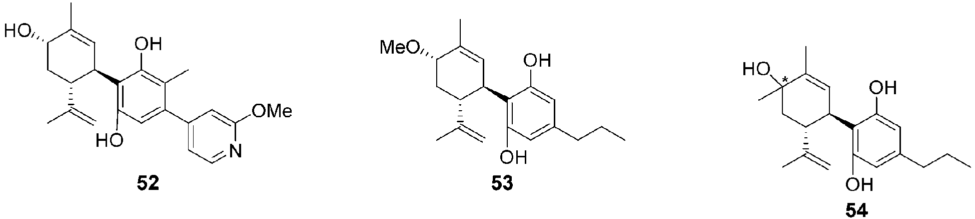

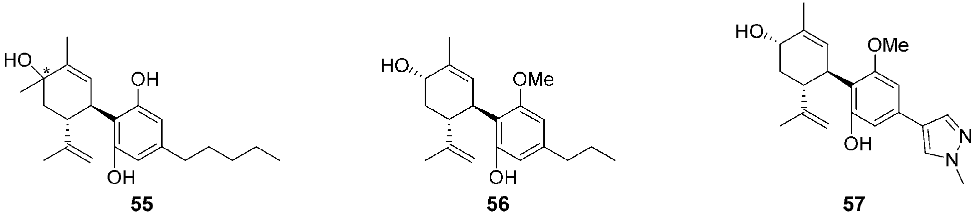

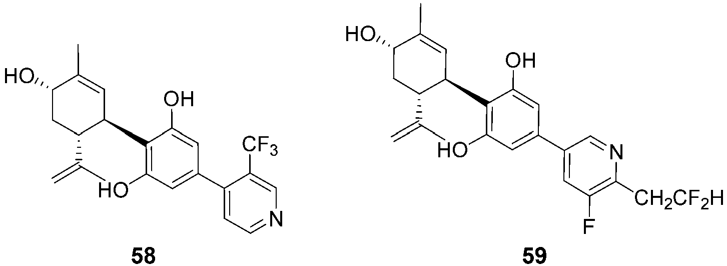

### Example 2: EVALUATION OF NOVEL RESORCINOLS FOR ANTICONVULSANT ACTIVITY USING THE MAXIMAL ELECTROSHOCK SEIZURE THRESHOLD (MEST) TEST IN THE MOUSE USING MINIMAL SAMPLE SIZES (mini MEST)

The efficacy of novel resorcinols **2, 3, 5, 8, 10, 15, 16, 17, 18, 19, 41 45, 46, 53, 56** and 58 were tested in a novel mouse model of generalised seizure, the mini-MEST (maximal electroshock seizure threshold) test, which uses lower n numbers than typically used.

The maximal electroshock seizure threshold (MEST) test is widely utilized preclinically to evaluate pro- or anti-convulsant properties of test compounds (Loscher et al., 1991).

In the MEST test the ability of a drug to alter the seizure threshold current required to induce hind limb tonic extensor convulsions is measured according to an "up and down" method of shock titration (Kimball et al., 1957). An increase in seizure threshold is indicative of anti-convulsant effect. Antiepileptic drugs including the sodium channel blockers (e.g. lamotrigine) with clinically proven efficacy against generalised tonic-clonic seizures all exhibit anti-convulsant properties in this test in the mouse.

Conversely, a reduction in seizure threshold is indicative of a pro-convulsant effect as observed with known convulsant agents such as picrotoxin.

The ability of a test compound to alter the stimulus intensity, expressed as current (mA), required to induce the presence of tonic hind limb extensor convulsions, is assessed in the MEST. The outcome of the presence (+) or absence (0) of tonic hind limb extensor convulsions observed from a current to produce tonic hind limb extension in 50% of animals in the treatment group (CC₅₀) determines the seizure threshold for the treatment group and the effects were then compared to the CC₅₀ of the vehicle control group.

### Methods

### Study Details:

Naïve mice were acclimatised to the procedure room in their home cages for up to 7 days, with food and water available ad libitum.

All animals were weighed at the beginning of the study and randomly assigned to treatment groups based on a mean distribution of body weight across groups. All animals were dosed at 10 mL/kg via intraperitoneal (i.p) injection, with either vehicle, test compound at 5-50 mg/kg, or diazepam at 2.5 mg/kg.

Animals were individually assessed for the production of a tonic hind limb extensor convulsion at 30 min post-dose for vehicle, 15-30 min post-dose for test compound (dependant on compound) and 30 min post-dose for diazepam, from a single electroshock.

The first animal within a treatment group was given a shock at the expected or estimated CC₅₀ current. For subsequent animals, the current was lowered or raised depending on the convulsions outcome from the preceding animal in log scale intervals.

Data generated from each treatment group were used to calculate the CC₅₀ ± SEM values for the treatment group.

### Test Compounds:

Vehicle: (5% ethanol, 10% solutol in 85% Saline) was prepared as follows: 1 mL of ethanol, 2 mL of solutol were warmed to 60°C, in 17 mL of saline (1:2:17).

Positive control: diazepam was used at 2.5mg/kg.

The test compounds used were **2, 3, 5, 8, 10, 15, 16, 17, 18, 19, 41, 45, 46, 53, 56** and **58** Test compounds were administered at 5-50mg/kg (i.p.) in a 1:2:17 ethanol:solutol:saline formulation.

### Sample Collection:

Each animal was humanely killed immediately after production of a convulsion by destruction of the brain from striking the cranium, followed by the confirmation of permanent cessation of the circulation from decapitation under The Humane Killing of Animals under Schedule 1 to the Animals (Scientific Procedures) Act 1986. Terminal blood and brain collection were performed following decapitation.

Blood was collected in Lithium-heparin tubes and centrifuged at 4°C for 10 minutes at 1500 x g. The resulting plasma was removed (>100 µL) and split into 2 aliquots of 0.5 mL Eppendorf tubes containing 10 µL of ascorbic acid (100 mg/mL) for stabilisation. Brains were removed, washed in saline and halved. Each half was placed into separate 2 mL screw cap cryovials, weighed and frozen on cardice.

### Statistical analysis

The data for each treatment group were recorded as the number of +'s and 0's at each current level employed and this information is then used to calculate the CC₅₀ value (current required for 50% of the animals to show seizure behaviour) ± standard error.

Test compound effects were also calculated as percentage change in CC₅₀ from the vehicle control group.

Significant difference between drug-treated animals and controls were assessed according to Litchfield and Wilcoxon (1949).

### Results

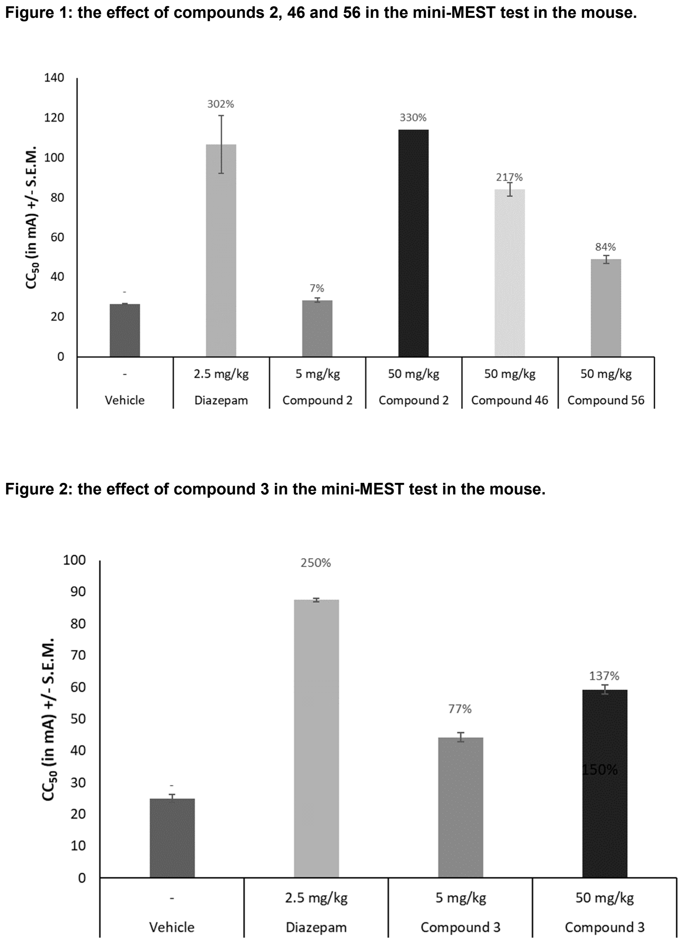

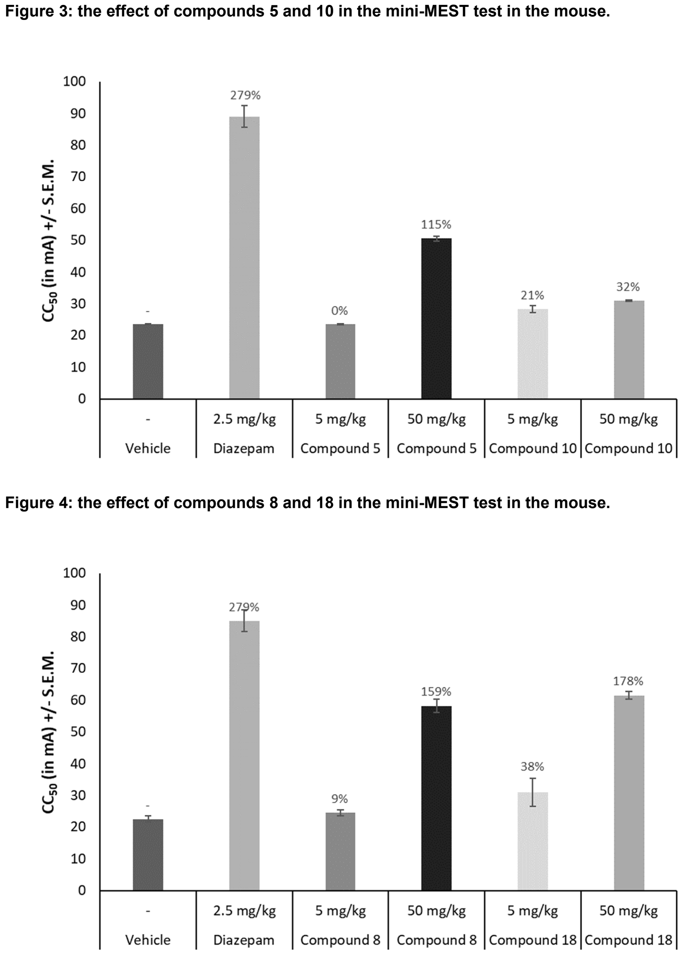

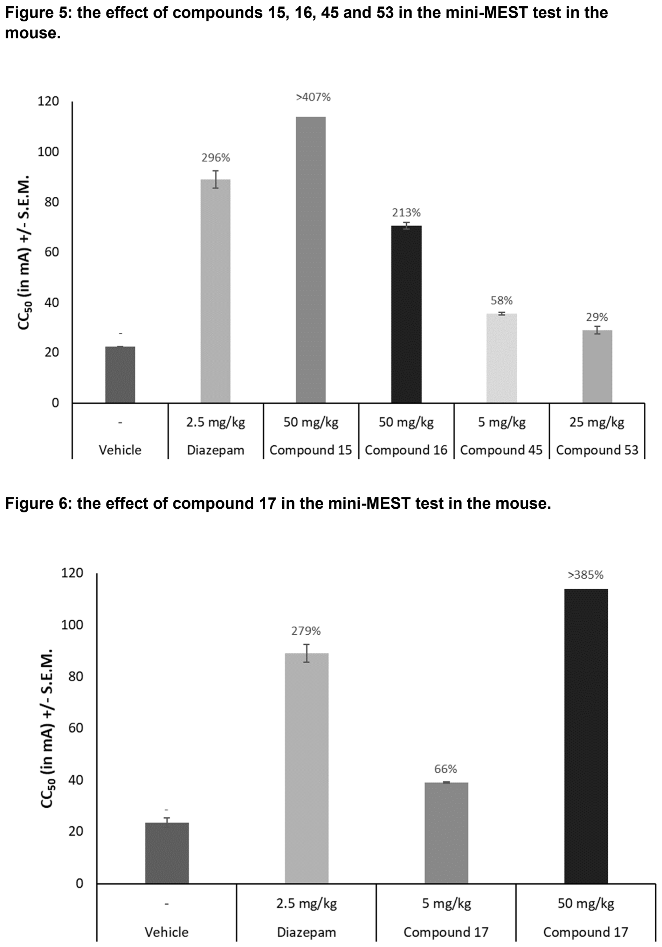

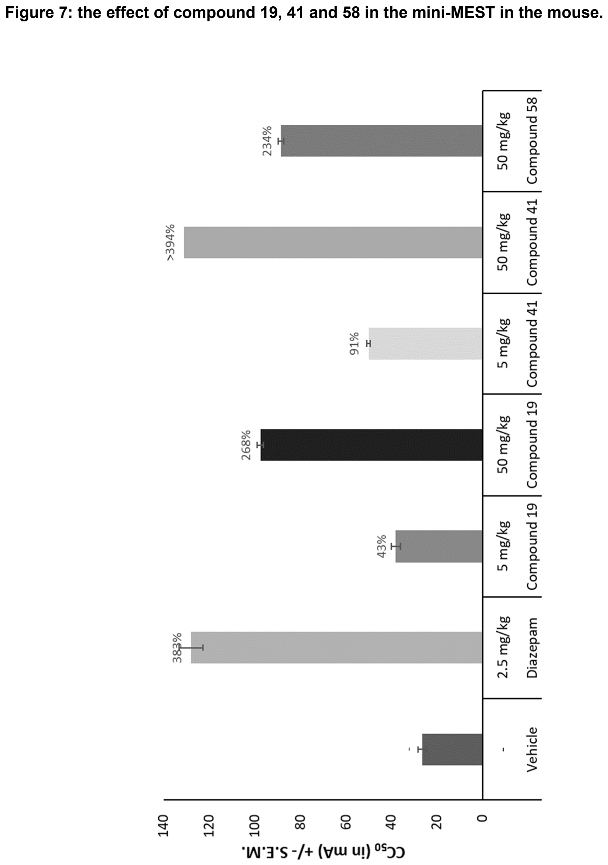

Figures 1 to 7 and Tables 2 to 8 describe the data produced in this experiment.

In the vehicle groups, the CC₅₀ values were calculated to be 22.5-26.5 mA.

In the diazepam (2.5 mg/kg) treated groups, administered i.p. 30 minutes before the test, the CC₅₀ values were 85.0-128.0 mA. These results were statistically significant (p<0.001) compared to their respective vehicle controls.

In the test compound treatment groups, administered i.p. between 15 and 30 minutes before the test, all thirteen compounds produced a statistically significant CC₅₀ value compared to vehicle in at least one dose.

Such data are indicative that these compounds will be of therapeutic benefit.

**Table 2: Evaluation of effect of Compounds 2, 46 and 56 in the mini-MEST test**

| **Treatment** | **Dose (mg/kg)** | **Test time post dose (min)** | **N** | **CC₅₀ +/-SEM** | **Significance** | **% change from vehicle** |
|---|---|---|---|---|---|---|
| **Vehicle** | - | 30 | 6 | 26.5 ± 0.3 | - | - |
| **Diazepam** | 2.5 | 30 | 6 | 106.5 ± 14.5 | P<0.001 | 302% |
| **Compound 2** | 5 | 15 | 6 | 28.3 ± 1.1 | n.s. | 7% |
| **Compound 2** | 50 | 30 | 6 | >114 | # | >330% |
| **Compound 46** | 50 | 30 | 6 | 84.0 ± 3.4 | P<0.001 | 217% |
| **Compound 56** | 50 | 30 | 6 | 48.8 ± 2.0 | P<0.001 | 84% |

| | | | | | | |
|---|---|---|---|---|---|---|
| n.s. - No statistically significant differences from vehicle was observed # Statistical significance not determined as CC₅₀ was not reached. | | | | | | |

**Table 3: Evaluation of effect of Compound 3 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 25.0+/-1.3 | - | - |
| **Diazepam** | 2.5 | 30 | 6 | 87.5+/-0.5 | P<0.001 | 250% |
| **Compound 3** | 5 | 15 | 6 | 44.2+/-1.4 | P<0.001 | 77% |
| **Compound 3** | 50 | 30 | 6 | 59.2+/-1.4 | P<0.001 | 137% |

**Table 4: Evaluation of effect of Compounds 5 and 10 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 23.5+/-0.3 | - | - |
| **Diazepam** | 2.5 | 30 | 6 | 89.0+/-3.4 | P<0.001 | 279 % |
| **Compound 5** | 5 | 15 | 6 | 23.5+/-0.3 | n.s. | 0% |
| **Compound 5** | 50 | 30 | 6 | 50.5+/-0.7 | P<0.001 | 115 % |
| **Compound 10** | 5 | 15 | 6 | 28.3+/-1.1 | P<0.001 | 21% |
| **Compound 10** | 50 | 30 | 6 | 31.0+/-0.2 | P<0.001 | 32% |

| | | | | | | |
|---|---|---|---|---|---|---|
| n.s. - No statistically significant differences from vehicle was observed | | | | | | |

**Table 5: Evaluation of effect of Compounds 8 and 18 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 22.5 ± 0.9 | - | - |
| **Diazepam** | 2.5 | 30 | 6 | 85.0 ± 3.4 | P<0.001 | 279 % |
| **Compound 8** | 5 | 15 | 6 | 24.5 ± 0.9 | n.s. | 9% |
| **Compound 8** | 50 | 30 | 6 | 58.2 ± 2.0 | P<0.001 | 159 % |
| **Compound 18** | 5 | 15 | 6 | 31.0 ± 4.5 | n.s. | 38% |
| **Compound 18** | 50 | 30 | 6 | 61.5 ± 1.3 | P<0.001 | 178% |

| | | | | | | |
|---|---|---|---|---|---|---|
| n.s. - No statistically significant differences from vehicle was observed | | | | | | |

**Table 6: Evaluation of effect of Compounds 15, 16, 45 and 53 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 22.5+/-0.9 | - | - |
| **Diazepam** | 2.5 | 30 | 6 | 89.0+/-3.4 | P<0.001 | 296 % |
| **Compound 15** | 50 | 30 | 6 | >114 | # | >407% |
| **Compound 16** | 50 | 30 | 6 | 70.5+/-1.3 | P<0.001 | 213 % |
| **Compound 45** | 5 | 15 | 6 | 35.5+/-0.5 | P<0.001 | 58% |
| **Compound 53** | 25 | 30 | 6 | 29.0+/-1.5 | P<0.001 | 29% |

| | | | | | | |
|---|---|---|---|---|---|---|
| # Statistical significance not determined as CC₅₀ was not reached. | | | | | | |

**Table 7: Evaluation of effect of Compound 17 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 23.5+/-1.9 | - | - |
| **Diazepam** | 2.5 | 30 | 6 | 89.0+/-3.4 | P<0.001 | 279 % |
| **Compound 17** | 5 | 15 | 6 | 39.0+/-0.2 | P<0.001 | 66% |
| **Compound 17** | 50 | 30 | 6 | >114 | # | >385% |

| | | | | | | |
|---|---|---|---|---|---|---|
| # Statistical significance not determined as CC₅₀ was not reached. | | | | | | |

**Table 8: Evaluation of effect of Compounds 19, 41 and 58 in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 26.5 ± 1.9 | - | - |
| **Diazepam** | 2.5 | 30 | 6 | 128.0 ± 5.2 | P<0.001 | 383 % |
| **Compound 19** | 5 | 15 | 6 | 38.0 ± 1.9 | P<0.001 | 43% |
| **Compound 19** | 50 | 30 | 6 | 97.5 ± 1.3 | P<0.001 | 268 % |
| **Compound 41** | 5 | 15 | 6 | 50.5 ± 0.7 | P<0.001 | 91% |
| **Compound 41** | 50 | 30 | 6 | >131.0 | # | >394% |
| **Compound 58** | 50 | 30 | 6 | 88.5 ± 1.3 | P<0.001 | 234 % |

| | | | | | | |
|---|---|---|---|---|---|---|
| # Statistical significance not determined as CC₅₀ was not reached. | | | | | | |

**Table 9: Evaluation of effect of Compound 7 in the mini-MEST test**

| **Treatment** | **Dose (mg/kg)** | **Test time post dose (min)** | **N** | **CC₅₀ +/-SEM** | **% change from vehicle** |
|---|---|---|---|---|---|
| **Vehicle** | - | 30 | 6 | 29.7 ± 1.1 | - |
| **Diazepam** | 2.5 | 30 | 6 | 97.8 ± 3.7 | 230% |
| **Compound 7** | 5 | 15 | 6 | 29.0 ± 1.5 | -2% |
| **Compound 7** | 50 | 30 | 6 | 75.0 ± 3.4 | 153% |

**Table 10: Evaluation of effect of Compounds 9, 49 and 52 in the mini-MEST test**

| **Treatment** | **Dose (mg/kg)** | **Test time post dose (min)** | **N** | **CC₅₀ +/-SEM** | **% change from vehicle** |
|---|---|---|---|---|---|
| **Vehicle** | - | 30 | 6 | 28.3 ± 1.1 | |
| **Diazepam** | 2.5 | 30 | 6 | 135.0 ± 0.6 | 376% |
| **Compound 9** | 50 | 30 | 6 | >131.0 | >344% |
| **Compound 49** | 50 | 30 | 6 | 97.5 ± 1.3 | 244% |
| **Compound 52** | 5 | 15 | 6 | 31.0 ± 4.5 | 9% |
| **Compound 52** | 50 | 30 | 6 | 76.5 ± 2.6 | 170% |

**Table 11: Evaluation of effect of Compound 26 in the mini-MEST test**

| **Treatment** | **Dose (mg/kg)** | **Test time post dose (min)** | **N** | **CC₅₀ +/-SEM** | **% change from vehicle** |
|---|---|---|---|---|---|
| **Vehicle** | - | 30 | 6 | 25.0 ± 1.1 | - |
| **Diazepam** | 2.5 | 30 | 6 | 97.8 ± 3.7 | 291% |
| **Compound 26** | 5 | 15 | 6 | 31.0 ± 0.2 | 24 % |
| **Compound 26** | 50 | 30 | 6 | 50.0 ± 2.6 | 100 % |

**Table 12: Evaluation of effect of Compounds 29, 30, 31 and 33 in the mini-MEST test**

| **Treatment** | **Dose (mg/kg)** | **Test time post dose (min)** | **N** | **CC₅₀ +/-SEM** | **% change from vehicle** |
|---|---|---|---|---|---|
| **Vehicle** | - | 30 | 6 | 26.5 ± 1.9 | - |
| **Diazepam** | 2.5 | 30 | 6 | 121.0 ± 9.0 | 357 % |
| **Compound 29** | 5 | 15 | 6 | 35.0 ± 2.6 | 32 % |
| **Compound 29** | 50 | 30 | 6 | 70.5 ± 1.3 | 166 % |
| **Compound 30** | 5 | 15 | 6 | 43.0 ± 1.9 | 62 % |
| **Compound 30** | 50 | 30 | 6 | >131 | >394 % |
| **Compound 31** | 5 | 15 | 6 | 33.0 ± 1.5 | 25% |
| **Compound 31** | 50 | 30 | 6 | 79.5 ± 1.3 | 200 % |
| **Compound 33** | 5 | 15 | 6 | 38.8 ± 1.4 | 47 % |
| **Compound 33** | 50 | 30 | 6 | 79.5 ± 0.9 | 200 % |

**Table 13: Evaluation of effect of Compounds 35, 42 and 47 in the mini-MEST test**

| **Treatment** | **Dose (mg/kg)** | **Test time post dose (min)** | **N** | **CC₅₀ +/-SEM** | **% change from vehicle** |
|---|---|---|---|---|---|
| **Vehicle** | - | 30 | 6 | 23.5 ± 0.3 | - |
| **Diazepam** | 2.5 | 30 | 6 | 121.0 ± 15.6 | 415% |
| **Compound 35** | 5 | 15 | 6 | 38.0 ± 1.9 | 62% |
| **Compound 35** | 50 | 30 | 6 | >131.0 | 457% |
| **Compound 42** | 50 | 30 | 6 | >131.0 | 457% |
| **Compound 47** | 25 | 30 | 6 | 79.5 ± 10.0 | 238% |

### Conclusions

These data demonstrate a therapeutic effect for the compounds.

These data are significant as they provide heretofore unknown evidence that these novel resorcinols may be of therapeutic value.

The compounds tested were those detailed as compound **2,** compound **3,** compound **5,** compound **8** and compound **10,** compound **15,** compound **16,** compound **17,** compound **18,** compound **19,** compound **41,** compound **45,** compound **46,** compound **53,** compound **56** and compound **58.** Such compounds are examples of the novel resorcinols of general Formulae I to V.

Further compounds that were tested were those detailed as compound **7,** compound **9,** compound **26,** compound **29,** compound **30,** compound **31,** compound **33,** compound **35,** compound **42,** compound **47,** compound **49** and compound **52.**

As representative compounds showed efficacy in the mini-MEST test such therapeutic efficacy can be attributed to the novel compounds of general Formulae I to V of the invention.

## Claims

1. A compound of formula (II), or a salt thereof: 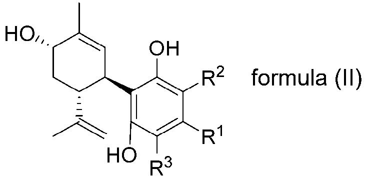 wherein:
R¹ is CH₂CH₂CH₃, Br, 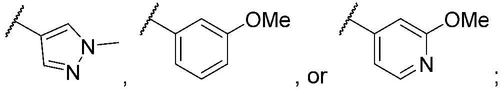
R² is CH₃, F or Cl; and
R³ is H or CH₃.

2. The compound of claim 1, where R¹ is CH₂CH₂CH₃.

3. The compound of claim 1 or claim 2, where R² is F or Cl.

4. The compound of any one of claims 1 to 3, where R³ is H.

5. A compound of formula (IV), or a salt thereof: 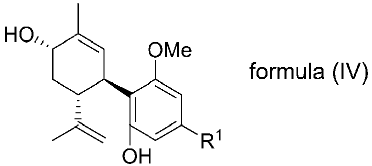 where:
R¹ is CH₂CH₂CH₃ or

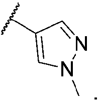

6. The compound of claim 5, where R¹ is CH₂CH₂CH₃.

7. The compound of claim 1 or claim 5, wherein the compound is selected from:

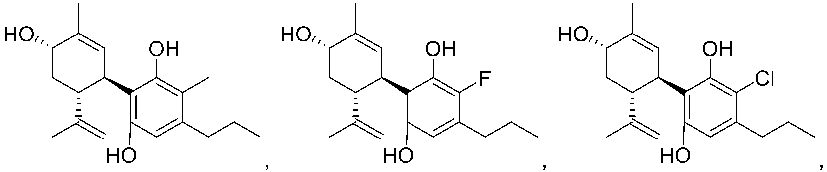

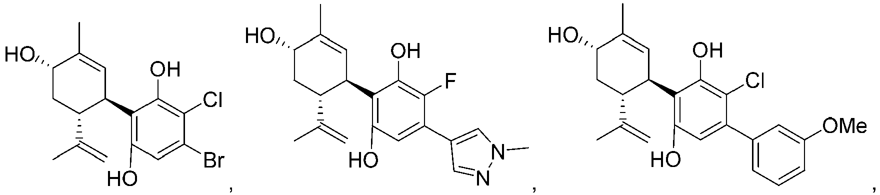

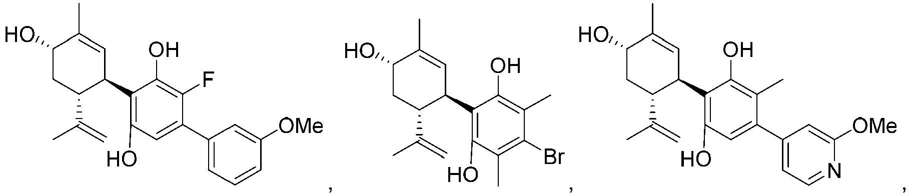

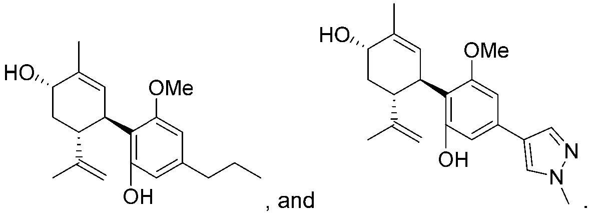

8. A pharmaceutical composition comprising the compound of any one of claims 1 to 7 and one or more additional pharmaceutically acceptable ingredients.

9. The pharmaceutical composition of claim 8, wherein the one or more additional pharmaceutically acceptable ingredients are selected from carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

10. The pharmaceutical composition of claim 8 or claim 9, wherein the composition is selected from a liquid, a solution, a suspension, an emulsion, a syrup, an electuary, a mouthwash, a drop, a tablet, a granule, a powder, a lozenge, a pastille, a capsule, a cachet, a pill, an ampoule, a bolus, a suppository, a pessary, a tincture, a gel, a paste, an ointment, a cream, a lotion, an oil, a foam, a spray, and an aerosol.

11. The compound of any one of claims 1 to 7, or the pharmaceutical composition of any one of claims 8 to 10, for use as a medicament.

12. The compound of any one of claims 1 to 7, or the pharmaceutical composition of any one of claims 8 to 10, for use in a method of treating epilepsy.

13. The compound of any one of claims 1 to 7, or the pharmaceutical composition of any one of claims 8 to 10, for use in a method of treating generalised seizure, focal onset seizure or tonic-colic seizure.
